# EUROPEAN PATENT APPLICATION

(11) **EP 4 851 161 A1**
(43) Date of publication of application: **09.12.2026**
(21) Application number: 25748014.5
(22) Date of filing: 27.01.2025
(51) Int. Cl.: C07D 417/14, A61K 51/04, A61P 25/28

(54) **COMPOUND FOR RECOGNIZING ALPHA-SYNUCLEIN AGGREGATE, AND USE THEREOF**

(30) Priority: 29.01.2024 CN 202410121133; 29.01.2024 CN 202410121172; 26.02.2024 CN 202410209817
(71) Applicant: Synusight Biotech (Shanghai) Co., Ltd., Shanghai 201318 (CN)
(72) Inventor: LI, Dan, Shanghai 201411 (CN); BIAN, Jiang, Shanghai 201411 (CN); LIU, Cong, Shanghai 201411 (CN); TAN, Li, Shanghai 201411 (CN); ZHANG, Shenqing, Shanghai 201411 (CN); HAN, Chao, Shanghai 201411 (CN)
(74) Representative: Bianchetti & Minoja with Trevisan & Cuonzo IPS SRL
(86) International application number: PCT/CN2025/075517
(87) International publication number: WO 2025/162452

(57) **Abstract**

A compound specifically binding to an α-synuclein aggregate, and a preparation method therefor and the use thereof. Specifically, the compound binding to the α-synuclein aggregate comprises compounds as shown in formula A or sub-general formulas thereof, or stereoisomers, pharmaceutically acceptable salts, solvates or stable isotope variants thereof. The compound is a small molecule tracer, which can specifically recognize the α-synuclein aggregate, and can be used for the preparation of a drug for the treatment or diagnosis of neurodegenerative diseases (such as Parkinson's disease, dementia with Lewy bodies, multiple system atrophy, Alzheimer's disease, amyotrophic lateral sclerosis, progressive supranuclear palsy and progressive muscular atrophy) related to the α-synuclein aggregate and other misfolded proteins.

## Description

### Cross-Reference

This application claims the priority of Chinese invention patent application No. 202410121172.8 filed on January 29, 2024, Chinese invention patent application No. 202410121133.8 filed on January 29, 2024, and Chinese invention patent application No. 202410209817.3 filed on February 26, 2024.

### Technical Field

The present invention relates to the pharmaceutical field, specifically to a compound specifically recognizing α-synuclein aggregates and use thereof.

### Background Technology

Neurodegenerative diseases (ND) are closely related to aging, including Alzheimer's disease (AD), Parkinson's disease (PD), amyotrophic lateral sclerosis (ALS), multiple system atrophy (MSA), dementia with Lewy bodies (DLB), progressive muscular atrophy, progressive supranuclear palsy (PSP), etc., characterized by progressive neuronal loss and degeneration. Currently, there are over 57 million ND patients worldwide. With the aging of the world's population, the number of ND patients is expected to triple by the middle of the 21st century, which will lead to a huge medical and public health burden. However, there is currently no cure for ND, and clinical drugs can only relieve or slow the symptoms of related diseases. ND has a long pathogenesis period; pathological changes occur for more than a decade before clinical symptoms appear, during which patients have already lost most neurons. Given the non-regenerative nature of neurons, early diagnosis and treatment of ND is an effective way to improve ND efficacy.

Abnormal protein aggregation is a common pathological change in ND and the most important early diagnostic biomarker for ND. Under physiological conditions, these proteins exist as disordered monomers, whereas under pathological conditions, they form ordered β-sheet structures and gradually aggregate into oligomers and fibrils. In AD, amyloid β (Aβ) forms amyloid plaques (AP) extracellularly, which are mainly deposited in the cortex, subcortical regions, and brainstem; simultaneously, another pathological protein, Tau, can fibrillize and form neurofibrillary tangles (NFT) deposited in the entorhinal cortex, hippocampus, and basal cortex. In PD, α-synuclein (α-syn) forms Lewy bodies and Lewy neurites, appearing in tissues and organs such as the substantia nigra, striatum, brainstem, and olfactory bulb.

Positron emission tomography (PET) and single-photon emission computed tomography (SPECT) are non-invasive imaging methods that can detect brain structure and function, thereby studying the pathological progression and pathogenesis of ND. This technology incorporates radionuclides into small molecule tracers, which can target certain disease-related proteins *in vivo.* The positrons or alpha photons emitted by the radionuclides are detected and localized by the imaging system's detectors, and the tracer uptake and spatial distribution are calculated by a processor and image reconstruction software, inferring changes in brain function and structure. These techniques have high spatial resolution, with PET visualization and quantification resolution reaching 3 millimeters.

Detecting aggregates in the brain via PET and SPECT technology is of great significance for the early diagnosis of synucleinopathies. Synucleinopathies include PD, DLB, and MSA. In PD patients, abnormal neuronal inclusions called Lewy bodies (LB) and Lewy neurites (LN) appear primarily in the substantia nigra and striatum. The cerebral cortex of DLB patients also contains abundant LB and LN. Unlike PD and DLB, α-syn aggregates in the brains of MSA patients are rarely found in neurons but exist in the cytoplasm of oligodendrocytes and nerve cells, forming glial cytoplasmic inclusions (GCI). These pathological changes occur early in synucleinopathies and are closely related to disease progression. Therefore, imaging these misfolded α-syn using brain imaging techniques enables early diagnosis of the disease, which is of great significance for disease treatment and the development of new drugs.

However, existing PET tracers exhibit weak binding activity to the protein and poor *in vivo* imaging capabilities, failing to meet clinical imaging needs. Therefore, there is an urgent need to develop a compound that can specifically recognize α-syn aggregates, aiding in the clinical early diagnosis and treatment of neurodegenerative diseases associated with α-synuclein aggregates, such as PD, DLB, and MSA.

### Summary of the Invention

To address the above issues, the present invention provides a compound with strong binding ability to α-synuclein aggregates. After binding to the target protein, it can be used to prepare formulations or formulation compositions for treating or diagnosing neurodegenerative diseases associated with α-synuclein aggregates and other misfolded proteins.

A first aspect of the present invention provides a compound specifically recognizing α-synuclein aggregates, said compound being a compound of Formula A, or a stereoisomer, pharmaceutically acceptable salt, solvate, or stable isotopic variant thereof, 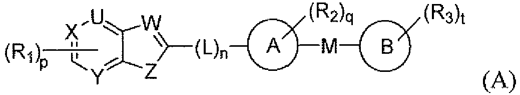 wherein each variable is as defined herein.

Specifically, the first aspect of the present invention provides compounds of the following sub-generic formulas, or stereoisomers, pharmaceutically acceptable salts, solvates, or stable isotopic variants thereof, 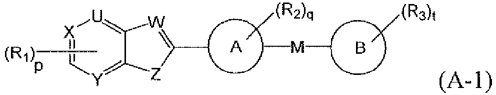 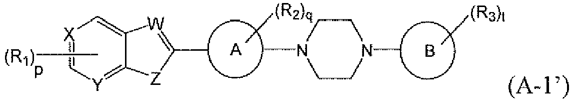 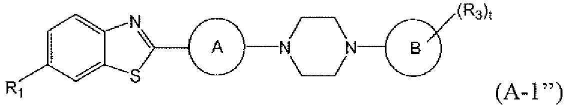 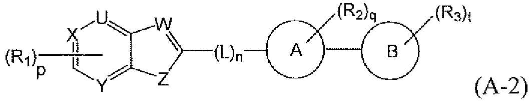 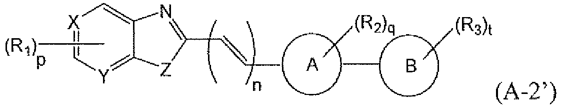 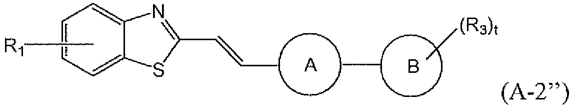 wherein each variable is as generally or specifically defined herein.

A second aspect of the present invention provides a composition specifically recognizing α-synuclein aggregates, said composition comprising
a. a compound according to the first aspect of the present invention; and optionally
b. a pharmaceutically acceptable carrier.

A third aspect of the present invention provides a use of a compound according to the first aspect of the present invention and a composition according to the second aspect of the present invention in the manufacture of a medicament for treating or diagnosing neurodegenerative diseases associated with α-synuclein aggregates and aggregates of other misfolded proteins.

A fourth aspect of the present invention provides a method for detecting α-synuclein aggregates in a subject, comprising the steps of:
(A) administering to the subject a safe and effective amount of a compound according to the first aspect of the present invention or a composition according to the second aspect of the present invention; and
(B) detecting the binding of said compound or composition to α-synuclein aggregates in the subject.

In a preferred embodiment, the detection in step (B) is performed by an imaging technique, preferably by the following imaging technologies: positron emission tomography, single-photon emission computed tomography, near-infrared brain function imaging, or a combination thereof.

In another preferred embodiment, the subject suffers from or is suspected of suffering from a neurodegenerative disease associated with α-synuclein aggregates or aggregates of other misfolded proteins.

In another preferred embodiment, the neurodegenerative disease is selected from the group consisting of Parkinson's disease, multiple system atrophy, progressive muscular atrophy, dementia with Lewy bodies, Alzheimer's disease, progressive supranuclear palsy, or amyotrophic lateral sclerosis.

A fifth aspect of the present invention provides a use of a compound according to the first aspect of the present invention and a composition according to the second aspect of the present invention in the manufacture of a kit for detecting the amount of α-synuclein aggregates and aggregates of other misfolded proteins.

### Brief Description of the Drawings

Figure 1 shows immunofluorescence staining images of the co-localization of the compound of the present application with α-syn aggregates in primary neurons.
Figure 2 shows immunofluorescence staining images of the co-localization of the compound of the present application with α-syn aggregates in the brain of **PFF** mice (Upper panel: Examples 1, 2, 3, 7, 11, 16, 26, 38 refer to Examples II-1, II-2, II-3, II-7, II-11, II-16, II-26, II-38, respectively).
Figure 3 shows immunofluorescence staining images of the co-localization of the compound of the present application with α-syn aggregates in the brain of PD patients (Upper panel: Examples 1, 2, 11, 26 refer to Examples I-1, I-2, I-11, I-26; Lower panel: Examples 1, 16, 19 refer to Examples II-1, II-16, II-19).
Figure 4 shows immunofluorescence staining images of the co-localization of the compound of the present application with α-syn aggregates in the brain of **PFF** mice after intravenous injection of the compound (Upper panel: Examples 1, 2 refer to Examples I-1, I-2; Lower panel: Examples 1, 16 refer to Examples II-1, II-16).
Figures 5A and 5B show autoradiography (ARG) images of the compound of the present application in mouse and PD patient brain sections, respectively.
Figure 6A shows a PET imaging of the compound of the present application 18F FD4 in a PFF-injected rat.
Figure 6B shows a PET imaging of the compound of the present application 18F FD4 in a PFF-injected marmoset.
Figure 6C shows a PET imaging of the compound of the present application FD17 in a PFF-injected rat.
Figure 6D shows a PET imaging of the compound of the present application FD17 in a PFF-injected marmoset.
Figure 6E shows a PET imaging of the compound of the present application FA35 in a PFF-injected rat.
Figure 6F shows a PET imaging of the compound of the present application FA23 in a PFF-injected rat.
Figure 7 shows a PET imaging of a human subject administered with the compound 18F FD4.
Figure 8 shows a PET imaging of a human subject administered with C0505.

### Detailed Description of the Invention

After long and in-depth research and extensive screening, the inventors have developed for the first time a compound specifically recognizing α-synuclein aggregates, which includes compounds represented by Formula A and its various sub-generic formulas, or stereoisomers, pharmaceutically acceptable salts, solvates, or stable isotopic variants thereof. The compound of the present application can be used as a detection reagent to probe the amount of α-synuclein aggregates and other pathological aggregated proteins in *in vitro* samples, or for preparing drugs for treating or diagnosing neurodegenerative diseases associated with pathological protein aggregates such as α-synuclein aggregates (e.g., Parkinson's disease, multiple system atrophy, dementia with Lewy bodies, Alzheimer's disease, amyotrophic lateral sclerosis, etc.), having very good application prospects.

### Terms

As used herein, the term "comprise" or "include (contain)" may be open, semi-closed, and closed. In other words, the term also includes "consist essentially of......" or "consist of......".

As used herein, the term "alkyl" refers to a monovalent straight or branched chain saturated hydrocarbon group consisting only of carbon and hydrogen atoms. For example, "C₁₋₆ alkyl" refers to an alkyl group having 1-6 (e.g., 1, 2, 3, 4, 5, or 6) carbon atoms or a range of carbon atoms composed of any different values therein, such as C₁₋₄ alkyl, C₁₋₃ alkyl, C₁₋₂ alkyl. Examples of alkyl include, but are not limited to: methyl, ethyl, propyl, isopropyl, butyl, isobutyl, or tert-butyl, etc.

As used herein, the term "alkylene" refers to a divalent group obtained by removing a hydrogen atom from an alkyl group as defined above, such as methylene (-CH₂-), ethylene (-CH₂CH₂-), etc.

As used herein, the term "alkenyl" refers to a straight or branched chain unsaturated hydrocarbon group composed of carbon and hydrogen atoms having at least one double bond, for example, "C₂₋₆ alkenyl" refers to an alkenyl group having 2-6 (e.g., 2, 3, 4, 5, or 6) carbon atoms or a range of carbon atoms composed of any different values therein, such as C₂₋₄ alkenyl, C₂₋₃ alkenyl, C₂₋₅ alkenyl. Examples of alkenyl include, but are not limited to: ethenyl, 1-propenyl, 2-propenyl, 1-butenyl, isobutenyl, and 1,3-butadienyl, etc.

As used herein, the term "alkynyl" refers to a straight or branched chain unsaturated hydrocarbon group composed of carbon and hydrogen atoms having at least one triple bond. For example, "C₂₋₆ alkynyl" refers to an alkynyl group having 2-6 (e.g., 2, 3, 4, 5, or 6) carbon atoms or a range of carbon atoms composed of any different values therein, such as C₂₋₄ alkynyl, C₂₋₃ alkynyl, C₂₋₅ alkynyl. Examples of alkynyl include, but are not limited to: ethynyl, 1-propynyl, 2-propynyl, and 1-butynyl, etc.

As used herein, the term "cycloalkyl" refers to a monovalent saturated carbocyclic group composed of carbon and hydrogen atoms, for example, "C₃₋₈ cycloalkyl" refers to a cycloalkyl group containing 3-8 (e.g., 3, 4, 5, 6, 7, or 8) carbon atoms or a range of carbon atoms composed of any different values therein, preferably C₃₋₆ cycloalkyl. Cycloalkyl can be monocyclic, such as cyclopropyl, cyclobutyl, cyclopentyl, cyclohexyl or the like, or bicyclic, such as bridged or spiro forms, preferably monocyclic.

As used herein, the term "haloalkyl" refers to a group obtained by replacing one or more hydrogens in an alkyl group as defined above with the same or different halogens. "Halo C₁₋₆ alkyl" or "C₁₋₆ haloalkyl" are used interchangeably, preferably halo C₁₋₄ alkyl. Examples of haloalkyl include, but are not limited to: -CH₂Cl, -CH₂CF₃, -CH₂CCl₃, perfluoroalkyl (e.g., -CF₃-, -CF₂CF₃), etc.

As used herein, the term "alkoxy" refers to a group of formula -OR_{z} or -R_{z}'-OR_{z}, wherein R_{z} is alkyl as defined herein, R_{z}' is alkylene, preferably -C₁₋₄ alkoxy or -O-C₁₋₄ alkyl. Examples of alkoxy include, but are not limited to: methoxy, ethoxy, isopropoxy, tert-butoxy, -CH₂O-CH₃, -CH₂CH₂-O-CH₃, -CH₂-O-CH₂CH₃, etc.

As used herein, the term "haloalkoxy" refers to a group obtained by replacing one or more hydrogens in an alkoxy group as defined above with the same or different halogens, preferably -C₁₋₄ haloalkoxy or -O-C₁₋₄ haloalkyl. Examples of haloalkoxy include, but are not limited to: trifluoromethoxy, difluoromethoxy, 2,2,2-trifluoroethoxy, 2-fluoroethoxy, etc.

As used herein, the term "halohydroxyalkoxy" refers to a group obtained by replacing one or more hydrogen atoms in a haloalkoxy group as defined above with a hydroxyl group, preferably -C₁₋₄ halohydroxyalkoxy or -O-C₁₋₄ halohydroxyalkyl, more preferably a -C₁₋₄ haloalkoxy group substituted with a hydroxyl group. Examples of halohydroxyalkoxy include, but are not limited to

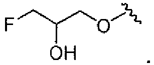

As used herein, the term "alkylamino" refers to a group of formula -NR_{y}Rₓ, wherein R_{y} and Rₓ are each independently H or alkyl as defined herein, and R_{y} and Rₓ are not simultaneously H, i.e., alkylamino can be monoalkylamino or dialkylamino, preferably -NHC₁₋₄ alkyl or -N(C₁₋₄ alkyl)₂. Examples of alkylamino include, but are not limited to: -NH-methyl, NH-ethyl, -N-dimethyl, -N-diethyl, etc.

As used herein, the term "haloalkylamino" refers to a group obtained by replacing one or more hydrogens in an alkylamino group as defined above with the same or different halogens, preferably - NHC₁₋₄ alkyl or -N(C₁₋₄ alkyl)₂, wherein the alkyl groups are each independently substituted by one or more halogens, preferably by one or more F and/or Cl.

As used herein, the term "halohydroxyalkylamino" refers to a group obtained by replacing one or more hydrogen atoms in a haloalkylamino group as defined above with a hydroxyl group, preferably a -C₁₋₄ haloalkylamino group substituted with a hydroxyl group. Examples of halohydroxyalkylamino include, but are not limited to

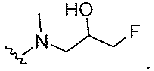

As used herein, the term "halogen" refers to halogens and isotopes thereof, including but not limited to F, ¹⁸F, Cl, ³²Cl, Br, I.

As used herein, the term "amino" refers to -NH₂.

As used herein, the term "carboxyl" refers to -COOH.

As used herein, the term "ester group" refers to -COOR_{w}, wherein R_{w} can be independently selected from the group consisting of: hydrogen, substituted or unsubstituted alkyl, substituted or unsubstituted cycloalkyl, substituted or unsubstituted cycloalkenyl, substituted or unsubstituted aryl, substituted or unsubstituted heteroaryl, substituted or unsubstituted heterocyclyl; preferably - C(O)OC₁₋₆ alkyl. Examples of ester groups include, but are not limited to: -COOCH₃, -COOCH₂CH₃, -COOCH₂CH₂CH₃, -COOCH₂CH(CH₃)₂, etc.

As used herein, the term "acyl group" refers to -COR_{w}, wherein R_{w} can be independently selected from the group consisting of: hydrogen, substituted or unsubstituted alkyl, substituted or unsubstituted cycloalkyl, substituted or unsubstituted cycloalkenyl, substituted or unsubstituted aryl, substituted or unsubstituted heteroaryl, substituted or unsubstituted heterocyclyl; preferably -C(O)C₁₋₆ alkyl. Examples of acyl groups include, but are not limited to: -COCH₃, -COCH₂CH₃, - COCH₂CH₂CH₃, -COCH₂CH(CH₃)₂, etc.

As used herein, the term "amide group" refers to -CONRᵥRᵥ', wherein Rᵥ and Rᵥ' can be independently selected from the group consisting of: hydrogen, substituted or unsubstituted alkyl, substituted or unsubstituted cycloalkyl, substituted or unsubstituted cycloalkenyl, substituted or unsubstituted aryl, substituted or unsubstituted heteroaryl, substituted or unsubstituted heterocyclyl. Rᵥ and Rᵥ' can be the same or different. Preferably, Rᵥ and Rᵥ' are independently selected from hydrogen and C₁₋₆ alkyl, i.e., the amide group is selected from -C(O)NH₂, -C(O)NHC₁₋₆ alkyl, - C(O)N(C₁₋₆ alkyl)₂. Examples of amide groups include, but are not limited to: -CONH₂, -CONHCH₃, -CON(CH₃)₂, etc.

As used herein, the term "sulfonamide group" refers to -SO₂NRᵥRᵥ' or RᵥSO₂NRᵥ'-, wherein Rᵥ and Rᵥ' can be independently selected from the group consisting of: hydrogen, substituted or unsubstituted alkyl, substituted or unsubstituted cycloalkyl, substituted or unsubstituted cycloalkenyl, substituted or unsubstituted aryl, substituted or unsubstituted heteroaryl, substituted or unsubstituted heterocyclyl. Rᵥ and Rᵥ' can be the same or different. Preferably, Rᵥ and Rᵥ' are independently selected from hydrogen and C₁₋₆ alkyl, i.e., the amide group is selected from -SO₂NH₂, -SO₂NHC₁₋₆ alkyl, - SO₂N(C₁₋₆ alkyl)₂, -NHSO₂-C₁₋₆ alkyl, -N(C₁₋₆ alkyl)-SO₂-C₁₋₆ alkyl. Examples of sulfonamide groups include, but are not limited to: -SO₂NH₂, -SO₂NHCH₃, -SO₂N(CH₃)₂, CH₃SO₂NH-, CH₃SO₂NCH₃-, etc.

As used herein, the term "heterocyclyl" refers to a fully saturated or partially saturated monocyclic, bicyclic, or polycyclic ring group containing one or more heteroatoms selected from N, S, or O in the ring skeleton, for example, "3-12 membered heterocyclyl", "3-8 membered heterocyclyl", "4-8 membered heterocyclyl", "5-8 membered heterocyclyl", "6-8 membered heterocyclyl" refer to groups having 3-12 ring members, 3-8 ring members, 4-8 ring members, 5-8 ring members, 6-8 ring members, respectively. The nitrogen or sulfur atom of the heterocyclyl may be oxidized, and the nitrogen atom may also be quaternized. The heterocyclyl can be attached to the residue of any heteroatom or carbon atom of the ring or ring system molecule. For the compounds of the present invention, a monocyclic 3-8 membered saturated heterocyclyl is preferred, such as a monocyclic 4-8 membered saturated heterocyclyl, a monocyclic 5-8 membered saturated heterocyclyl, or a monocyclic 6-8 membered saturated heterocyclyl. Monocyclic heterocycles include, but are not limited to: azetidinyl, pyrrolidinyl, oxetanyl, pyrazolinyl, imidazolinyl, imidazolidinyl, oxazolidinyl, isoxazolidinyl, thiazolidinyl, isothiazolidinyl, tetrahydrofuranyl, piperidinyl, piperazinyl, 2-oxopiperazinyl, 2-oxopiperidinyl, 2-oxopyrrolidinyl, hexahydroazepinyl, 4-piperidonyl, tetrahydropyranyl, morpholinyl, thiomorpholinyl, thiomorpholinyl sulfoxide, thiomorpholinyl sulfone, 1,3-dioxanyl, and tetrahydro-1,1-dioxothiophenyl, etc. Polycyclic heterocyclyl includes, but is not limited to, spiro, fused, and bridged heterocyclyl groups, wherein the spiro, fused, and bridged heterocyclyl groups are optionally connected to other groups via a single bond, or are further fused to other cycloalkyl, heterocyclyl, aryl, and heteroaryl groups via any two or more atoms on the ring.

As used herein, the term "aryl" refers to an aromatic cyclic hydrocarbon group (including monocyclic, bicyclic, or polycyclic groups), for example, "C₆₋₁₂ aryl" refers to an aromatic cyclic hydrocarbon group having 6-12 (6, 7, 8, 9, 10, 11, or 12) ring carbon atoms, and "C₆₋₁₀ aryl" refers to an aromatic cyclic hydrocarbon group having 6-10 (6, 7, 8, 9, or 10) ring carbon atoms. When two or more aromatic rings are present (e.g., bicyclic, etc.), the aromatic rings of the aryl group may be linked by a single bond (e.g., biphenyl) or fused (e.g., naphthalene, anthracene, etc.). Examples of aryl (especially monocyclic and bicyclic groups) include, but are not limited to: phenyl, biphenyl, or naphthyl. Aryl groups can be fused to heterocyclyl groups via a single bond or any two adjacent ring carbon atoms, for example: benzotetrahydrofuranyl, benzotetrahydropyranyl, benzodioxolyl, 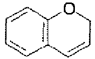 etc.

As used herein, the term "heteroaryl" refers to an aromatic cyclic group (including monocyclic, bicyclic, or polycyclic groups) containing 1, 2, 3, or 4 heteroatoms selected from N, S, or O in the ring skeleton, for example, "5-12 membered heteroaryl" refers to a monocyclic, bicyclic, or tricyclic group having 5 to 12 (5, 6, 7, 8, 9, 10, 11, or 12) ring atoms, and "5-10 membered heteroaryl", "5-8 membered heteroaryl", "6-10 membered heteroaryl" refer to monocyclic or bicyclic groups having 5 to 10 (5, 6, 7, 8, 9, 10, 11, or 12), 5-8, or 6-10 ring atoms, respectively. Examples of heteroaryl include, but are not limited to: imidazolyl, oxazolyl, isoxazolyl, thiazolyl, isothiazolyl, oxadiazolyl, thiadiazolyl, pyrazinyl, thienyl, furanyl, pyranyl, pyridyl, pyrrolyl, pyrazolyl, pyrimidinyl, quinolinyl, isoquinolinyl, benzofuranyl, benzothienyl, benzothiopyranyl, benzimidazolyl, benzoxazolyl, benzoxadiazolyl, benzothiazolyl, benzothiadiazolyl, benzopyranyl, indolyl, isoindolyl, triazolyl, triazinyl, quinoxalinyl, purinyl, quinazolinyl, quinolizinyl, naphthyridinyl, pteridinyl, carbazolyl, azepinyl, diazepinyl, acridinyl, etc.

As used herein, the term "substituted" means that one or more hydrogen atoms on a specific group are replaced by a specific substituent. The specific substituents are those described in the corresponding substituents above, or those appearing in the examples. Unless otherwise specified, a substituted group may have a substituent selected from a specific group at any substitutable position on the group, and said substituent may be the same or different at each position. Those skilled in the art will understand that combinations of substituents contemplated by the present invention are those that are stable or chemically feasible.

Unless otherwise specified as substituted or unsubstituted, the groups of the present invention may be substituted by a substituent selected from the group consisting of: D, halogen, cyano, nitro, hydroxy, amino, -C₁₋₆ alkyl, -C₂₋₆ alkenyl, -C₂₋₆ alkynyl, -C₁₋₆ alkoxy, 3-12 membered heterocyclyl, C₃-C₁₂ cycloalkyl, 5-10 membered heteroaryl, and -C₆₋₁₀ aryl.

As used herein, "optionally" means that the subsequently described event or circumstance may but need not occur, and that the description includes instances where the event or circumstance occurs and instances where it does not.

As used herein, the term "multiple" refers to 2, 3, 4, 5, or a positive integer greater than 5.

As used herein, the terms "α-synuclein aggregate", "α-syn aggregate", and "α-syn fibril" are synonymous.

As used herein, the terms "pathological aggregated protein" and "misfolded protein" are synonymous.

As used herein, "compound of the present invention" refers to the compound represented by Formula (A) and its various sub-generic formulas, and also includes its stereoisomers, optical isomers, pharmaceutically acceptable salts, crystalline forms, isotopic derivatives, prodrugs, metabolites, solvates, or hydrates thereof.

As used herein, the term "pharmaceutically acceptable salts" includes pharmaceutically acceptable acid addition salts and pharmaceutically acceptable base addition salts.

"Pharmaceutically acceptable acid addition salts" refer to salts formed with inorganic or organic acids that retain the biological effectiveness of the free base without other side effects. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, hexanoates, octanoates, decanoates, undecylenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, methanesulfonates, benzenesulfonates, p-toluenesulfonates, alginates, ascorbates, salicylates, 4-aminosalicylates, naphthalenedisulfonates, etc. These salts can be prepared by methods known in the art.

"Pharmaceutically acceptable base addition salts" refer to salts formed with inorganic or organic bases that retain the biological effectiveness of the free acid without other side effects. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts, and magnesium salts. Salts derived from organic bases include, but are not limited to, salts of primary, secondary, and tertiary amines, substituted amines, including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared by methods known in the art.

As used herein, the term "solvate" refers to a complex formed by a compound of Formula (A) and its various sub-generic formulas coordinated with solvent molecules in a specific ratio.

As used herein, the term "safe and effective amount" refers to an amount of the compound sufficient to treat or diagnose the disease without causing serious side effects.

As used herein, the term "pharmaceutically acceptable carrier" refers to one or more compatible solid or liquid fillers or gel materials, which are suitable for human use and must have sufficient purity and sufficiently low toxicity. "Compatibility" herein means that the components of the composition can be admixed with the compound of the present invention and with each other without significantly reducing the efficacy of the compound.

### Compounds of the Invention

Unless otherwise specified, the structural formulas described herein are intended to include all stereoisomers (such as cis-trans isomers, enantiomers, diastereomers, and conformers): R and S configurations for asymmetric centers, (Z) and (E) isomers for double bonds, cis-trans isomers for cycloalkanes, etc. Therefore, the individual stereochemical isomers of the compounds of the present invention or mixtures of their enantiomers, diastereomers, cis-trans isomers, or conformers fall within the scope of the present invention.

The compounds of the present invention may contain cis-trans isomers and/or one or more chiral carbon atoms, and thus may produce stereoisomeric forms such as cis-trans isomers, chiral isomers, enantiomers, diastereomers, and other combinations.

Cis-trans isomerism refers to a diastereoisomeric phenomenon arising from the different spatial arrangements of groups due to restricted free rotation within a compound molecule. This restriction is usually caused by functional groups in the organic compound structure that cannot rotate freely, such as C=C double bonds, C=N double bonds, C=S double bonds, N=N double bonds, heterocyclic rings, or cycloalkanes. Organic molecules containing such isomers, such as alkenes, azo compounds, alicyclic hydrocarbons, etc., are considered cis-trans isomers. Cis refers to the same ligand being in adjacent positions, generally indicated by "cis" or "cis-"; trans refers to the same ligand being in diagonal positions, generally indicated by "trans" or "trans-".

Each chiral carbon atom can be defined as (R)- or (S)- based on stereochemistry. The present invention is intended to include all possible isomers, as well as racemates, any proportion of enantiomer mixtures, or optically pure forms. It should be noted that when the compound of the present invention contains only one chiral center, reference to the racemate is equivalent to referring to both existing enantiomers, and those skilled in the art can prepare the individual enantiomers based on the disclosure in the context of the specification and conventional means in the art.

For the preparation of the compounds of the present invention, racemates, cis-trans isomers, chiral isomers, diastereomers, or enantiomers can be selected as starting materials or intermediates. Optically active isomers can be prepared using chiral synthons or chiral reagents, or resolved using conventional techniques, such as crystallization and chiral chromatography.

Conventional techniques for preparing/separating individual optical isomers (i.e., cis-trans isomers and chiral/enantiomers) include chiral synthesis from suitable cis-trans precursors or optically pure precursors, or resolution of racemates (or racemates of salts or derivatives) using, for example, chiral high-performance liquid chromatography.

To design the synthesis of a specific stereoisomer of a compound of the present invention, it can be prepared by asymmetric synthesis or by derivatization with a chiral auxiliary, separation of the resulting stereoisomeric mixture, and removal of the chiral auxiliary to obtain the pure cis-trans monomer, chiral monomer, or mixed stereoisomer. If the molecule contains a cis-trans isomer center, the pure cis or trans product can be obtained by column chromatography purification (normal phase silica gel column or reverse phase HPLC preparation); alternatively, if the molecule contains a basic functional group, such as an amino acid, or an acidic functional group, such as a carboxyl group, it can form diastereomeric salts with a suitable optically active acid or base, which are then separated by conventional means such as fractional crystallization or chromatography, yielding the pure enantiomer.

The present invention also includes isotopically labeled compounds (i.e., isotopic derivatives), which are equivalent to the original compounds disclosed herein. However, in reality, substitution of one or more atoms with an atom having a different atomic mass or mass number often occurs. Examples of isotopes in the isotopic derivatives of the present invention include isotopes of hydrogen, carbon, nitrogen, oxygen, phosphorus, sulfur, fluorine, and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl. Isotopic derivatives of the compounds of the present invention are all within the protection scope of the present invention.

In some embodiments, the compounds of the present invention are isotopically labeled with ¹⁸F, for example, as shown in some representative examples of the present invention. It should be noted that although some example compounds of the present application are not yet isotopically labeled, those skilled in the art can prepare the isotopically labeled forms (e.g., ¹⁸F-labeled forms) of these compounds based on the disclosure of the present application, and these compounds are also encompassed by the present application.

In some embodiments, the compounds of the present invention can be ³H-labeled and ¹⁴C-labeled compounds, which are useful in drug and substrate tissue distribution experiments. The preparation and detection of compounds labeled with tritium (i.e. ³H) and carbon-¹⁴ (i.e. ¹⁴C) are relatively easy and are the preferred isotopes.

In some embodiments, the compounds of the present invention may have heavier isotope substitution, such as deuterium, i.e., 2H, which has advantages in certain therapies due to its good metabolic stability, such as increasing half-life *in vivo* or reducing dosage, so it may be preferred in some cases.

Isotopically labeled compounds can be prepared using a general method, by replacing the non-isotopic reagent with a readily available isotopically labeled reagent using the schemes disclosed in the examples.

Metabolic products of the compounds of Formula (A) and its various sub-generic formulas and their pharmaceutically acceptable salts, as well as prodrugs that can be converted *in vivo* to the compounds of Formula (A) and its various sub-generic formulas and their pharmaceutically acceptable salts, are also included within the protection scope of the present invention.

Specifically, the present invention provides the following compound embodiments:
**Embodiment 1:** A compound of Formula A, or a stereoisomer, pharmaceutically acceptable salt, solvate, or stable isotopic variant thereof, 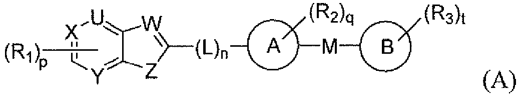 wherein,
X, Y, and U are each independently selected from CH and N, and when any one of them is CH, the hydrogen atom on said CH can be replaced by R₁;
W is selected from CH and N;
Z is selected from CH₂, NH, O, and S;
L is selected from -CH=CH- and -C=C-, and when n is greater than 1, -(L)ₙ- is a chain formed by connecting Ls that are the same or different;
Ring A is selected from C₆₋₈ cycloalkane, C₆₋₁₂ aromatic ring, 6-10 membered heteroaromatic ring, and 6-8 membered heterocyclic ring;
Ring B is selected from C₆₋₁₂ aromatic ring, C₄₋₈ cycloalkane, 5-10 membered heteroaromatic ring, and 4-8 membered heterocyclic ring;
M is a direct bond or a 4-8 membered heterocyclic ring;
R₁ are each independently selected from: deuterium, tritium, hydroxy, amino, halogen, nitro, cyano, -COOH, -C₁₋₆ alkyl, -C₂₋₆ alkenyl, -C₂₋₆ alkynyl, -O-C₁₋₆ alkyl, -NHC₁₋₆ alkyl, -N(C₁₋₆ alkyl)₂, -SO₂-C₁₋₆ alkyl, acyl, ester, amide, sulfonamide, -NH-C₃₋₈ cycloalkyl, -N(C₁₋₆ alkyl)(C₃₋₈ cycloalkyl), -C₃₋₈ cycloalkyl, 3-8 membered heterocyclyl, 5-10 membered heteroaryl,
wherein the -C₁₋₆ alkyl or the C₁₋₆ alkyl as part of a group, -C₃₋₈ cycloalkyl or the -C₃₋₈ cycloalkyl as part of a group, 3-8 membered heterocyclyl, and 5-10 membered heteroaryl in R₁ are each independently optionally substituted by one or more substituents, said substituents are each independently selected from deuterium, tritium, -O-C₁₋₆ alkyl, -C₁₋₆ alkyl, halogen, hydroxy, oxo, - NHC₁₋₆ alkyl, -N(C₁₋₆ alkyl)₂, -O-(3-6 membered heterocyclyl), -O-(p-toluenesulfonyl), and two substituents attached to the same carbon atom optionally together with the carbon atom to which they are attached form a 3-6 membered heterocyclic ring;
R₂ and R₃ are each independently selected from: deuterium, tritium, hydroxy, amino, halogen, nitro, cyano, -COOH, -C₁₋₆ alkyl, -C₂₋₆ alkenyl, -C₂₋₆ alkynyl, -O-C₁₋₆ alkyl, -NHC₁₋₆ alkyl, -N(C₁₋₆ alkyl)₂, -SO₂-C₁₋₆ alkyl, acyl, ester, amide, sulfonamide, -NH-C₃₋₈ cycloalkyl, -N(C₁₋₆ alkyl)(C₃₋₈ cycloalkyl), -C₃₋₈ cycloalkyl, 3-8 membered heterocyclyl, 5-10 membered heteroaryl, and C₆₋₁₂ aryl, wherein the -C₃₋₈ cycloalkyl, 3-8 membered heterocyclyl, 5-10 membered heteroaryl, and C₆₋₁₂ aryl each independently are optionally substituted by halogen, hydroxy, -OC₁₋₆ alkyl, -NHC₁₋₆ alkyl, or - N(C₁₋₆ alkyl)₂,
wherein the -C₁₋₆ alkyl or the C₁₋₆ alkyl as part of another group or substituent in R₂ and R₃ is optionally substituted by one or more substituents each independently selected from: deuterium, tritium, halogen, hydroxy, -O-(3-6 membered heterocyclyl), -O-(p-toluenesulfonyl);
n is an integer from 0 to 3;
p is an integer from 1 to 4;
q and t are each independently integers from 0 to 5;
wherein the heteroaryl and heterocyclyl each independently contain 1, 2, 3, or 4 heteroatoms selected from N, S, or O; and
wherein each occurrence of halogen is optionally in its isotopic form, and the carbon atoms and/or hydrogen atoms in the substituents are optionally in their isotopic forms.

**Embodiment 2:** The compound according to any preceding embodiment, or a stereoisomer, pharmaceutically acceptable salt, solvate, or stable isotopic variant thereof, wherein the acyl, ester, amide, sulfonamide are selected from -CO-C₁₋₆ alkyl, -C(O)OC₁₋₆ alkyl, -C(O)NH₂, -C(O)NHC₁₋₆ alkyl, -C(O)N(C₁₋₆ alkyl)₂, -NHCO-C₁₋₆ alkyl, -N(C₁₋₆ alkyl)-CO-C₁₋₆ alkyl, -SO₂NH₂, -SO₂NHC₁₋₆ alkyl, -SO₂N(C₁₋₆ alkyl)₂, -NHSO₂-C₁₋₆ alkyl, -N(C₁₋₆ alkyl)-SO₂-C₁₋₆ alkyl, and depending on the substituent where it is located (e.g., R₁, R₂, or R₃), wherein the -C₁₋₆ alkyl is optionally substituted by the corresponding substituents as defined in the preceding embodiments.
**Embodiment 3:** The compound according to any preceding embodiment, or a stereoisomer, pharmaceutically acceptable salt, solvate, or stable isotopic variant thereof, wherein W is selected from CH and N; and Z is selected from O and S.
**Embodiment 4:** The compound according to any preceding embodiment, or a stereoisomer, pharmaceutically acceptable salt, solvate, or stable isotopic variant thereof, wherein X, Y, and U are all CH, or one or two of X, Y, and U are N and the rest are CH.
**Embodiment 5:** The compound according to any preceding embodiment, or a stereoisomer, pharmaceutically acceptable salt, solvate, or stable isotopic variant thereof, wherein the fused ring containing X, Y, U, W, and Z is selected from: 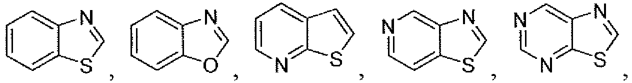 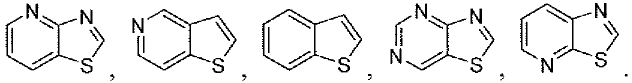
**Embodiment 6:** The compound according to any preceding embodiment, or a stereoisomer, pharmaceutically acceptable salt, solvate, or stable isotopic variant thereof, wherein p is an integer from 1 to 2, preferably p is 1.
**Embodiment 7.1:** The compound according to any preceding embodiments 1-6, or a stereoisomer, pharmaceutically acceptable salt, solvate, or stable isotopic variant thereof, wherein R₁ can be halogen, nitro, or cyano, such as halogen and nitro, e.g., F, Br, NO₂.
**Embodiment 7.2:** The compound according to any preceding embodiments 1-6, or a stereoisomer, pharmaceutically acceptable salt, solvate, or stable isotopic variant thereof, wherein R₁ can be hydroxy or -O-C₁₋₆ alkyl, wherein the -C₁₋₆ alkyl is optionally substituted by one or more substituents, e.g., 1 or 2 substituents, said substituents as defined in Embodiment 1, e.g., each independently selected from halogen, hydroxy, -O-(3-6 membered heterocyclyl), -O-(p-toluenesulfonyl), wherein the halogen is preferably F or 18F, and the -O-(3-6 membered heterocyclyl) is preferably -O-THP.
**Embodiment 7.3:** The compound according to any preceding embodiments 1-6, or a stereoisomer, pharmaceutically acceptable salt, solvate, or stable isotopic variant thereof, wherein R₁ can be amino, -NHC₁₋₆ alkyl, -N(C₁₋₆ alkyl)₂, -NHCO-C₁₋₆ alkyl, -N(C₁₋₆ alkyl)-CO-C₁₋₆ alkyl, - NHSO₂-C₁₋₆ alkyl, -N(C₁₋₆ alkyl)-SO₂-C₁₋₆ alkyl, -NH-C₃₋₈ cycloalkyl, -N(C₁₋₆ alkyl)(C₃₋₈ cycloalkyl), e.g., R₁ can be -NHC₁₋₆ alkyl, -N(C₁₋₆ alkyl)₂, -NHCO-C₁₋₆ alkyl, or -N(C₁₋₆ alkyl)-CO-C₁₋₆ alkyl,
wherein the -C₁₋₆ alkyl is optionally substituted by one or more substituents, e.g., 1 or 2 substituents, said substituents as defined in Embodiment 1, e.g., each independently selected from halogen, hydroxy, -O-(3-6 membered heterocyclyl), -O-(p-toluenesulfonyl), wherein the halogen is preferably F or 18F, and the -O-(3-6 membered heterocyclyl) is preferably -O-THP.
**Embodiment 7.4:** The compound according to any preceding embodiments 1-6, or a stereoisomer, pharmaceutically acceptable salt, solvate, or stable isotopic variant thereof, wherein R₁ can be -COOH, -SO₂-C₁₋₆ alkyl, -CO-C₁₋₆ alkyl, -C(O)OC₁₋₆ alkyl, -C(O)NH₂, -C(O)NHC₁₋₆ alkyl, - C(O)N(C₁₋₆ alkyl)₂, -NHCO-C₁₋₆ alkyl, -N(C₁₋₆ alkyl)-CO-C₁₋₆ alkyl, -SO₂NH₂, -SO₂NHC₁₋₆ alkyl, - SO₂N(C₁₋₆ alkyl)₂, -NHSO₂-C₁₋₆ alkyl, or -N(C₁₋₆ alkyl)-SO₂-C₁₋₆ alkyl, e.g., R₁ can be -COOH or - NHCO-C₁₋₆ alkyl,
wherein the -C₁₋₆ alkyl is optionally substituted by one or more substituents, e.g., 1 or 2 substituents, said substituents as defined in Embodiment 1, e.g., each independently selected from halogen, hydroxy, -O-(3-6 membered heterocyclyl), -O-(p-toluenesulfonyl), wherein the halogen is preferably F or 18F, and the -O-(3-6 membered heterocyclyl) is preferably -O-THP.
**Embodiment 7.5:** The compound according to any preceding embodiments 1-6, or a stereoisomer, pharmaceutically acceptable salt, solvate, or stable isotopic variant thereof, wherein R_{f} can be -C₃₋₈ cycloalkyl, 3-8 membered heterocyclyl, 5-10 membered heteroaryl, e.g., R₁ can be -C₃₋₆ cycloalkyl, 5-8 membered heterocyclyl, or 5-6 membered heteroaryl, e.g., 5-8 membered heterocyclyl; each independently optionally substituted by one or more substituents, said substituents as defined in Embodiment 1, e.g., each independently selected from -O-C₁₋₆ alkyl, -C₁₋₆ alkyl, halogen, hydroxy, oxo, -NHC₁₋₆ alkyl, -N(C₁₋₆ alkyl)₂.
**Embodiment 7.6:** The compound according to any preceding embodiments 1-6, or a stereoisomer, pharmaceutically acceptable salt, solvate, or stable isotopic variant thereof, wherein R₁ are each independently selected from: halogen, nitro, hydroxy, -O-C₁₋₆ alkyl, -NHC₁₋₆ alkyl, -N(C₁₋₆ alkyl)₂, -COOH, -NHCO-C₁₋₆ alkyl, -N(C₁₋₆ alkyl)-CO-C₁₋₆ alkyl, and 5-8 membered heterocyclyl, wherein the -C₁₋₆ alkyl or the C₁₋₆ alkyl as part of a groupand the 5-8 membered heterocyclyl in R₁ are each independently optionally substituted by one or more, e.g., 1-2 substituents, said substituents are each independently selected from deuterium, tritium, -O-C₁₋₆ alkyl, -C₁₋₆ alkyl, halogen, hydroxy, oxo, -NHC₁₋₆ alkyl, -N(C₁₋₆ alkyl)₂, -O-(3-6 membered heterocyclyl), -O-(p-toluenesulfonyl), wherein the halogen appearing in R₁ is optionally in isotopic form, e.g., 18F.
**Embodiment 7.7:** The compound according to any preceding embodiments 1-6, or a stereoisomer, pharmaceutically acceptable salt, solvate, or stable isotopic variant thereof, wherein R₁ are each independently selected from halogen, nitro, hydroxy, -O-C₁₋₆ alkyl, -O-C₁₋₆ haloalkyl, -O-C₁₋₆ halohydroxyalkyl, -O-C₁₋₆ alkyl substituted by O-(3-6 membered heterocyclyl) and/or -O-(p-toluenesulfonyl), -NHC₁₋₆ alkyl, -NHC₁₋₆ haloalkyl, -NHC₁₋₆ halohydroxyalkyl, -NH-C₁₋₆ alkyl substituted by O-(3-6 membered heterocyclyl) and/or -O-(p-toluenesulfonyl), -N(C₁₋₆ alkyl)₂, - COOH, -NHCO-C₁₋₆ alkyl, -N(C₁₋₆ alkyl)-CO-C₁₋₆ alkyl, and 5-6 membered heterocyclyl, wherein the 5-6 membered heterocyclyl is optionally substituted by one or more, e.g., 1-2 substituents, said substituents are each independently selected from deuterium, tritium, -O-C₁₋₆ alkyl, -C₁₋₆ alkyl, halogen, hydroxy, oxo, -NHC₁₋₆ alkyl, -N(C₁₋₆ alkyl)₂,
preferably R₁ is selected from -O-C₁₋₆ halohydroxyalkyl and -NHC₁₋₆ halohydroxyalkyl;
more preferably R₁ is selected from -O-C₁₋₆ halohydroxyalkyl;
wherein the halogen appearing in R₁ is optionally in isotopic form, e.g., 18F.

**Embodiment 7.8:** The compound according to any preceding embodiments 1-6, or a stereoisomer, pharmaceutically acceptable salt, solvate, or stable isotopic variant thereof, wherein p is 2, one of R₁ is selected from H and halogen, and the other is selected from hydroxy, -O-C₁₋₆ alkyl, -O-methyl, -O-C₁₋₆ haloalkyl, -O-C₁₋₆ halohydroxyalkyl, NH₂, -NHC₁₋₆ alkyl, -NHCH₃, -NHC₁₋₆ haloalkyl, -NHC₁₋₆ halohydroxyalkyl, -N(C₁₋₆ alkyl)₂, -N(CH₃)(C₁₋₆ alkyl), -N(C₁₋₆ alkyl)(C₁₋₆ haloalkyl), -N(C₁₋₆ alkyl)(C₁₋₆ halohydroxyalkyl), -N(CH₃)(C₁₋₆ halohydroxyalkyl), -N(C₁₋₆ haloalkyl)(C₁₋₆ haloalkyl), -N(C₁₋₆ haloalkyl)(C₁₋₆ halohydroxyalkyl), -NH(C₃₋₈ cycloalkyl), -N(C₃₋₈ cycloalkyl)(C₁₋₆ alkyl), -N(C₃₋₈ cycloalkyl)(CH₃), -N(C₃₋₈ cycloalkyl)(C₁₋₆ haloalkyl), -N(C₃₋₈ cycloalkyl)(C₁₋₆ halohydroxyalkyl), wherein the alkyl is optionally substituted by 1-3 -O-(3-6 membered heterocyclyl) or -O-(p-toluenesulfonyl).
**Embodiment 7.9:** The compound according to any preceding embodiments 1-6, or a stereoisomer, pharmaceutically acceptable salt, solvate, or stable isotopic variant thereof, wherein p is 1, and R₁ is selected from -O-C₁₋₆ haloalkyl, -O-C₁₋₆ halohydroxyalkyl, -N(C₁₋₆ alkyl)(C₁₋₆ haloalkyl), -N(C₁₋₆ alkyl)(C₁₋₆ halohydroxyalkyl), -N(C₁₋₆ haloalkyl)(C₁₋₆ haloalkyl), -N(C₁₋₆ haloalkyl)(C₁₋₆ halohydroxyalkyl), -N(C₃₋₈ cycloalkyl)(C₁₋₆ haloalkyl), -N(C₃₋₈ cycloalkyl)(C₁₋₆ halohydroxyalkyl).
**Embodiment 7.10:** The compound according to any preceding embodiments 1-6, or a stereoisomer, pharmaceutically acceptable salt, solvate, or stable isotopic variant thereof, wherein R₁ are each independently selected from: F, Br, 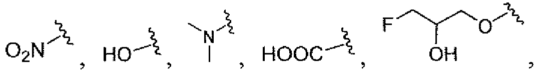 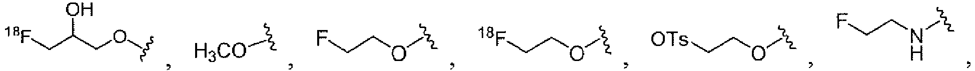 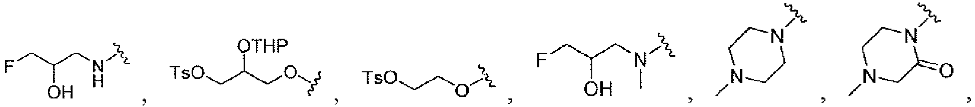 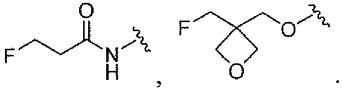
**Embodiment 7.11:** The compound according to any preceding embodiments 1-6, or a stereoisomer, pharmaceutically acceptable salt, solvate, or stable isotopic variant thereof, wherein R₁ are each independently selected from: 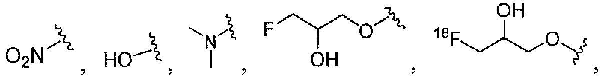 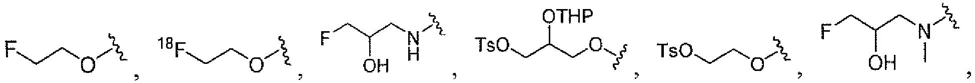 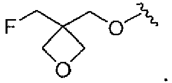
**Embodiment 7.12:** The compound according to any preceding embodiments 1-6, or a stereoisomer, pharmaceutically acceptable salt, solvate, or stable isotopic variant thereof, wherein R₁ are each independently selected from: 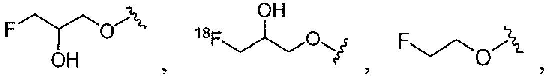 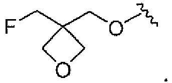
**Embodiment 7.13:** The compound according to any preceding embodiments 1-6, or a stereoisomer, pharmaceutically acceptable salt, solvate, or stable isotopic variant thereof, wherein R₁ is selected from -O-C₁₋₆ halohydroxyalkyl and -NHC₁₋₆ halohydroxyalkyl, e.g., -O-C₁₋₆-C₁₋₆ halohydroxyalkyl, specifically selected from 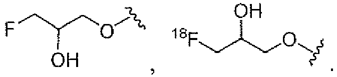
**Embodiment 7.14:** The compound according to any preceding embodiment, or a stereoisomer, pharmaceutically acceptable salt, solvate, or stable isotopic variant thereof, wherein p is 1, and R₁ is located on the ring carbon atom adjacent to Y; or p is 2, and R₁ are attached respectively to Y and the ring carbon atom adjacent to Y, or R₁ are attached respectively to X and the ring carbon atom between X and Y;
preferably p is 1, and R₁ is located on the ring carbon atom adjacent to Y.
**Embodiment 7.15:** The compound according to any preceding embodiment, or a stereoisomer, pharmaceutically acceptable salt, solvate, or stable isotopic variant thereof, wherein when R₁ simultaneously carries a hydroxy group and a halogen, the hydroxy group and the halogen are attached to different skeletal atoms, for example, different carbon atoms of a C₁₋₆ alkyl group, preferably attached to adjacent carbon atoms.
**Embodiment 8.1:** The compound according to any preceding embodiment, or a stereoisomer, pharmaceutically acceptable salt, solvate, or stable isotopic variant thereof, wherein: n=0.
**Embodiment 8.2:** The compound according to any preceding embodiment, or a stereoisomer, pharmaceutically acceptable salt, solvate, or stable isotopic variant thereof, wherein: n is 1, and L is -CH=CH-, or L is -C=C-.
**Embodiment 8.3:** The compound according to any preceding embodiment, or a stereoisomer, pharmaceutically acceptable salt, solvate, or stable isotopic variant thereof, wherein n is 2, and (L)₂ is -CH=CH-CH=CH-, -CH=CH-C=C-, -C=C-CH=CH-, or -C≡C-C≡C-, preferably -CH=CH-CH=CH- or -CH=CH-C=C-.
**Embodiment 8.4:** The compound according to any preceding embodiment, or a stereoisomer, pharmaceutically acceptable salt, solvate, or stable isotopic variant thereof, wherein n is 3, and (L)₃ is -CH=CH-CH=CH-CH=CH-, -CH=CH-CH=CH-C=C-, -CH=CH-C=C-CH=CH-, -C=C-CH=CH-CH=CH-, -CH=CH-C=C-C=C-, -C=C-CH=CH-C=C-, or -C=C-C=C-CH=CH-, preferably - CH=CH-CH=CH-CH=CH-, -CH=CH-CH=CH-C=C-, -CH=CH-C=C-CH=CH-, or -CH=CH-C≡C-C=C-.
**Embodiment 9:** The compound according to any preceding embodiment, or a stereoisomer, pharmaceutically acceptable salt, solvate, or stable isotopic variant thereof, wherein Formula (A) has the following sub-generic formulas: 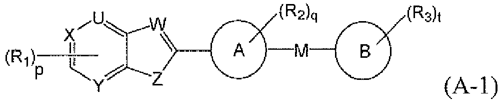 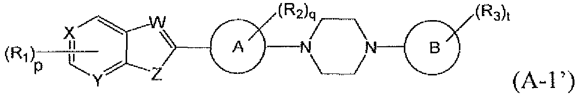 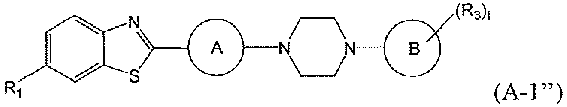 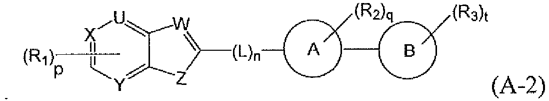 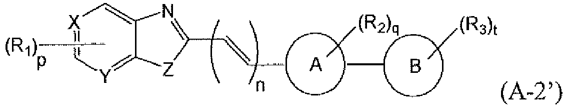 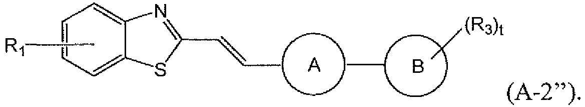
**Embodiment 10.1:** The compound according to any preceding embodiments 1-9, or a stereoisomer, pharmaceutically acceptable salt, solvate, or stable isotopic variant thereof, wherein Ring A is a C₆₋₁₂ aromatic ring, e.g., a C6-10 aromatic ring, e.g., a benzene ring.
**Embodiment 10.2:** The compound according to any preceding embodiments 1-9, or a stereoisomer, pharmaceutically acceptable salt, solvate, or stable isotopic variant thereof, wherein Ring A is a 6-10 membered heteroaromatic ring, e.g., a 6-membered heteroaromatic ring containing 1 or 2 nitrogen heteroatoms, e.g., 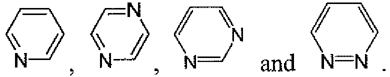
**Embodiment 10.3:** The compound according to any preceding embodiments 1-9, or a stereoisomer, pharmaceutically acceptable salt, solvate, or stable isotopic variant thereof, wherein Ring A is a 6-8 membered heterocyclic ring, e.g., a 6-membered heterocyclic ring containing 1 or 2 nitrogen heteroatoms, e.g., 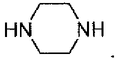
**Embodiment 10.4:** The compound according to any preceding embodiments 1-9, or a stereoisomer, pharmaceutically acceptable salt, solvate, or stable isotopic variant thereof, wherein Ring A is selected from a C₆₋₁₂ aromatic ring, a 6-10 membered heteroaromatic ring, and a 6-8 membered heterocyclic ring, e.g., Ring A is selected from a C₆₋₁₀ aromatic ring, a 6-8 membered heteroaromatic ring, and a 6-8 membered heterocyclic ring, e.g., Ring A is selected from a benzene ring and a 6-membered heteroaromatic ring or a 6-membered heterocyclic ring containing 1 or 2 nitrogen heteroatoms, e.g., Ring A is selected from a benzene ring and a 6-membered heteroaromatic ring containing 1 or 2 nitrogen heteroatoms, e.g., A is selected from 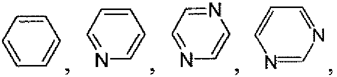 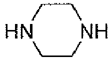 and 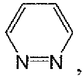 preferably Ring A is selected from 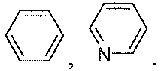
**Embodiment 10.5:** The compound according to any preceding embodiments 1-9, or a stereoisomer, pharmaceutically acceptable salt, solvate, or stable isotopic variant thereof, wherein Ring A is selected from a C₆₋₈ aromatic ring and 6-8 membered heteroaromatic ring, e.g., Ring A is selected from 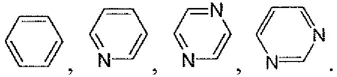
**Embodiment 10.6:** The compound according to any preceding embodiments 1-9, or a stereoisomer, pharmaceutically acceptable salt, solvate, or stable isotopic variant thereof, wherein Ring A is selected from a C₆₋₈ aromatic ring and a 6-8 membered heteroaromatic ring, e.g., Ring A is selected from 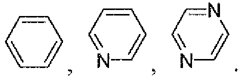
**Embodiment 10.7:** The compound according to any preceding embodiments 1-9, or a stereoisomer, pharmaceutically acceptable salt, solvate, or stable isotopic variant thereof, wherein Ring A is selected from a C₆₋₈ aromatic ring and a 6-8 membered heteroaromatic ring, e.g., Ring A is selected from 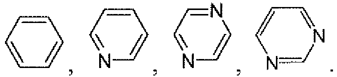
**Embodiment 10.8:** The compound according to any preceding embodiment, or a stereoisomer, pharmaceutically acceptable salt, solvate, or stable isotopic variant thereof, wherein A is attached to the rest of the molecule via ring carbon atoms, or when Ring A is a heterocycle, also via ring heteroatom(s).
**Embodiment 11.1:** The compound according to any preceding embodiment, or a stereoisomer, pharmaceutically acceptable salt, solvate, or stable isotopic variant thereof, wherein Ring A is optionally substituted by 0-5 of said substituents R₂, e.g., q is 0-4, 0-3, 0-2, 0-1, 1, preferably q is 0.
**Embodiment 11.2:** The compound according to any preceding embodiment, or a stereoisomer, pharmaceutically acceptable salt, solvate, or stable isotopic variant thereof, wherein Ring A is optionally substituted by one R₂, R₂ is selected from halogen, nitro, cyano, and halogen-substituted - C₁₋₆ alkyl, preferably selected from halogen, nitro, and cyano, more preferably R₂ is selected from halogen or an isotope thereof, e.g., R₂ is selected from F and 18F.
**Embodiment 12.1:** The compound according to any preceding embodiment, or a stereoisomer, pharmaceutically acceptable salt, solvate, or stable isotopic variant thereof, wherein Ring B is selected from a C₆₋₁₂ aromatic ring, e.g., a C₆₋₁₀ aromatic ring, e.g., a benzene ring or a naphthalene ring.
**Embodiment 12.2:** The compound according to any preceding embodiment, or a stereoisomer, pharmaceutically acceptable salt, solvate, or stable isotopic variant thereof, wherein Ring B is selected from a 5-10 membered heteroaromatic ring, e.g., 5-8 membered heteroaryl, further e.g., a 5-6 membered heteroaromatic ring containing 1-3, e.g., 1-2 heteroatoms selected from nitrogen, oxygen, and sulfur; e.g., B is selected from 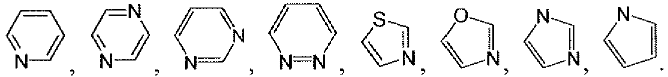
**Embodiment 12.3:** The compound according to any preceding embodiment, or a stereoisomer, pharmaceutically acceptable salt, solvate, or stable isotopic variant thereof, wherein Ring B is selected from a C₆₋₁₂ aromatic ring and a 5-10 membered heteroaromatic ring, e.g., a C₆₋₁₀ aromatic ring and a 5-8 membered heteroaromatic ring, e.g., Ring B is selected from a benzene ring, a naphthalene ring, and a 5-6 membered heteroaromatic ring containing 1-3, e.g., 1-2 heteroatoms selected from nitrogen, oxygen, and sulfur, e.g., B is selected from 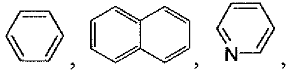 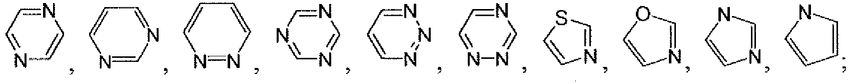 preferably Ring B is selected from 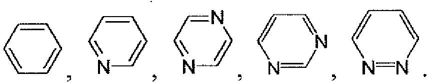
**Embodiment 12.4:** The compound according to any preceding embodiment, or a stereoisomer, pharmaceutically acceptable salt, solvate, or stable isotopic variant thereof, wherein Ring B is selected from a benzene ring and a 5-8 membered heteroaromatic ring, e.g., Ring B is selected from 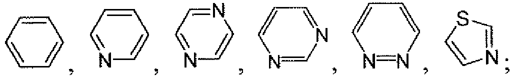
or for example Ring B is selected from 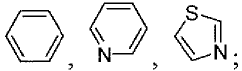
or Ring B is a 6-membered heteroaromatic ring containing 2 or 3 heteroatoms selected from N, S, or O, e.g.,

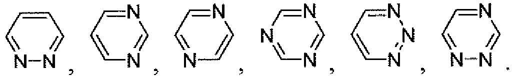

**Embodiment 13.1:** The compound according to any preceding embodiment, or a stereoisomer, pharmaceutically acceptable salt, solvate, or stable isotopic variant thereof, wherein Ring B is optionally substituted by 0-5 of said substituents R₃, e.g., t is 0-4, 0-3, 0-2, 0-1, 0, 1-2, preferably t is 1.
**Embodiment 13.2:** The compound according to any preceding embodiment, or a stereoisomer, pharmaceutically acceptable salt, solvate, or stable isotopic variant thereof, wherein Ring B is optionally substituted by 1 or 2 R₃, R₃ is selected from hydroxy, amino, halogen, nitro, cyano, -C₁₋₆ alkyl, -O-C₁₋₆ alkyl, -NHC₁₋₆ alkyl, -N(C₁₋₆ alkyl)₂, -C₃₋₈ cycloalkyl, 5-8 membered heterocyclyl, and C₆₋₁₀ aryl; wherein said -C₃₋₈ cycloalkyl, 5-8 membered heterocyclyl, and C₆₋₁₀ aryl are preferably - C₃₋₆ cycloalkyl, 5-7 membered heterocyclyl, and C₆ aryl, respectively, each independently optionally substituted by halogen, hydroxy, -OC₁₋₆ alkyl, -NHC₁₋₆ alkyl, or -N(C₁₋₆ alkyl)₂;
wherein the -C₁₋₆ alkyl appearing in R₃ is optionally substituted by 1 or 2 substituents each independently selected from: deuterium, tritium, halogen, hydroxy, -O-(3-6 membered heterocyclyl), and -O-(p-toluenesulfonyl), e.g., deuterium, tritium, halogen, and hydroxy, e.g., halogen or an isotope thereof (e.g., F or 18F), and hydroxy.
**Embodiment 13.3:** The compound according to any preceding embodiment, or a stereoisomer, pharmaceutically acceptable salt, solvate, or stable isotopic variant thereof, wherein Ring B is optionally substituted by 1 or 2 R₃, R₃ is selected from hydroxy, amino, halogen, nitro, cyano, -C₁₋₆ alkyl, -O-C₁₋₆ alkyl, -NHC₁₋₆ alkyl, -N(C₁₋₆ alkyl)₂, 5-7 membered heterocyclyl, and C₆ aryl; wherein said 5-7 membered heterocyclyl and C₆ aryl are each independently optionally substituted by -NHC₁₋₆ alkyl or -N(C₁₋₆ alkyl)₂;
wherein the -C₁₋₆ alkyl appearing in R₃ is optionally substituted by 1 or 2 substituents each independently selected from: halogen or an isotope thereof (e.g., F or 18F), and hydroxy.
**Embodiment 13.4:** The compound according to any preceding embodiment, or a stereoisomer, pharmaceutically acceptable salt, solvate, or stable isotopic variant thereof, wherein Ring B is optionally substituted by 1 or 2 R₃, R₃ is selected from: hydroxy, amino, F, Br, nitro, cyano, -O-CH₃, -NHCH₃, -N(CH₃)₂, 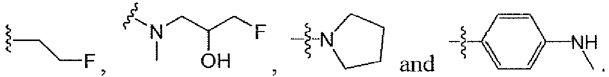
**Embodiment 13.5:** The compound according to any preceding embodiment, or a stereoisomer, pharmaceutically acceptable salt, solvate, or stable isotopic variant thereof, wherein Ring B is substituted by one substituent R₃, R₃ is selected from -NHC₁₋₆ alkyl, e.g., -NHCH₃.
**Embodiment 14.1:** The compound according to any preceding embodiment, or a stereoisomer, pharmaceutically acceptable salt, solvate, or stable isotopic variant thereof, wherein M is a 6-8 membered nitrogen-containing heterocyclic ring, e.g., a 6-membered nitrogen-containing heterocyclic ring, e.g., 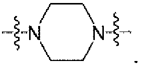
**Embodiment 14.2:** The compound according to any preceding embodiment, or a stereoisomer, pharmaceutically acceptable salt, solvate, or stable isotopic variant thereof, wherein n is 0, and M is a 6-membered nitrogen-containing heterocyclic ring, e.g., 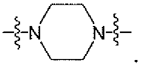
**Embodiment 15:** The compound according to any of embodiments 1-9, or a stereoisomer, pharmaceutically acceptable salt, solvate, or stable isotopic variant thereof, having the following formula: 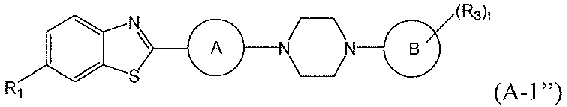
wherein each group and variable is as defined in the corresponding preceding embodiments, specifically
R₁ is selected from halogen, nitro, hydroxy, -O-C₁₋₆ alkyl, -O-C₁₋₆ haloalkyl, -O-C₁₋₆ halohydroxyalkyl, -O-C₁₋₆ alkyl substituted by O-(3-6 membered heterocyclyl) and/or -O-(p-toluenesulfonyl), -NHC₁₋₆ alkyl, -NHC₁₋₆ haloalkyl, -NHC₁₋₆ halohydroxyalkyl, -NH-C₁₋₆ alkyl substituted by O-(3-6 membered heterocyclyl) and/or -O-(p-toluenesulfonyl), -N(C₁₋₆ alkyl)₂, - COOH, -NHCO-C₁₋₆ alkyl, -N(C₁₋₆ alkyl)-CO-C₁₋₆ alkyl, and 5-6 membered heterocyclyl, wherein the 5-6 membered heterocyclyl is optionally substituted by one or more substituents, said substituents are each independently selected from deuterium, tritium, -O-C₁₋₆ alkyl, -C₁₋₆ alkyl, halogen, hydroxy, oxo, -NHC₁₋₆ alkyl, -N(C₁₋₆ alkyl)₂, wherein the halogen appearing in R₁ is optionally in isotopic form, e.g., 18F;
Ring A is selected from a C₆₋₁₂ aromatic ring and a 6-10 membered heteroaromatic ring;
Ring B is selected from a C₆₋₁₂ aromatic ring and a 5-10 membered heteroaromatic ring;
R₃ is selected from hydroxy, amino, halogen, nitro, cyano, -O-C₁₋₆ alkyl, -NHC₁₋₆ alkyl, -N(C₁₋₆ alkyl)₂, 4-7 membered heterocyclyl;
t is an integer from 0 to 2,
wherein the heteroaryl and heterocyclyl each independently contain 1, 2, or 3 heteroatoms selected from N, S, or O; and
wherein each occurrence of halogen is optionally in its isotopic form, and the carbon atoms and/or hydrogen atoms in the substituents are optionally in their isotopic forms;

**Embodiment 15.1:** The compound according to embodiment 15, or a stereoisomer, pharmaceutically acceptable salt, solvate, or stable isotopic variant thereof, wherein:
R₃ is selected from -O-C₁₋₆ halohydroxyalkyl and -NHC₁₋₆ halohydroxyalkyl, preferably R₁ is selected from -O-C₁₋₆ halohydroxyalkyl;
Ring A is selected from a benzene ring and a 6-membered heteroaromatic ring containing 1 or 2 nitrogen heteroatoms;
Ring B is selected from a benzene ring and a 5-6 membered heteroaromatic ring containing 1 or 2 heteroatoms selected from nitrogen, oxygen, and sulfur;
R₃ is selected from -NHC₁₋₆ alkyl;
t is 1;
wherein each occurrence of halogen is preferably F or 18F, and the carbon atoms and/or hydrogen atoms in the substituents are optionally in their isotopic forms.

**Embodiment 15.2:** A compound of the following formula, or a stereoisomer, pharmaceutically acceptable salt, solvate, or stable isotopic variant thereof, 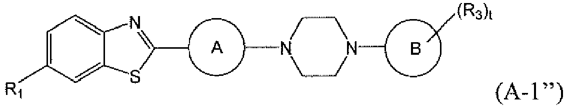 wherein:
R₁ is selected from hydroxy, -O-C₁₋₆ alkyl, -O-methyl, -O-C₁₋₆ haloalkyl, -O-C₁₋₆ halohydroxyalkyl, NH₂, -NHC₁₋₆ alkyl, -NHCH₃, -NHC₁₋₆ haloalkyl, -NHC₁₋₆ halohydroxyalkyl, - N(C₁₋₆ alkyl)₂, -N(CH₃)(C₁₋₆ alkyl), -N(C₁₋₆ alkyl)(C₁₋₆ haloalkyl), -N(C₁₋₆ alkyl)(C₁₋₆ halohydroxyalkyl), -N(CH₃)(C₁₋₆ halohydroxyalkyl), -N(C₁₋₆ haloalkyl)(C₁₋₆ haloalkyl), -N(C₁₋₆ haloalkyl)(C₁₋₆ halohydroxyalkyl), -NH(C₃₋₈ cycloalkyl), -N(C₃₋₈ cycloalkyl)(C₁₋₆ alkyl), -N(C₃₋₈ cycloalkyl)(CH₃), -N(C₃₋₈ cycloalkyl)(C₁₋₆ haloalkyl), -N(C₃₋₈ cycloalkyl)(C₁₋₆ halohydroxyalkyl), wherein the alkyl is optionally substituted by 1-3 -O-(3-6 membered heterocyclyl) or -O-(p-toluenesulfonyl);
Ring A is 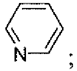
Ring B is selected from a C₆₋₁₂ aromatic ring, a C₄₋₈ cycloalkane, a 5-8 membered heteroaromatic ring, and a 4-8 membered heterocyclic ring;
R₃ is selected from deuterium, tritium, hydroxy, amino, halogen, nitro, cyano, carboxyl, C₁₋₆ alkyl, C₁₋₆ haloalkyl, C₁₋₆ hydroxyalkyl, C₂₋₆ alkenyl, C₂₋₆ alkynyl, -C₁₋₆ alkoxy, C₁₋₆ haloalkoxy, C₁₋₆ alkylamino, C₁₋₆ haloalkylamino, methylsulfonyl, C₂₋₆ acyl, C₂₋₆ ester, C₂₋₆ amide, C₂₋₆ sulfonamide, C₁₋₆ halohydroxyalkoxy, C₃₋₈ cycloalkyl, 3-8 membered heterocyclyl, 5-8 membered heteroaryl.
t is an integer from 0 to 5;
wherein each occurrence of halogen is optionally in isotopic form.

**Embodiment 16:** The compound according to any of embodiments 1-9, or a stereoisomer, pharmaceutically acceptable salt, solvate, or stable isotopic variant thereof, having the following formula: 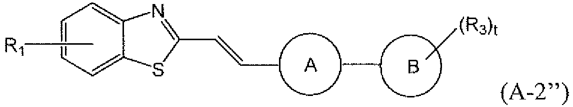
wherein each group and variable is as defined in the corresponding preceding embodiments, specifically
R1 is selected from halogen, nitro, hydroxy, -O-C₁₋₆ alkyl, -O-C₁₋₆ haloalkyl, -O-C₁₋₆ halohydroxyalkyl, -O-C₁₋₆ alkyl substituted by O-(3-6 membered heterocyclyl) and/or -O-(p-toluenesulfonyl), -NHC₁₋₆ alkyl, -NHC₁₋₆ haloalkyl, -NHC₁₋₆ halohydroxyalkyl, -NH-C₁₋₆ alkyl substituted by O-(3-6 membered heterocyclyl) and/or -O-(p-toluenesulfonyl), -N(C₁₋₆ alkyl)₂, - COOH, -NHCO-C₁₋₆ alkyl, -N(C₁₋₆ alkyl)-CO-C₁₋₆ alkyl, and 5-6 membered heterocyclyl, wherein the 5-6 membered heterocyclyl is optionally substituted by one or more substituents, said substituents are each independently selected from deuterium, tritium, -O-C₁₋₆ alkyl, -C₁₋₆ alkyl, halogen, hydroxy, oxo, -NHC₁₋₆ alkyl, -N(C₁₋₆ alkyl)₂,
Ring A is selected from a C₆₋₁₂ aromatic ring and a 6-10 membered heteroaromatic ring;
Ring B is selected from a C₆₋₁₂ aromatic ring and a 5-10 membered heteroaromatic ring;
R₃ is selected from hydroxy, amino, halogen, nitro, cyano, -C₁₋₆ alkyl, -O-C₁₋₆ alkyl, -NHC₁₋₆ alkyl, -N(C₁₋₆ alkyl)₂, 5-7 membered heterocyclyl, and C₆ aryl; wherein said 5-7 membered heterocyclyl and C₆ aryl are each independently optionally substituted by -NHC₁₋₆ alkyl or -N(C₁₋₆ alkyl)₂, wherein the -C₁₋₆ alkyl appearing in R₃ is optionally substituted by 1 or 2 substituents each independently selected from halogen or an isotope thereof (e.g., F or 18F) and hydroxy;
t is an integer from 0 to 2,
wherein the heteroaryl and heterocyclyl each independently contain 1, 2, or 3 heteroatoms selected from N, S, or O; and
wherein each occurrence of halogen is optionally in its isotopic form, and the carbon atoms and/or hydrogen atoms in the substituents are optionally in their isotopic forms.

**Embodiment 16.1:** The compound according to embodiment 16, or a stereoisomer, pharmaceutically acceptable salt, solvate, or stable isotopic variant thereof, wherein
R₁ is selected from -O-C₁₋₆ halohydroxyalkyl and -NHC₁₋₆ halohydroxyalkyl, preferably R₁ is selected from -O-C₁₋₆ halohydroxyalkyl,
Ring A is selected from a benzene ring and a 6-membered heteroaromatic ring containing 1 or 2 nitrogen heteroatoms;
Ring B is selected from a benzene ring and a 5-6 membered heteroaromatic ring containing 1 or 2 heteroatoms selected from nitrogen, oxygen, and sulfur;
R₃ is selected from -NHC₁₋₆ alkyl;
t is 1;
wherein each occurrence of halogen is preferably F or 18F, and the carbon atoms and/or hydrogen atoms in the substituents are optionally in their isotopic forms.

**Embodiment 16.2:** The compound according to embodiment 16, or a stereoisomer, pharmaceutically acceptable salt, solvate, or stable isotopic variant thereof, 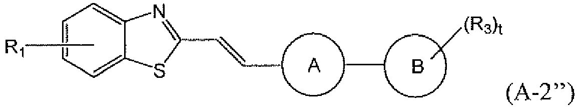 wherein
R1 is selected from -O-C₁₋₆ haloalkyl, -O-C₁₋₆ halohydroxyalkyl, -N(C₁₋₆ alkyl)(C₁₋₆ haloalkyl), -N(C₁₋₆ alkyl)(C₁₋₆ halohydroxyalkyl), -N(C₁₋₆ haloalkyl)(C₁₋₆ haloalkyl), -N(C₁₋₆ haloalkyl)(C₁₋₆ halohydroxyalkyl), -N(C₃₋₈ cycloalkyl)(C₁₋₆ haloalkyl), -N(C₃₋₈ cycloalkyl)(C₁₋₆ halohydroxyalkyl);
Ring A is 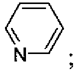
Ring B is selected from a 6-membered heteroaromatic ring;
R₃ is selected from deuterium, tritium, hydroxy, amino, halogen, nitro, cyano, carboxyl, C₁₋₆ alkyl, C₁₋₆ haloalkyl, C₁₋₆ hydroxyalkyl, C₂₋₆ alkenyl, C₂₋₆ alkynyl, -C₁₋₆ alkoxy, C₁₋₆ haloalkoxy, C₁₋₆ alkylamino, C₁₋₆ haloalkylamino, methylsulfonyl, C₂₋₆ acyl, C₂₋₆ ester, C₂₋₆ amide, C₂₋₆ sulfonamide, C₁₋₆ halohydroxyalkoxy, C₃₋₈ cycloalkyl, 3-8 membered heterocyclyl, 5-8 membered heteroaryl.
t is an integer from 0 to 5;
wherein each occurrence of halogen is optionally in isotopic form.

**Embodiment 16.3:** The compound according to embodiment 16, or a stereoisomer, pharmaceutically acceptable salt, solvate, or stable isotopic variant thereof, wherein:
Ring A is 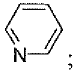
Ring B is selected from a C₆₋₁₂ aromatic ring, a C₄₋₈ cycloalkane, a 5-8 membered heteroaromatic ring;
R₁ is selected from: deuterium, tritium, hydroxy, C₁₋₆ alkoxy, C₁₋₆ alkyl, C₃₋₈ cycloalkyl, 3-8 membered heterocyclyl, 5-8 membered heteroaryl, C₁₋₆ alkylamino, halogen, C₁₋₆ haloalkoxy, C₁₋₆ haloalkylamino, C₂₋₆ alkenyl, C₂₋₆ alkynyl, C₂₋₆ acyl, nitro, cyano, carboxyl, C₂₋₆ ester, amino, C₂₋₆ amide, -SO₂CH₃, C₂₋₆ sulfonamide; wherein the hydrogen atoms on the C₁₋₆ alkyl, C₁₋₆ alkoxy, and C₁₋₆ alkylamino can be optionally substituted by 1-3 groups selected from C₁₋₆ alkoxy, C₁₋₆ alkyl, halogen, hydroxy, OTHP, -O-(3-6 membered heterocyclyl), -O-(p-toluenesulfonyl), and when two substituents are on the same carbon atom, they can together with the carbon atom to which they are attached form a 3-6 membered heterocyclic ring;
R₃ is selected from: deuterium, tritium, hydroxy, C₁₋₆ alkoxy, C₁₋₆ alkyl, C₃₋₈ cycloalkyl, 3-8 membered heterocyclyl, 3-8 membered heteroaryl, C₁₋₆ alkylamino, halogen, C₂₋₆ alkenyl, C₂₋₆ alkynyl, C₂₋₆ acyl, nitro, cyano, carboxyl, C₂₋₆ ester, amino, C₂₋₆ amide, -SO₂CH₃, C₂₋₆ sulfonamide; wherein the C₁₋₆ alkoxy and C₁₋₆ alkylamino are optionally substituted by 1-3 substituents selected from halogen, hydroxy, OTHP, -O-(3-6 membered heterocyclyl), -O-(p-toluenesulfonyl);
wherein the heteroaryl, heteroaromatic ring, heterocyclyl, and heterocyclic ring each independently contain 1, 2, 3, or 4 heteroatoms selected from N, S, or O;
said halogen includes isotopes thereof.
It should be noted that in any of the above embodiments 15 to 16.3, specific examples of each variable are as exemplified in the corresponding preceding embodiments,

**Embodiment 17:** The compound according to any preceding embodiment, or a stereoisomer, pharmaceutically acceptable salt, solvate, or stable isotopic variant thereof, wherein the stable isotopic variant comprises an atom selected from 2H, 3H, 13C, 11C, 14C, 15N, 18O, 17O, 31P, 32P, 35S, 18F, and 36Cl; preferably comprises 18F.
**Embodiment 18:** A compound, or a stereoisomer, pharmaceutically acceptable salt, solvate, or stable isotopic variant thereof, selected from:

| | | | |
|---|---|---|---|
| I-1 | 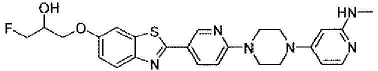 | I-26 | 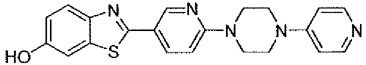 |
| I-2 | 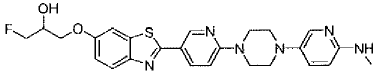 | I-27 | 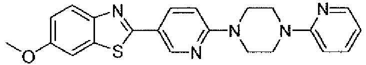 |
| I-3 | 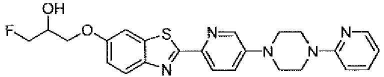 | I-28 | 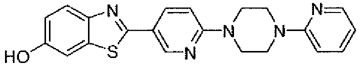 |
| I-4 | 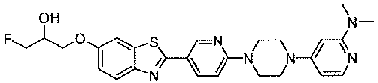 | I-29 | 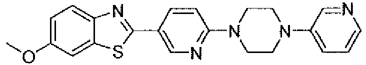 |
| I-5 | 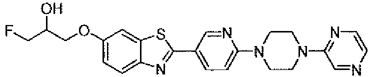 | I-30 | 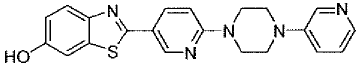 |
| I-6 | 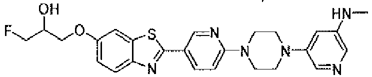 | I-31 | 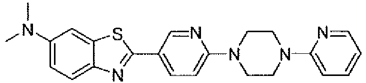 |
| I-7 | 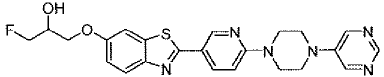 | I-32 | 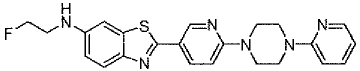 |
| I-8 | 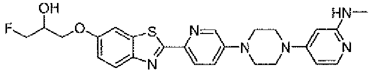 | I-33 | 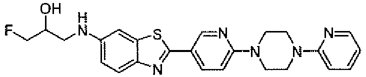 |
| I-9 | 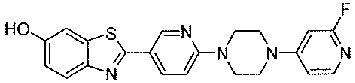 | I-34 | 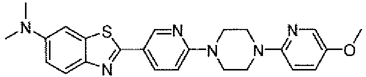 |
| I-10 | 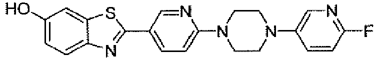 | I-35 | 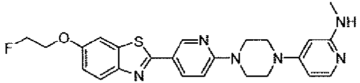 |
| I-11 | 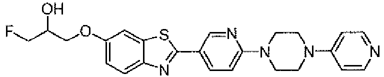 | I-36 | 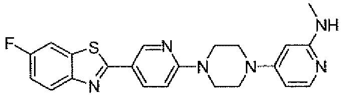 |
| I-12 | 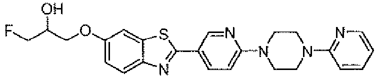 | I-37 | 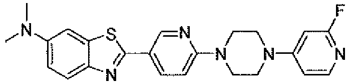 |
| I-13 | 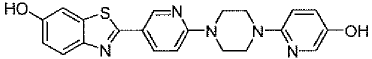 | I-38 | 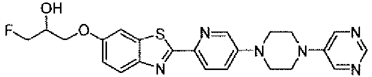 |
| I-14 | 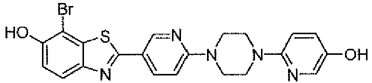 | I-39 | 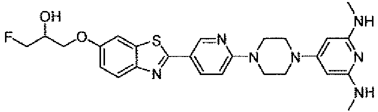 |
| I-15 | 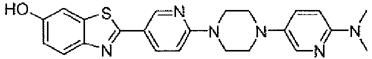 | I-40 | 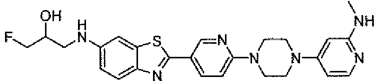 |
| I-16 | 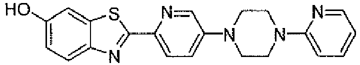 | I-41 | 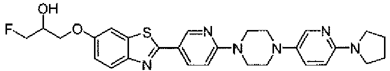 |
| I-17 | 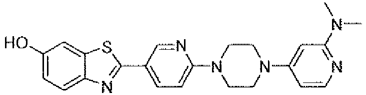 | I-42 | 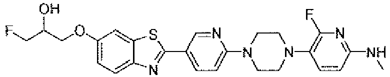 |
| I-18 | 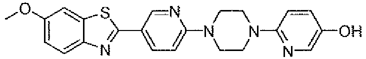 | I-43 | 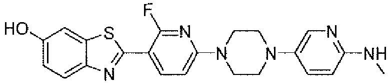 |
| I-19 | 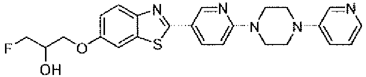 | I-44 | 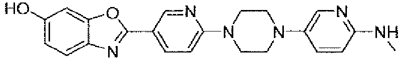 |
| I-20 | 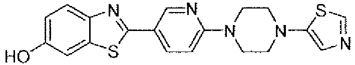 | I-45 | 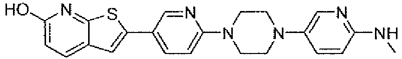 |
| I-21 | 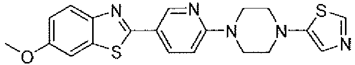 | I-46 | 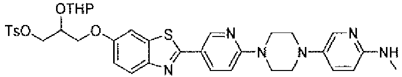 |
| I-22 | 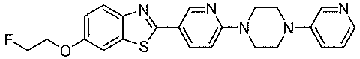 | I-47 | 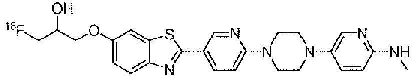 |
| I-23 | 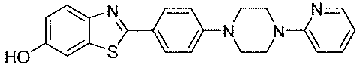 | I-48 | 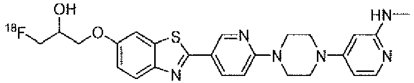 |
| I-24 | 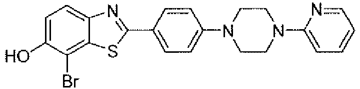 | I-49 | 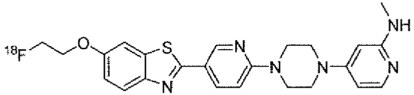 |
| I-25 | 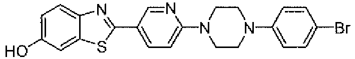 | I-50 | 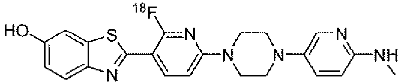 |
| | | I-51 | 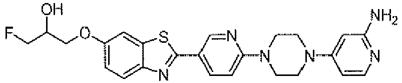 |
| II-1 | 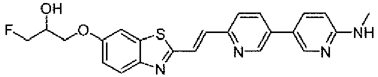 | II-16 | 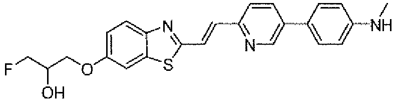 |
| II-2 | 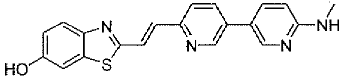 | II-17 | 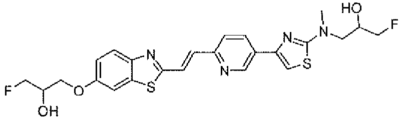 |
| II-3 | 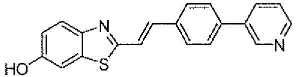 | II-18 | 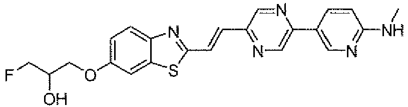 |
| II-4 | 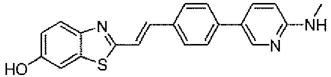 | II-19 | 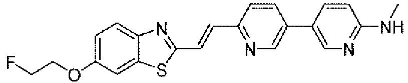 |
| II-5 | 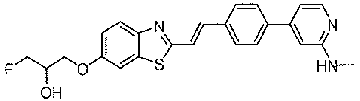 | II-20 | 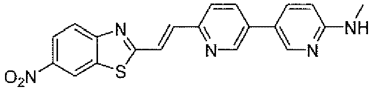 |
| II-6 | 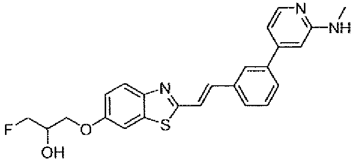 | II-21 | 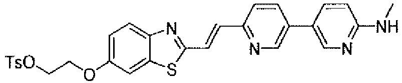 |
| II-7 | 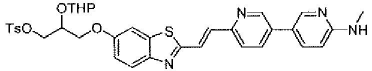 | II-22 | 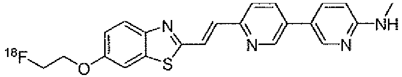 |
| II-8 | 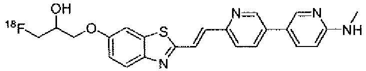 | II-23 | 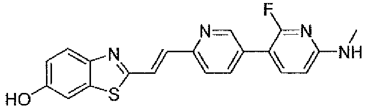 |
| II-9 | 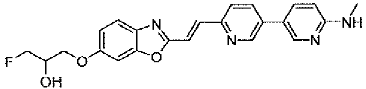 | II-24 | 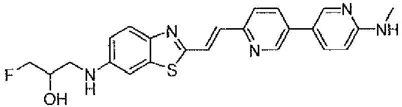 |
| II-10 | 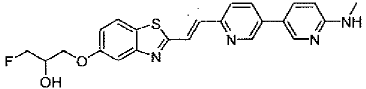 | II-25 | 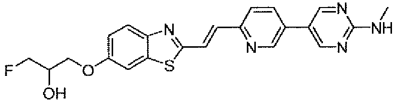 |
| II-11 | 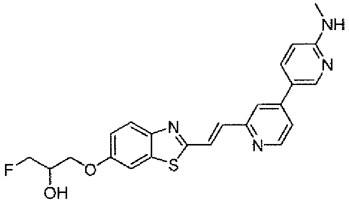 | II-26 | 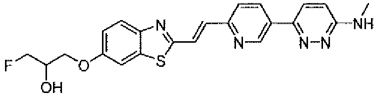 |
| II-12 | 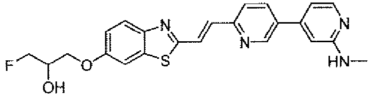 | II-27 | 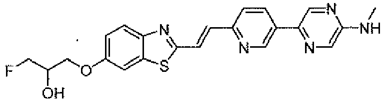 |
| II-13 | 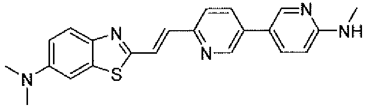 | II-28 | 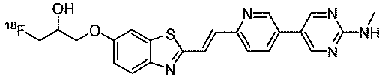 |
| II-14 | 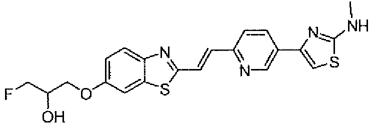 | II-29 | 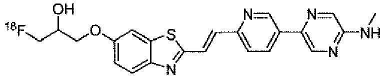 |
| II-15 | 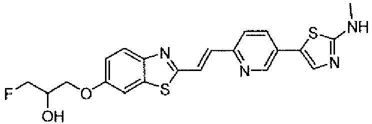 | | |
| III-1 | 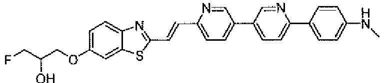 | III-13 | 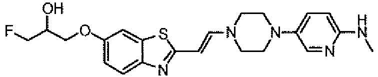 |
| III-2 | 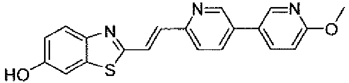 | III-14 | 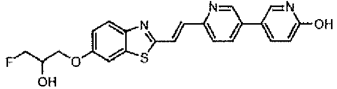 |
| III-3 | 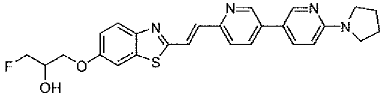 | III-15 | 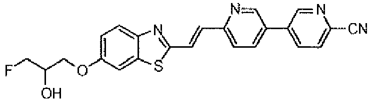 |
| III-4 | 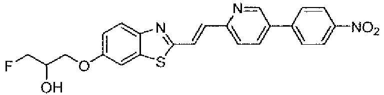 | III-16 | 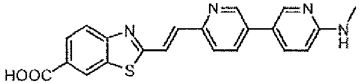 |
| III-5 | 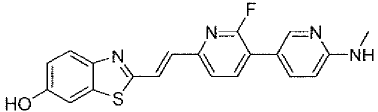 | III-17 | 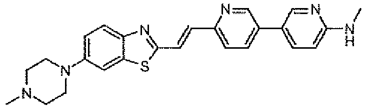 |
| III-6 | 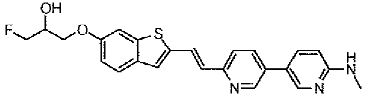 | III-18 | 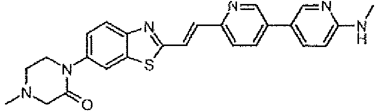 |
| III-7 | 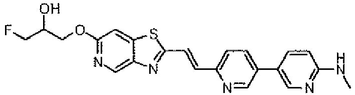 | III-19 | 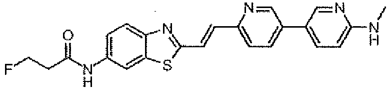 |
| III-8 | 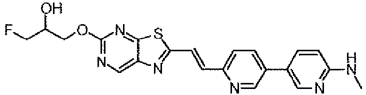 | III-20 | 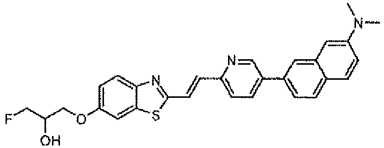 |
| III-9 | 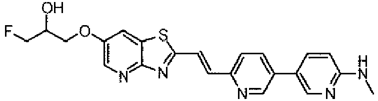 | III-21 | 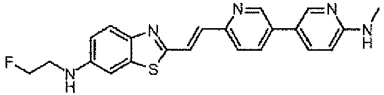 |
| III-10 | 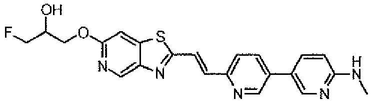 | III-22 | 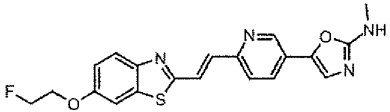 |
| III-11 | 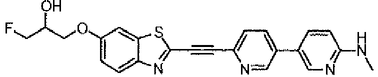 | III-12 | 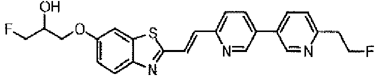 |
| III-23 | 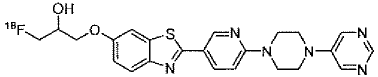 | | |

In a more specific embodiment, the compound of the present invention is selected from the following compounds and their stereoisomers, or pharmaceutically acceptable salts or solvates thereof:

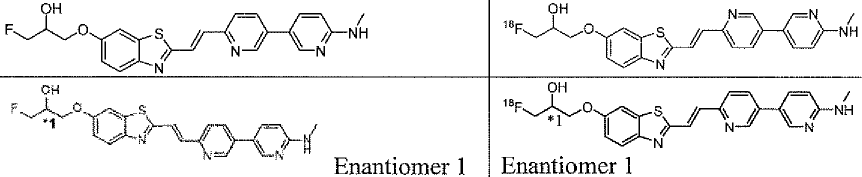

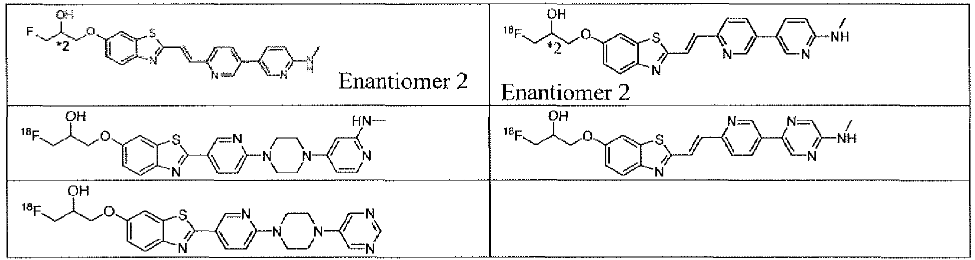

In other embodiments, some compounds of Formula A of the present invention can also be defined as follows: 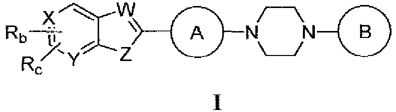 or a pharmaceutically acceptable salt thereof, or a solvate thereof,
wherein X and Y are each independently selected from CH or N; and when said X or Y is CH, the hydrogen atom on said CH may be substituted by R_{b} or R_{c};
W is selected from CH or N;
W and Z are each independently selected from CH₂, NH, O, or S;
Ring A is selected from the group consisting of: 4-8 membered saturated cycloalkane, benzene ring, 5-8 membered heteroaromatic ring, 4-8 membered heterocyclic ring;
Ring B is selected from the group consisting of: naphthalene ring, biphenyl, 4-8 membered saturated cycloalkane, benzene ring, 5-8 membered heteroaromatic ring, 4-8 membered heterocyclic ring;
0, 1, 2, 3, 4, or 5 hydrogens on Ring A or Ring B are substituted by a substituent selected from the group consisting of: deuterium, tritium, hydroxy, C₁₋₆ alkoxy, C₁₋₆ halohydroxyalkoxy, C₁₋₆ alkyl, C₃₋₈ cycloalkyl, 3-8 membered heterocyclyl, 3-8 membered heteroaryl, C₁₋₆ alkylamino, halogen, C₁₋₆ haloalkyl, C₁₋₆ haloalkoxy, C₁₋₆ haloalkylamino, C₂₋₆ alkenyl, C₂₋₆ alkynyl, C₂₋₆ acyl, C₁₋₆ hydroxyalkyl, nitro, cyano, carboxyl, C₂₋₆ ester, amino, C₂₋₆ amide, methylsulfonyl, C₂₋₆ sulfonamide;
R_{b} and R_{c} are each independently selected from the group consisting of: deuterium, tritium, hydroxy, C₁₋₆ alkoxy, C₁₋₆ halohydroxyalkoxy, C₁₋₆ alkyl, C₃₋₈ cycloalkyl, 3-8 membered heterocyclyl, 5-8 membered heteroaryl, C₁₋₆ alkylamino, halogen, C₁₋₆ haloalkyl, C₁₋₆ haloalkoxy, C₁₋₆ haloalkylamino, C₁₋₆ halohydroxyalkylamino, C₂₋₆ alkenyl, C₂₋₆ alkynyl, C₂₋₆ acyl, C₁₋₆ hydroxyalkyl, nitro, cyano, carboxyl, C₂₋₆ ester, amino, C₂₋₆ amide, methylsulfonyl, C₂₋₆ sulfonamide, or JR'; wherein 0, 1, 2, or 3 hydrogen atoms on the C₁₋₆ alkoxy can be substituted by R_{d} and Rₑ, said R_{d} and Rₑ are each independently selected from the group consisting of: C₁₋₆ alkoxy, halogen, hydroxy, TsO, OTHP, -O-(3-6 membered heterocyclyl), -O-(p-toluenesulfonyl), and when R_{d} and Rₑ are substituted on the same carbon atom, R_{d} and Rₑ can together with the carbon atom to which they are attached form a 3-6 membered heterocyclic ring; J is O or NRₐ; Rₐ is selected from the group consisting of: hydrogen, C₁₋₆ alkyl, C₁₋₆ haloalkyl, 3-8 membered cycloalkyl; R' is selected from the group consisting of: hydrogen, methyl, C₁₋₆ alkyl, C₁₋₆ haloalkyl, C₁₋₆ haloalkyl substituted by hydroxy, wherein 0, 1, 2, or 3 hydrogen atoms on the C₁₋₆ alkyl can be substituted by -O-(3-6 membered heterocyclyl) or -O-(p-toluenesulfonyl);
said heteroaryl, heteroaromatic ring, heterocyclyl, and heterocyclic ring each independently contain 1, 2, 3, or 4 heteroatoms selected from N, S, or O;
said halogen includes isotopes thereof.

In a preferred embodiment, Ring A is selected from the group consisting of: 6-8 membered aromatic ring, 5-8 membered heteroaromatic ring; preferably, Ring A is selected from the group consisting of:

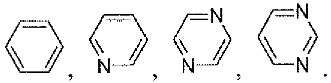

In a preferred embodiment, Ring B is selected from the group consisting of: benzene ring, 5-8 membered heteroaromatic ring; preferably, Ring B is selected from the group consisting of:

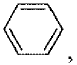

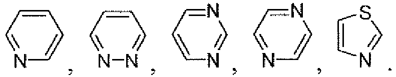

In a preferred embodiment, R_{b} is hydrogen or halogen, R_{c} is W-R',
wherein W is O or NRₐ, Rₐ is selected from the group consisting of: hydrogen, C₁₋₆ alkyl, C₁₋₆ haloalkyl, 3-8 membered cycloalkyl;
R' is selected from the group consisting of: hydrogen, methyl, C₁₋₆ alkyl, C₁₋₆ haloalkyl, C₁₋₆ haloalkyl substituted by hydroxy, wherein 0, 1, 2, or 3 hydrogen atoms on the C₁₋₆ alkyl can be substituted by -O-(3-6 membered heterocyclyl) or -O-(p-toluenesulfonyl);
preferably, R_{c} is selected from the group consisting of:

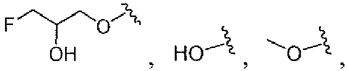

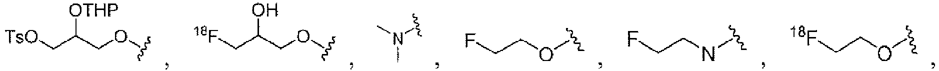

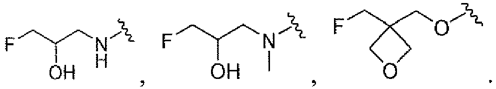

In a preferred embodiment, the compound includes a compound of Formula I', or a pharmaceutically acceptable salt thereof, or a solvate thereof, 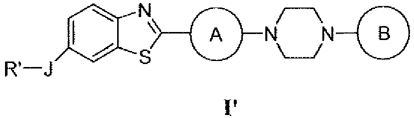
wherein J is O or NRₐ; Rₐ is selected from the group consisting of: hydrogen, C₁₋₆ alkyl, C₁₋₆ haloalkyl, 3-8 membered cycloalkyl;
R' is selected from the group consisting of: hydrogen, methyl, C₁₋₆ alkyl, C₁₋₆ haloalkyl, C₁₋₆ haloalkyl substituted by hydroxy, wherein 0, 1, 2, or 3 hydrogen atoms on the C₁₋₆ alkyl can be substituted by -O-(3-6 membered heterocyclyl) or -O-(p-toluenesulfonyl);
Ring A is 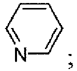
Ring B is as defined in claim 1.

In other embodiments, some compounds of Formula A of the present invention can also be defined as follows: 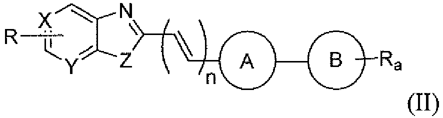 or a pharmaceutically acceptable salt thereof, or a solvate thereof,
wherein X and Y are each independently selected from CH or N; and when said X or Y is CH, the hydrogen atom on said CH may be substituted by R;
Z is selected from CH₂, O, NH, or S;
n is 1, 2, or 3;
Ring A is selected from the group consisting of: 6-8 membered saturated cycloalkane, benzene ring, 5-8 membered heteroaromatic ring, 6-8 membered heterocyclic ring;
Ring B is selected from the group consisting of: naphthalene ring, biphenyl, benzene ring, 4-8 membered saturated cycloalkane, 5-membered heteroaromatic ring (e.g., pyridine), 7-8 membered heteroaromatic ring;
R is selected from the group consisting of: deuterium, tritium, hydroxy, C₁₋₆ alkoxy, C₁₋₆ alkyl, C₃₋₈ cycloalkyl, 3-8 membered heterocyclyl, 3-8 membered heteroaryl, C₁₋₆ alkylamino, halogen, C₁₋₆ haloalkoxy, C₁₋₆ haloalkylamino, C₂₋₆ alkenyl, C₂₋₆ alkynyl, C₂₋₆ acyl, nitro, cyano, carboxyl, C₂₋₆ ester, amino, C₂₋₆ amide, methylsulfonyl, C₂₋₆ sulfonamide; wherein 0, 1, 2, or 3 hydrogen atoms on the C₁₋₆ alkyl, C₁₋₆ alkoxy, and C₁₋₆ alkylamino can be substituted by R_{b} or R_{c}, said R_{b} and R_{c} are each independently selected from the group consisting of: C₁₋₆ alkoxy, C₁₋₆ alkyl, halogen, hydroxy, TsO, OTHP, -O-(3-6 membered heterocyclyl), -O-(p-toluenesulfonyl), and when R_{b} and R_{c} are substituted on the same carbon atom, R_{b} and R_{c} can together with the carbon atom to which they are attached form a 3-6 membered heterocyclic ring;
Rₐ is selected from the group consisting of: deuterium, tritium, hydroxy, C₁₋₆ alkoxy, C₁₋₆ alkyl, C₃₋₈ cycloalkyl, 3-8 membered heterocyclyl, 3-8 membered heteroaryl, C₁₋₆ alkylamino, halogen, C₂₋₆ alkenyl, C₂₋₆ alkynyl, C₂₋₆ acyl, nitro, cyano, carboxyl, C₂₋₆ ester, amino, C₂₋₆ amide, methylsulfonyl, C₂₋₆ sulfonamide; wherein 0, 1, 2, or 3 hydrogen atoms on the C₁₋₆ alkoxy and C₁₋₆ alkylamino can be substituted by a substituent selected from the group consisting of: halogen, hydroxy, TsO, OTHP, -O-(3-6 membered heterocyclyl), -O-(p-toluenesulfonyl);
Unless otherwise specified, 0, 1, 2, 3, 4, or 5 hydrogens on the above groups can be further substituted by a substituent selected from the group consisting of: deuterium, tritium, hydroxy, halogen, C₁₋₆ alkoxy, C₁₋₆ halohydroxyalkoxy, C₁₋₆ alkyl, C₃₋₈ cycloalkyl, 3-8 membered heterocyclyl, 3-8 membered heteroaryl, C₁₋₆ alkylamino, C₁₋₆ haloalkyl, C₁₋₆ haloalkoxy, C₁₋₆ haloalkylamino, C₁₋₆ halohydroxyalkylamino, C₂₋₆ alkenyl, C₂₋₆ alkynyl, acyl, C₁₋₆ hydroxyalkyl, nitro, cyano, carboxyl, ester, amino, amide, methylsulfonyl, sulfonamide;
said heteroaryl, heteroaromatic ring, heterocyclyl, and heterocyclic ring each independently contain 1, 2, 3, or 4 heteroatoms selected from N, S, or O;
said halogen includes isotopes thereof.

In another preferred embodiment, said -O-(3-6 membered heterocyclyl) is -O-(tetrahydropyranyl).

In a preferred embodiment, Ring A is selected from the group consisting of: 6-8 membered aromatic ring, 5-8 membered heteroaromatic ring; preferably, Ring A is selected from the group consisting of:

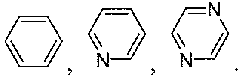

In a preferred embodiment, Ring B is selected from the group consisting of: benzene ring, 5-8 membered heteroaromatic ring; preferably, Ring B is selected from the group consisting of:

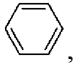

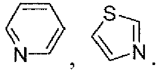

In a preferred embodiment, R is selected from the group consisting of: 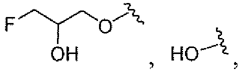 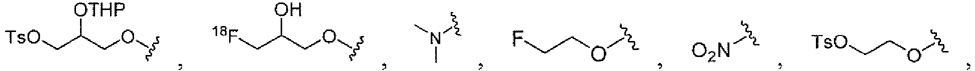 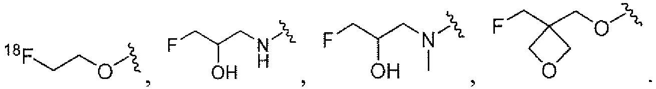 In a preferred embodiment, the compound includes a compound of Formula I', or a pharmaceutically acceptable salt thereof, or a solvate thereof, 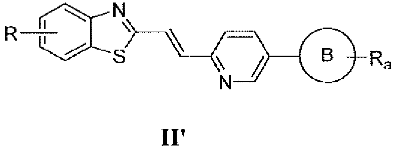 wherein Ring B, R, and Rₐ are as defined above.

In other embodiments, some compounds of Formula A of the present invention can also be defined as follows: 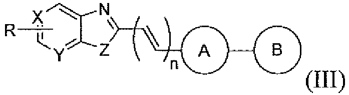 or a pharmaceutically acceptable salt thereof, or a solvate thereof,
wherein X and Y are each independently selected from CH or N;
Z is selected from CH₂, O, NH, or S;
n is 1, 2, or 3;
Ring A is selected from the group consisting of: 6-8 membered saturated cycloalkane, benzene ring, 5-8 membered heteroaromatic ring, 6-8 membered heterocyclic ring;
Ring B is a 6-membered heteroaromatic ring;
0, 1, 2, 3, 4, or 5 hydrogens on Ring A or Ring B are substituted by a substituent selected from the group consisting of: deuterium, tritium, hydroxy, C₁₋₆ alkoxy, C₁₋₆ halohydroxyalkoxy, C₁₋₆ alkyl, C₃₋₈ cycloalkyl, 3-8 membered heterocyclyl, 3-8 membered heteroaryl, C₁₋₆ alkylamino, halogen, C₁₋₆ haloalkyl, C₁₋₆ haloalkoxy, C₁₋₆ haloalkylamino, C₁₋₆ halohydroxyalkylamino, C₂₋₆ alkenyl, C₂₋₆ alkynyl, acyl, C₁₋₆ hydroxyalkyl, nitro, cyano, carboxyl, C₂₋₆ ester, amino, C₂₋₆ amide, methylsulfonyl, C₂₋₆ sulfonamide;
R is selected from the group consisting of: deuterium, tritium, hydroxy, C₁₋₆ alkoxy, C₁₋₆ alkyl, C₃₋₈ cycloalkyl, 3-8 membered heterocyclyl, 3-8 membered heteroaryl, C₃₋₆ alkylamino, halogen, C₁₋₆ haloalkyl, C₂₋₆ alkenyl, C₂₋₆ alkynyl, C₂₋₆ acyl, C₁₋₆ hydroxyalkyl, nitro, cyano, carboxyl, C₂₋₆ ester, amino, C₂₋₆ amide, methylsulfonyl, C₂₋₆ sulfonamide; wherein 0, 1, 2, or 3 hydrogen atoms on the C₁₋₆ alkoxy and C₁₋₆ alkylamino can be substituted by R_{b} or R_{c}, said R_{b} and R_{c} are each independently selected from the group consisting of: C₁₋₆ alkoxy, halogen, hydroxy, TsO, OTHP, and when R_{b} and R_{c} are substituted on the same carbon atom, R_{b} and R_{c} can together with the carbon atom to which they are attached form a 3-6 membered heterocyclic ring;
said heteroaryl, heteroaromatic ring, heterocyclyl, and heterocyclic ring each independently contain 1, 2, 3, or 4 heteroatoms selected from N, S, or O;
said halogen includes isotopes thereof.
In a preferred embodiment, Ring A is selected from the group consisting of:

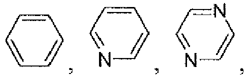

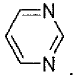

In a preferred embodiment, Ring B is a 6-membered heteroaryl group containing 2 or 3 heteroatoms selected from N, S, or O;
preferably, Ring B is selected from the group consisting of:

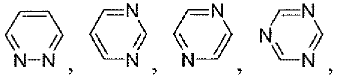

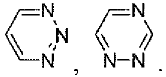

In a preferred embodiment, R is W-R',
wherein W is O or NRₐ; said Rₐ is selected from the group consisting of: C₁₋₆ alkyl, C₁₋₆ haloalkyl, 3-8 membered cycloalkyl;
R' is C₁₋₆ haloalkyl substituted by hydroxy, preferably selected from the group consisting of:

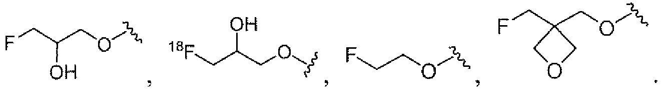

In a preferred embodiment, the compound includes a compound of Formula I', or a pharmaceutically acceptable salt thereof, or a solvate thereof, 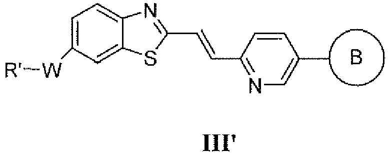
wherein W is O or NRₐ, Rₐ is selected from the group consisting of: C₁₋₆ alkyl, C₁₋₆ haloalkyl, 3-8 membered cycloalkyl; R' is selected from the group consisting of: C₁₋₆ haloalkyl substituted by hydroxy, unsubstituted C₁₋₆ haloalkyl;
Ring B is as defined above.

It should be noted that for compounds of formulas (I), (II), (III), in addition to the definitions above, each substituent may have the general, specific, or preferred meanings defined above for the corresponding substituent in Formula (A), and technical solutions formed by these substituents or any combination thereof are thus also encompassed by the present invention.

It should be understood that within the scope of the present invention, the above-mentioned technical features of the present invention and the technical features specifically described in the following text (e.g., in the Examples) can be combined with each other to form new or preferred technical solutions. Due to space limitations, they will not be repeated here.

### Main Advantages of the Invention:

The compounds of the present application have novel structures, can specifically recognize α-synuclein aggregates, and can be used in the manufacture of medicaments for treating or diagnosing neurodegenerative diseases associated with α-synuclein aggregates and other misfolded proteins, having significant clinical implications.

### Pharmaceutical Composition and Administration Method

Since the compounds of the present invention can recognize α-synuclein aggregates and are used for treating or diagnosing neurodegenerative diseases associated with α-synuclein aggregates and other pathological aggregated proteins (such as Parkinson's disease, dementia with Lewy bodies, multiple system atrophy, Alzheimer's disease, amyotrophic lateral sclerosis, etc.). Therefore, the compounds of the present invention and their stereoisomers, optical isomers, pharmaceutically acceptable salts, crystalline forms, isotopic derivatives, prodrugs, metabolites, solvates or hydrates thereof, as well as pharmaceutical compositions containing the compound of the present invention as the main active ingredient, can be used for treating (stabilizing, alleviating, or curing) or diagnosing neurodegenerative diseases (such as Parkinson's disease, dementia with Lewy bodies, multiple system atrophy, Alzheimer's disease, amyotrophic lateral sclerosis, progressive supranuclear palsy, progressive muscular atrophy, etc.) associated with α-synuclein aggregates and other pathological aggregated proteins.

The pharmaceutical composition of the present invention contains a safe and effective amount of the compound of the present invention and a pharmaceutically acceptable excipient or carrier. Generally, the pharmaceutical composition contains 1-2000 mg of the compound of the present invention per dose, more preferably 10-200 mg of the compound of the present invention per dose. Preferably, said "one dose" is a capsule or tablet.

Examples of pharmaceutically acceptable carriers include cellulose and its derivatives (such as sodium carboxymethylcellulose, sodium ethylcellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agents (such as sodium lauryl sulfate), colorants, flavoring agents, stabilizers, antioxidants, preservatives, pyrogen-free water, etc.

The administration mode of the compound or pharmaceutical composition of the present invention includes but is not limited to intravenous injection.

The compound of the present invention can be administered alone or in combination with other pharmaceutically acceptable compounds.

### Preparation Methods

In one aspect, the present application provides a method for preparing a compound of Formula (A-1), comprising the following methods:
Method 1: Where R₁ is not hydroxy, amino, C₁₋₆ halohydroxyalkoxy, C₁₋₆ halohydroxyalkylamino, C₁₋₆ haloalkoxy, or C₁₋₆ haloalkylamino, 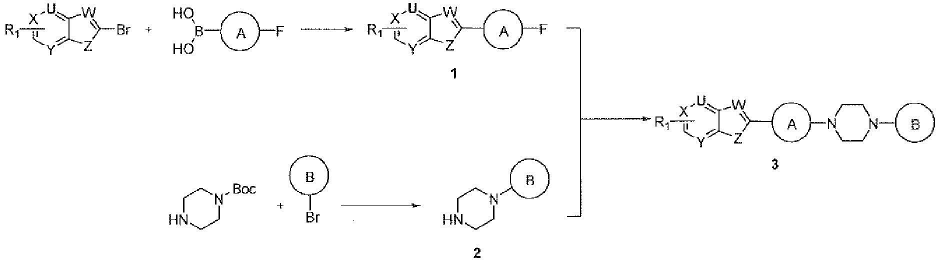 wherein X, Y, U, Z, W, Ring A, and Ring B are as defined above;
Method 2: Where R₁ is optionally substituted hydroxy or optionally substituted amino and the substituent is C₁₋₆ alkyl or C₃₋₈ cycloalkyl, 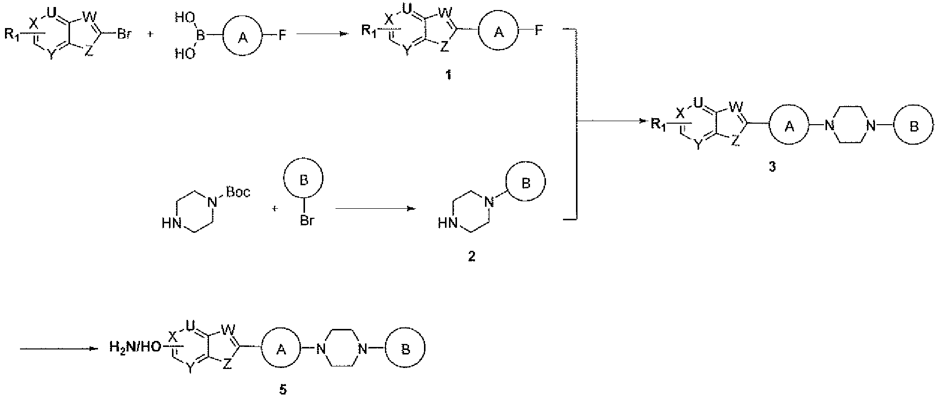 wherein X, Y, U, Z, W, Ring A, and Ring B are as defined above;
Method 3: Where R₁ is substituted C₁₋₆ alkoxy or substituted C₁₋₆ alkylamino, and the substituent is selected from halogen and OH, 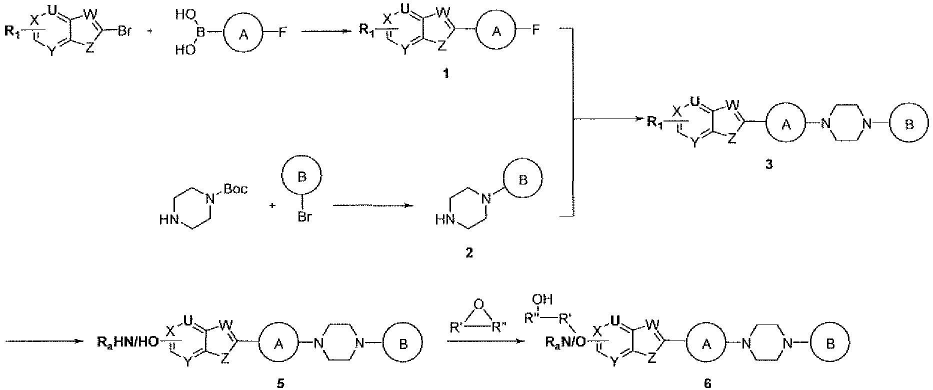
   wherein R' is selected from -C₁₋₆ alkyl and C₁₋₆ haloalkyl;
   R" is selected from C₁₋₆ haloalkyl;
   Rₐ is selected from H or -C₁₋₆ alkyl;
   X, Y, U, Z, W, Ring A, and Ring B are as defined above;
Method 4: Where R₁ is substituted C₁₋₆ alkoxy or substituted C₁₋₆ alkylamino, and the substituent is selected from halogen, -O-(p-toluenesulfonyl), -O-(3-6 membered heterocyclyl), 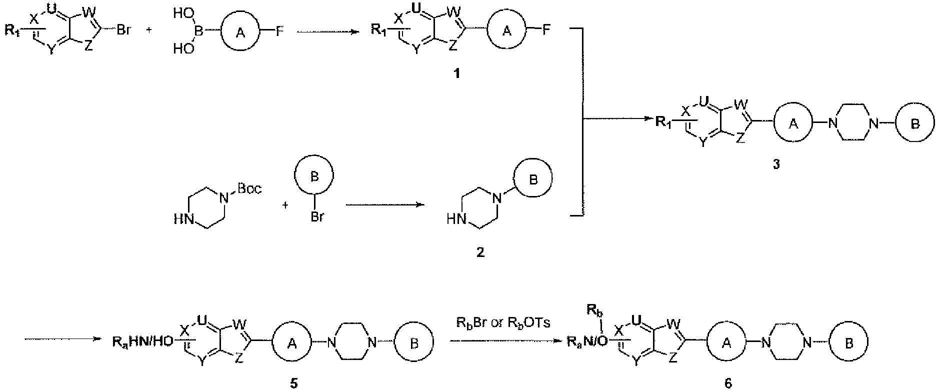
   wherein R_{b} is C₁₋₆ alkyl, and 0, 1, 2, 3, 4, or 5 hydrogen atoms thereof are substituted by a substituent selected from the group consisting of: halogen, -O-(p-toluenesulfonyl), -O-(3-6 membered heterocyclyl);
   Rₐ is selected from H or -C₁₋₆ alkyl;
   X, Y, U, Z, W, Ring A, and Ring B are as defined above.

In this regard, when the compound of formula (A) is defined by formula (I) as shown in the specification above, the corresponding preparation method can also be represented as follows:
Method 1. Where R_{b} is not hydroxy, amino, C₁₋₆ halohydroxyalkoxy, C₁₋₆ halohydroxyalkylamino, C₁₋₆ haloalkoxy, or C₁₋₆ haloalkylamino, 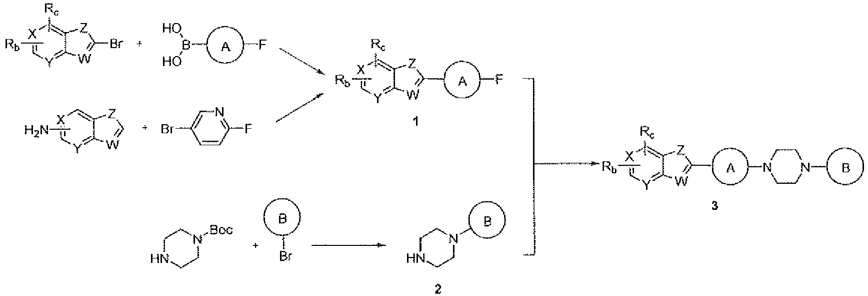 R_{c}, X, U, Z, W, Ring A, and Ring B are as defined above;
Method 2. Where R_{b} is hydroxy or amino, 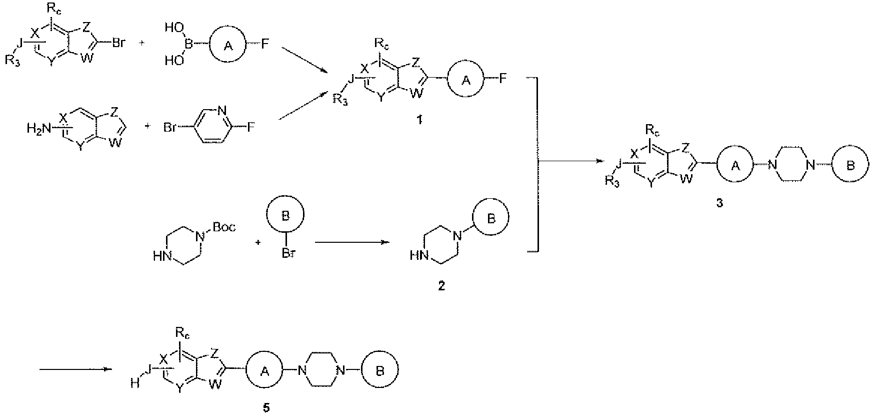 wherein R₃ is C₁₋₆ alkyl or C₁₋₆ cycloalkyl; R_{c}, X, U, Z, W, Ring A, and Ring B are as defined above;
Method 3. Where R_{b} is C₁₋₆ halohydroxyalkoxy or C₁₋₆ halohydroxyalkylamino, 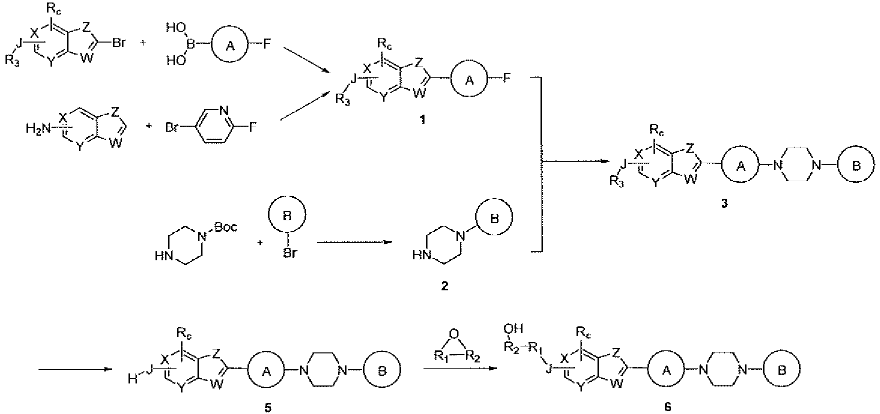
   wherein said R₁ is selected from the group consisting of: C₁₋₆ alkyl, C₁₋₆ haloalkyl, 3-8 membered cycloalkyl;
   said R₂ is selected from the group consisting of: C₁₋₆ haloalkyl;
   R₃, R_{c}, J, X, U, Z, W, Ring A, and Ring B are as defined above;
**Method 4.** Where R_{b} is C₁₋₆ haloalkoxy or C₁₋₆ haloalkylamino, 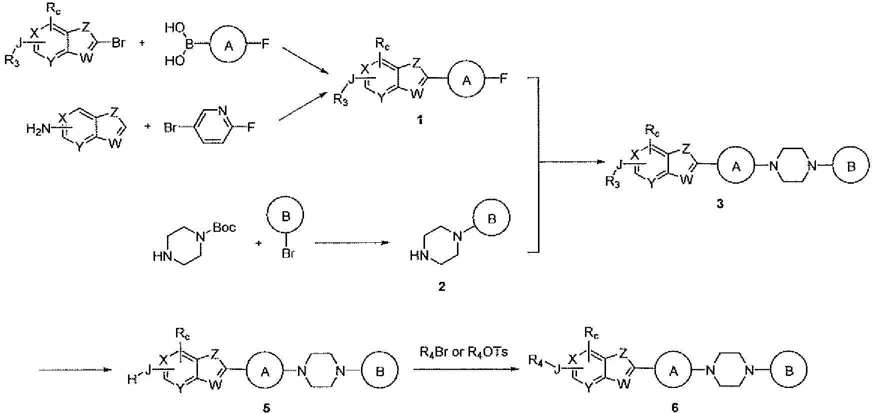
   wherein R₄ is selected from the group consisting of: C₁₋₆ alkyl, and 0, 1, 2, 3, 4, or 5 hydrogen atoms on the C₁₋₆ alkoxy are substituted by a substituent selected from the group consisting of: halogen, -O-(p-toluenesulfonyl), -O-(3-6 membered heterocyclyl);
   R₃, R_{c}, J, X, U, Z, W, Ring A, and Ring B are as defined above.

**On the other hand,** the present application provides a method for preparing a compound as shown in Formula (A-2), comprising the following methods:
**Method 1:** Where R₁ is not hydroxy, C₁₋₆ hydroxyalkoxy, C₁₋₆ halohydroxyalkoxy, or C₁₋₆ halohydroxyalkylamino, 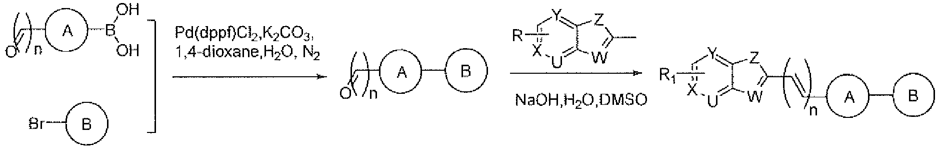 wherein X, Y, U, W, Z, n, Ring A, and Ring B are as defined above;
**Method 2:** Where R₁ is hydroxy, 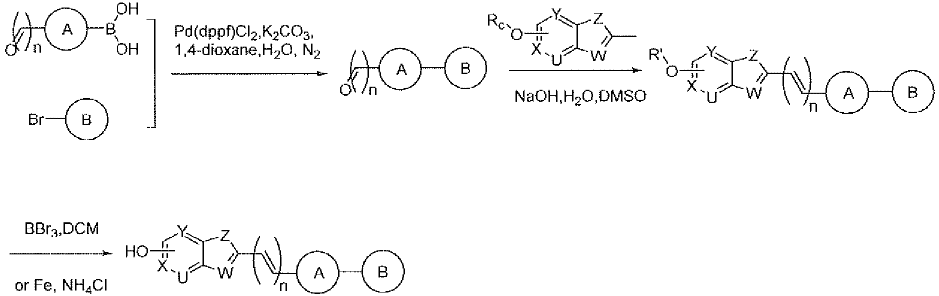 wherein R_{c} is selected from: C₁₋₆ haloalkyl; X, Y, U, W, Z, n, Ring A, and Ring B are as defined above;
Method 3: Where R₁ is C₁₋₆ halohydroxyalkoxy or C₁₋₆ halohydroxyalkylamino, 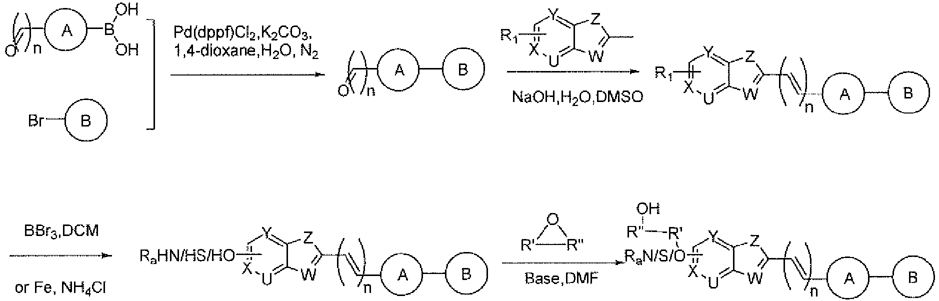
   wherein R' is selected from the group consisting of: C₁₋₆ alkyl and C₁₋₆ haloalkyl;
   R" is selected from the group consisting of: C₁₋₆ haloalkyl;
   Rₐ is selected from H or -C₁₋₆ alkyl;
   X, Y, U, W, Z, n, Ring A, and Ring B are as defined above;
**Method 4:** Where R₁ is C₁₋₆ alkoxy, and said C₁₋₆ alkoxy is optionally substituted by 1-5 substituents selected from the group consisting of: halogen, -O-(p-toluenesulfonyl), -O-(3-6 membered heterocyclyl), 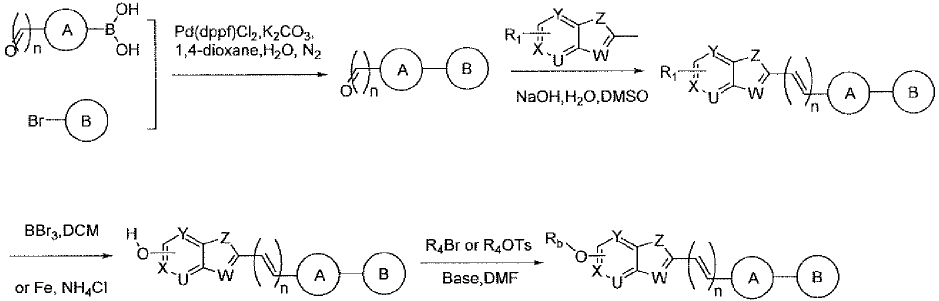
   wherein R_{b} is selected from the group consisting of: C₁₋₆ alkyl, and 0, 1, 2, 3, 4, or 5 hydrogen atoms thereof are substituted by a substituent selected from the group consisting of: halogen, -O-(p-toluenesulfonyl), -O-(3-6 membered heterocyclyl);
   W, U, X, Y, Z, n, Ring A, and Ring B are as defined above.

In this regard, when the compound of Formula (A) is defined by Formula (II) or Formula (III) as shown in the specification above, the corresponding preparation method can also be represented as follows:
**Method 1:** Where R is not hydroxy, C₁₋₆ hydroxyalkoxy, C₁₋₆ halohydroxyalkoxy, or C₁₋₆ halohydroxyalkylamino, 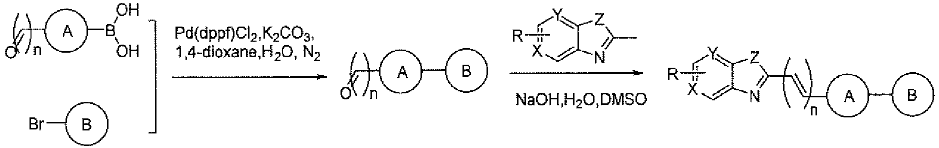 wherein X, Y, Z, n, Ring A, and Ring B are as defined above;
**Method 2:** Where R is hydroxy, 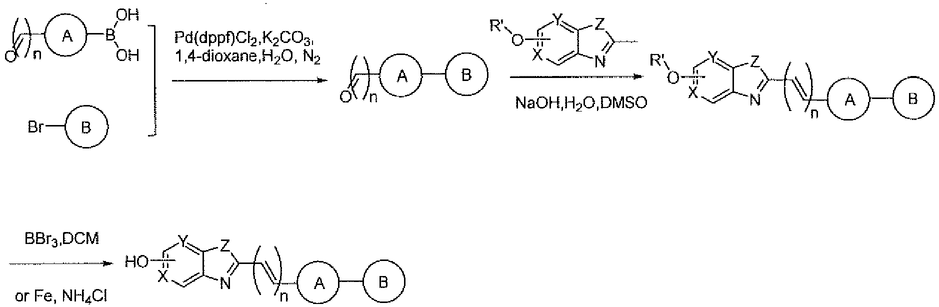 wherein R' is selected from: C₁₋₆ haloalkyl; X, Y, Z, n, Ring A, and Ring B are as defined above;
**Method 3:** Where R is C₁₋₆ halohydroxyalkoxy or C₁₋₆ halohydroxyalkylamino 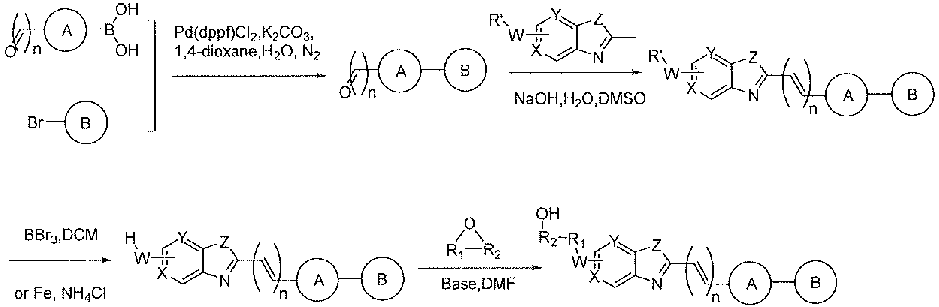
   wherein W is O, S, or NH;
   said R₁ is selected from the group consisting of: C₁₋₆ alkyl, C₁₋₆ haloalkyl, 3-8 membered cycloalkyl;
   said R₂ is selected from the group consisting of: C₁₋₆ haloalkyl;
   X, Y, Z, n, Ring A, and Ring B are as defined above;
**Method 4:** Where R is C₁₋₆ alkoxy, and 0, 1, 2, 3, 4, or 5 hydrogen atoms on the C₁₋₆ alkoxy are substituted by a substituent selected from the group consisting of: halogen, -O-(p-toluenesulfonyl), - O-(3-6 membered heterocyclyl), 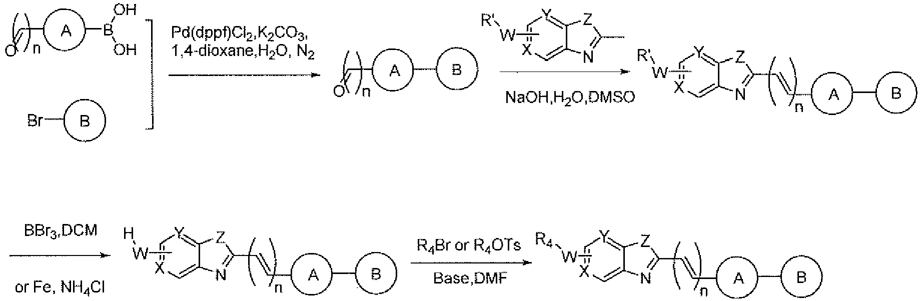
   wherein R₄ is selected from the group consisting of: C₁₋₆ alkyl, and 0, 1, 2, 3, 4, or 5 hydrogen atoms on the C₁₋₆ alkoxy are substituted by a substituent selected from the group consisting of: halogen, -O-(p-toluenesulfonyl), -O-(3-6 membered heterocyclyl);
   W, X, Y, Z, n, Ring A, and Ring B are as defined above.

### Abbreviations

Ts: p-toluenesulfonyl
THP: tetrahydropyranyl
Ex-vivo: ex vivo experiment

The present invention is further described below in conjunction with specific examples. It should be understood that these examples are only for illustrating the present invention and are not intended to limit the scope of the present invention. Experimental methods without specified conditions in the following examples are generally conducted under conventional conditions or conditions recommended by the manufacturer. Unless otherwise indicated, percentages and parts are by weight. Known starting materials of the present invention can be synthesized using or following methods known in the art, or are commercially available from various reagent companies. The structures of the compounds were determined by nuclear magnetic resonance (NMR) and/or mass spectrometry.

### Example I-1: Preparation of Compound I-1

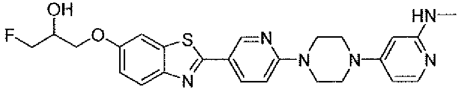

### Synthetic route of Compound I-1:

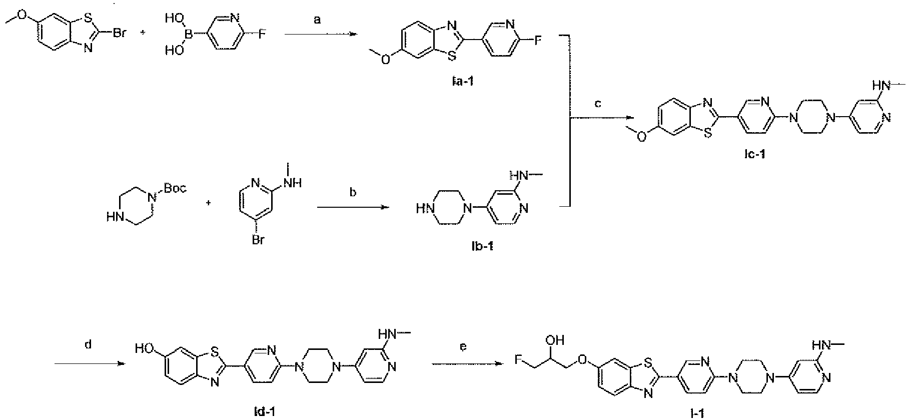

### Step 1: Preparation of Intermediate Ia-1

6-Methoxy-2-bromobenzothiazole (537 mg, 3 mmol) and 2-fluoro-5-pyridineboronic acid (423 mg, 3 mmol) were dissolved in 15 mL of solvent (1,4-dioxane:H₂O = 4:1). K₂CO₃ (1.24 g, 9 mmol) and Pd(dppf)Cl₂ (219 mg, 0.3 mmol) were added. The mixture was reacted at 80 °C for 8 h under N₂ protection. The reaction solution was then poured into 100 mL of saturated NH₄Cl solution, extracted three times with ethyl acetate, washed three times with brine, dried over anhydrous sodium sulfate, and then purified by column chromatography to give Ia-1 as a light yellow solid in 49% yield. ESI-MS (positive): 261.0 [M + 1]⁺.

### Step 2: Preparation of Intermediate Ib-1

1-tert-Butoxycarbonylpiperazine (373 mg, 2 mmol), 4-bromo-2-(methylamino)pyridine (357 mg, 2 mmol), Cs₂CO₃ (1.96 g, 6 mmol), Pd₂(dba)₃ (36 mg, 0.04 mmol), and XantPhos (70 mg, 0.12 mmol) were added to a round-bottom flask. 10 mL of THF was added. The mixture was reacted at 80 °C for 12 h under N₂ protection. After the reaction, THF was removed under reduced pressure by vacuum distillation. The product was dissolved in 20 mL of ethyl acetate, washed three times with brine, dried over anhydrous sodium sulfate, and then purified by column chromatography. The purified product was dissolved in 20 mL of dichloromethane, 2 mL of trifluoroacetic acid was added, and the mixture was stirred at room temperature for 2 h. The solvent was removed under reduced pressure by vacuum distillation to give Intermediate Ib-1 as a yellow solid in 57% yield. ESI-MS (positive): 193.1 [M + 1]⁺.

### Step 3: Preparation of Intermediate Ic-1

Intermediate Ia-1 (0.2 mmol) and Ib-1 (0.2 mmol) were dissolved in 1 mL of NMP. DIPEA (200 µL, 1 mmol) was added. The mixture was reacted in a microwave reactor at 180 °C for 4 h under N₂ protection. After the reaction, 10 mL of water was added, and the mixture was extracted with 20 mL of ethyl acetate, washed three times with brine, dried over anhydrous sodium sulfate, and then purified by column chromatography to give Ic-1 as a light yellow solid in 50% yield. ESI-MS (positive): 432.8 [M + 1]⁺.

### Step 4: Preparation of Intermediate Id-1

Intermediate Ic-1 (0.1 mmol) was dissolved in 0.3 mL of dichloromethane. The solution was cooled to -78 °C, then BBr₃ (0.3 mL, 1 M in DCM) was added dropwise. The mixture was reacted at room temperature for 12 h. After the reaction, the reaction solution was poured into 5 mL of water, extracted with 20 mL of ethyl acetate, washed three times with brine, dried over anhydrous sodium sulfate, and evaporated to dryness to give Intermediate Id-1 as a yellow solid in 81% yield. ESI-MS (positive): 418.9 [M + 1]⁺. ¹H NMR (400 MHz, DMSO-d₆) δ 12.14 - 11.98 (m, 1H), 9.82 (s, 1H), 8.74 (d, J = 2.5 Hz, 1H), 8.13 (dd, J = 9.0, 2.5 Hz, 1H), 7.78 (d, J = 8.8 Hz, 1H), 7.66 (t, J = 6.3 Hz, 1H), 7.56 (d, J = 5.2 Hz, 1H), 7.38 (d, J = 2.4 Hz, 1H), 7.01 - 6.92 (m, 2H), 6.61 (dd, J = 7.6, 2.5 Hz, 1H), 5.92 (s, 1H), 3.88 - 3.79 (m, 4H), 3.76 - 3.71 (m, 4H), 2.86 (d, J = 4.8 Hz, 3H).

### Step 5: Preparation of Product I-1

Intermediate Id-1 (0.05 mmol) was dissolved in 0.5 mL of DMF. K₂CO₃ (20 mg, 0.15 mmol) and epifluorohydrin (8 µL, 0.1 mmol) were added. The solution was reacted at 80 °C for 4 h. After the reaction, the reaction solution was purified by column chromatography to give the final product **I-1** as a light yellow solid in 61% yield. ESI-MS (positive): 495.0 [M + 1]⁺. ¹H NMR (400 MHz, DMSO-d₆) δ 12.07 (s, 1H), 8.78 (d, J = 2.6 Hz, 1H), 8.16 (dd, J = 9.0, 2.6 Hz, 1H), 7.87 (d, J = 8.9 Hz, 1H), 7.75 - 7.63 (m, 2H), 7.58 (d, J = 5.6 Hz, 1H), 7.12 (dd, J = 8.9, 2.6 Hz, 1H), 6.99 (d, J = 9.1 Hz, 1H), 6.61 (dd, J = 7.7, 2.5 Hz, 1H), 5.92 (s, 1H), 4.58 (dd, J = 9.5, 4.3 Hz, 1H), 4.52 - 4.39 (m, 1H), 4.13 - 4.01 (m, 3H), 3.88 - 3.83 (m, 4H), 3.76 - 3.72 (m, 4H), 2.86 (d, J = 4.7 Hz, 3H).

### Example I-2: Preparation of Compound I-2

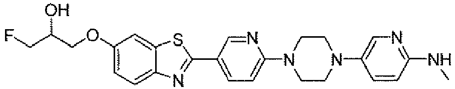

The synthetic method of Example **I-1** was followed, except that 4-bromo-2-(methylamino)pyridine was replaced with 5-bromo-2-(methylamino)pyridine. Compound I-2 was obtained as a yellow solid in 21% yield. ESI-MS (positive): 495.0 [M + 1]⁺. ¹H NMR (400 MHz, DMSO-d₆) δ 8.77 (d, J = 2.5 Hz, 1H), 8.42 (s, 1H), 8.14 (dd, J = 9.0, 2.5 Hz, 1H), 8.01 (dd, J = 9.8, 2.7 Hz, 1H), 7.87 (d, J = 8.9 Hz, 1H), 7.71 (d, J = 2.6 Hz, 1H), 7.26 (d, J = 2.7 Hz, 1H), 7.15 - 6.99 (m, 3H), 4.62 - 4.58 (m, 1H), 4.50 - 4.41 (m, 1H), 4.14 - 4.02 (m, 3H), 3.82 (t, J = 5.0 Hz, 4H), 3.14 (t, J = 5.0 Hz, 4H), 2.91 (s, 3H).

### Example I-3: Preparation of Compound I-3

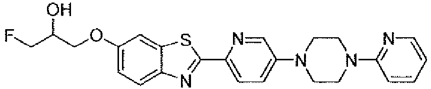

The synthetic method of Example **I-1** was followed, except that 2-fluoro-5-pyridineboronic acid was replaced with 3-fluoro-6-pyridineboronic acid, and 4-bromo-2-(methylamino)pyridine was replaced with 2-bromopyridine. Compound I-3 was obtained as a light yellow solid in 5% yield. ESI-MS (positive): 466.1 [M + 1]⁺. ¹H NMR (400 MHz, DMSO-d₆) δ 8.45 (d, J = 2.9 Hz, 1H), 8.15 - 8.05 (m, 2H), 7.89 (d, J = 8.9 Hz, 1H), 7.82 - 7.75 (m, 1H), 7.70 (d, J = 2.6 Hz, 1H), 7.53 (dd, J = 9.0, 2.9 Hz, 1H), 7.18 - 7.08 (m, 2H), 6.82 (t, J = 6.3 Hz, 1H), 4.58 (dd, J = 9.6, 4.3 Hz, 1H), 4.46 (dd, J = 9.9, 4.8 Hz, 1H), 4.14 - 4.01 (m, 3H), 3.78 - 3.74 (m, 4H), 3.59 - 3.55 (m, 4H).

### Example I-4: Preparation of Compound I-4

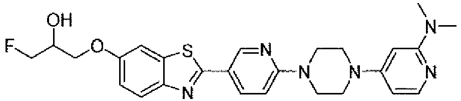

The synthetic method of Example **I-1** was followed, except that 4-bromo-2-(methylamino)pyridine was replaced with 4-bromo-2-(dimethylamino)pyridine. Compound I-4 was obtained as a yellow solid in 11% yield. ESI-MS (positive): 509.0 [M + 1]⁺. ¹H NMR (400 MHz, DMSO-d₆) δ 11.93 (s, 1H), 8.78 (d, J = 2.5 Hz, 1H), 8.16 (dd, J = 8.9, 2.5 Hz, 1H), 7.87 (d, J = 8.9 Hz, 1H), 7.75 - 7.66 (m, 2H), 7.12 (dd, J = 8.9, 2.5 Hz, 1H), 7.00 (d, J = 9.0 Hz, 1H), 6.66 (dd, J = 7.6, 2.3 Hz, 1H), 5.96 (d, J = 2.3 Hz, 1H), 4.58 (dd, J = 9.6, 4.3 Hz, 1H), 4.51 - 4.41 (m, 1H), 4.15 - 4.02 (m, 3H), 3.87 - 3.82 (m, 4H), 3.81 - 3.76 (m, 4H), 3.13 (s, 6H).

### Example I-5: Preparation of Compound I-5

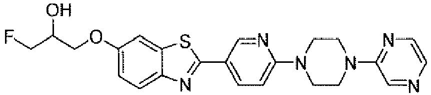

The synthetic method of Example **I-1** was followed, except that 4-bromo-2-(methylamino)pyridine was replaced with 2-bromopyrazine. Compound I-5 was obtained as a brown solid in 15% yield. ESI-MS (positive): 467.2 [M + 1]⁺. ¹H NMR (400 MHz, DMSO-d₆) δ 8.77 (d, J = 2.6 Hz, 1H), 8.38 (s, 1H), 8.19 - 8.11 (m, 2H), 7.92 - 7.84 (m, 2H), 7.71 (d, J = 2.6 Hz, 1H), 7.12 (dd, J = 8.9, 2.6 Hz, 1H), 7.04 (d, J = 9.1 Hz, 1H), 4.58 (dd, J = 9.5, 4.2 Hz, 1H), 4.51 - 4.38 (m, 1H), 4.16 - 4.02 (m, 3H), 3.83 - 3.79 (m, 4H), 3.75 - 3.71 (m, 4H).

### Example I-6: Preparation of Compound I-6

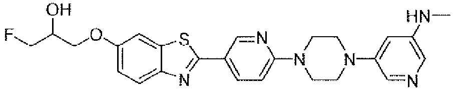

The synthetic method of Example I-1 was followed, except that 4-bromo-2-(methylamino)pyridine was replaced with 5-bromo-3-(methylamino)pyridine. Compound **I-6** was obtained as a yellow solid in 22% yield. ESI-MS (positive): 495.2 [M + 1]⁺. ¹H NMR (400 MHz, DMSO-d₆) δ 8.78 (d, J = 2.5 Hz, 1H), 8.16 (dd, J = 8.9, 2.7 Hz, 1H), 7.87 (d, J = 8.9 Hz, 1H), 7.80 - 7.75 (m, 1H), 7.71 (d, J = 2.6 Hz, 1H), 7.61 - 7.43 (m, 1H), 7.13 (dd, J = 8.9, 2.6 Hz, 1H), 7.09 - 7.04 (m, 1H), 6.98 - 6.90 (m, 1H), 4.58 (dd, J = 9.5, 4.3 Hz, 1H), 4.50 - 4.39 (m, 1H), 4.13 - 4.02 (m, 3H), 3.84 (t, J = 4.8 Hz, 4H), 3.38 - 3.30 (m, 4H), 2.80 (s, 3H).

### Example I-7: Preparation of Compound I-7

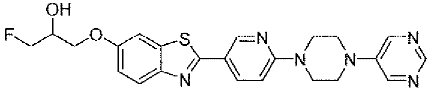

The synthetic method of Example **I-1** was followed, except that 4-bromo-2-(methylamino)pyridine was replaced with 5-bromopyrimidine. Compound I-7 was obtained as a brown solid in 9% yield. ESI-MS (positive): 467.0 [M + 1]⁺. ¹H NMR (400 MHz, DMSO-d₆) δ 8.81 - 8.75 (m, 1H), 8.66 - 8.53 (m, 3H), 8.19 - 8.11 (m, 1H), 7.92 - 7.83 (m, 1H), 7.75 - 7.67 (m, 1H), 7.19 - 7.03 (m, 2H), 4.66 - 4.53 (m, 1H), 4.51 - 4.42 (m, 1H), 4.09 - 4.03 (m, 3H), 3.87 - 3.81 (m, 4H), 3.48 - 3.40 (m, 4H).

### Example I-8: Preparation of Compound I-8

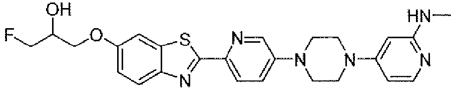

The synthetic method of Example **I-1** was followed, except that 2-fluoro-5-pyridineboronic acid was replaced with 3-fluoro-6-pyridineboronic acid. Compound **I-8** was obtained as a yellow solid in 20% yield. ESI-MS (positive): 495.1 [M + 1]⁺. ¹H NMR (400 MHz, DMSO-d₆) δ 12.04 (s, 1H), 8.42 (d, J = 2.9 Hz, 1H), 8.11 (d, J = 8.8 Hz, 1H), 7.88 (d, J = 8.9 Hz, 1H), 7.70 - 7.63 (m, 2H), 7.55 (d, J = 5.4 Hz, 1H), 7.49 (dd, J = 8.9, 2.9 Hz, 1H), 7.12 (dd, J = 8.9, 2.6 Hz, 1H), 6.65 (dd, J = 7.5, 2.5 Hz, 1H), 5.98 - 5.90 (m, 1H), 4.63 - 4.52 (m, 1H), 4.50 - 4.40 (m, 1H), 4.14 - 4.02 (m, 3H), 3.76 (t, J = 5.0 Hz, 4H), 3.58 (t, J = 5.3 Hz, 4H), 2.87 (d, J = 4.8 Hz, 3H).

### Example I-9: Preparation of Compound I-9

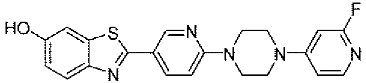

The synthetic method for Intermediate Id-1 of Example **I-1** was followed, except that 4-bromo-2-(methylamino)pyridine was replaced with 4-bromo-2-fluoropyridine. Compound **I-9** was obtained as a yellow solid in 6% yield. ESI-MS (positive): 408.0 [M + 1]⁺. ¹H NMR (400 MHz, DMSO-d₆) δ 9.81 (s, 1H), 8.73 (d, J = 2.5 Hz, 1H), 8.11 (dd, J = 9.0, 2.5 Hz, 1H), 7.84 (d, J = 6.1 Hz, 1H), 7.78 (d, J = 8.8 Hz, 1H), 7.38 (d, J = 2.4 Hz, 1H), 7.01 (d, J = 9.0 Hz, 1H), 6.95 (dd, J = 8.8, 2.5 Hz, 1H), 6.88 - 6.78 (m, 1H), 6.52 (s, 1H), 3.83 - 3.77 (m, 4H), 3.59 - 3.54 (m, 4H).

### Example I-10: Preparation of Compound I-10

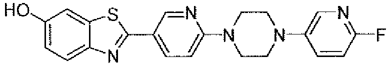

The synthetic method for Intermediate Id-1 of Example **I-1** was followed, except that 4-bromo-2-(methylamino)pyridine was replaced with 5-bromo-2-fluoropyridine. Compound **I-10** was obtained as a yellow solid in 11% yield. ESI-MS (positive): 408.0 [M + 1]⁺. ¹H NMR (400 MHz, DMSO-d₆) δ 9.81 (s, 1H), 8.73 (d, J = 2.5 Hz, 1H), 8.11 (dd, J = 9.0, 2.5 Hz, 1H), 7.92 - 7.86 (m, 1H), 7.78 (d, J = 8.7 Hz, 1H), 7.71 - 7.64 (m, 1H), 7.38 (d, J = 2.5 Hz, 1H), 7.10 - 7.03 (m, 2H), 6.96 (dd, J = 8.8, 2.5 Hz, 1H), 3.81 (t, J = 5.2 Hz, 4H), 3.28 (t, J = 5.1 Hz, 4H).

### Example I-11: Preparation of Compound I-11

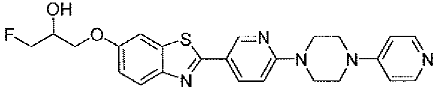

The synthetic method of Example **I-1** was followed, except that 4-bromo-2-(methylamino)pyridine was replaced with 4-bromopyridine. Compound I-11 was obtained as a yellow solid in 21% yield. ESI-MS (positive): 466.0 [M + 1 ]⁺. ¹H NMR (400 MHz, DMSO-d₆) δ 8.78 (d, J = 2.4 Hz, 1H), 8.33 - 8.27 (m, 2H), 8.17 (dd, J = 9.0, 2.5 Hz, 1H), 7.87 (d, J = 8.9 Hz, 1H), 7.71 (d, J = 2.5 Hz, 1H), 7.32 - 7.18 (m, 2H), 7.13 (dd, J = 8.9, 2.5 Hz, 1H), 7.04 - 6.96 (m, 1H), 4.63 - 4.53 (m, 1H), 4.52 - 4.41 (m, 1H), 4.16 - 4.01 (m, 3H), 3.89 (s, 8H).

### Example I-12: Preparation of Compound I-12

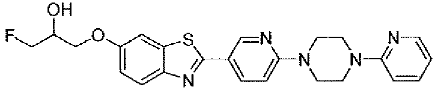

The synthetic method of Example **I-1** was followed, except that 4-bromo-2-(methylamino)pyridine was replaced with 2-bromopyridine. Compound **I-12** was obtained as a yellow solid in 13% yield. ESI-MS (positive): 466.0 [M + 1]⁺. ¹H NMR (400 MHz, DMSO-d₆) δ 8.80 - 8.73 (m, 1H), 8.16 (dd, J = 9.0, 2.6 Hz, 1H), 8.10 (d, 1H), 7.91 - 7.81 (m, 2H), 7.71 (d, J = 2.6 Hz, 1H), 7.21 (d, J = 8.9 Hz, 1H), 7.12 (dd, J = 8.9, 2.6 Hz, 1H), 7.03 (d, J = 9.1 Hz, 1H), 6.86 (t, J = 6.3 Hz, 1H), 4.64 - 4.52 (m, 1H), 4.51 - 4.40 (m, 1H), 4.15 - 4.01 (m, 3H), 3.93 - 3.73 (m, 8H).

### Example I-13: Preparation of Compound I-13

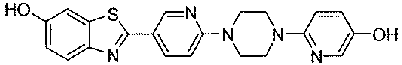

The synthetic method for Intermediate Id-1 of Example **I-1** was followed, except that 4-bromo-2-(methylamino)pyridine was replaced with 2-bromo-5-hydroxypyridine. Compound **I-13** was obtained as a yellow solid in 23% yield. ESI-MS (positive): 405.9 [M + 1]⁺. ¹H NMR (400 MHz, DMSO-d₆) δ 10.01 (s, 1H), 8.74 (d, J = 2.5 Hz, 1H), 8.17 -- 8.09 (m, 1H), 7.78 (d, J = 8.8 Hz, 1H), 7.66 (d, J = 2.8 Hz, 1H), 7.45 (d, J = 9.4 Hz, 1H), 7.40 -- 7.36 (m, 1H), 7.13 (d, J = 9.4 Hz, 1H), 7.04 (d, J = 9.1 Hz, 1H),6.98 -- 6.94 (m, 1H), 3.85 -- 3.78 (m, 4H), 3.63 -- 3.57 (m, 4H).

### Example I-14: Preparation of Compound I-14

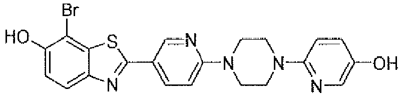

The synthetic method for Intermediate Id-1 of Example **I-1** was followed, except that 4-bromo-2-(methylamino)pyridine was replaced with 2-bromo-5-hydroxypyridine. Compound **I-14** was obtained as a yellow solid in 2% yield. ESI-MS (positive): 484.1 [M + 1]⁺. ¹H NMR (400 MHz, DMSO-d₆) δ 10.69 (s, 1H), 8.77 (s, 1H), 8.18 - 8.09 (m, 1H), 7.81 (d, J = 8.8 Hz, 1H), 7.67 (d, J = 2.9 Hz, 1H), 7.42 (d, J = 9.4 Hz, 1H), 7.15 (d, J = 8.9 Hz, 1H), 7.10 (d, J = 9.4 Hz, 1H), 7.04 (d, J = 9.1 Hz, 1H), 3.83 (t, J = 5.3 Hz, 4H), 3.66 - 3.57 (m, 4H).

### Example I-15: Preparation of Compound I-15

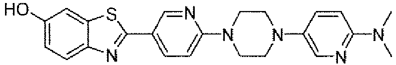

The synthetic method for Intermediate Id-1 of Example **I-1** was followed, except that 4-bromo-2-(methylamino)pyridine was replaced with 5-bromo-2-(dimethylamino)pyridine. Compound **I-15** was obtained as a dark yellow solid in 8% yield. ESI-MS (positive): 433.0 [M + 1]⁺. ¹H NMR (400 MHz, DMSO-d₆) δ 9.76 (s, 1H), 8.67 (d, J = 2.5 Hz, 1H), 8.05 (dd, J = 9.0, 2.5 Hz, 1H), 7.96 (d, J = 9.7 Hz, 1H), 7.71 (d, J = 8.8 Hz, 1H), 7.33 - 7.26 (m, 2H), 7.14 (d, J = 9.9 Hz, 1H), 7.00 (d, J = 9.1 Hz, 1H), 6.89 (dd, J = 8.8, 2.5 Hz, 1H), 3.78 - 3.71 (m, 4H), 3.15 - 3.05 (m, 10H).

### Example I-16: Preparation of Compound I-16

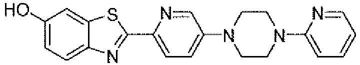

The synthetic method for Intermediate Id-1 of Example **I-1** was followed, except that 2-fluoro-5-pyridineboronic acid was replaced with 3-fluoro-6-pyridineboronic acid, and 4-bromo-2-(methylamino)pyridine was replaced with 2-bromopyridine. Compound **I-16** was obtained as a dark yellow solid in 14% yield. ESI-MS (positive): 389.9 [M + 1]⁺. ¹H NMR (400 MHz, DMSO-d₆) δ 9.83 (s, 1H), 8.43 (d, J = 2.8 Hz, 1H), 8.14 - 8.05 (m, 2H), 7.90 - 7.77 (m, 2H), 7.52 (dd, J = 8.9, 2.9 Hz, 1H), 7.36 (d, J = 2.4 Hz, 1H), 7.22 (d, J = 9.0 Hz, 1H), 6.96 (dd, J = 8.8, 2.5 Hz, 1H), 6.85 (t, J = 6.4 Hz, 1H), 3.82 - 3.75 (m, 4H), 3.60 - 3.54 (m, 4H).

### Example I-17: Preparation of Compound I-17

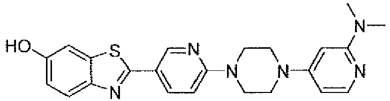

The synthetic method for Intermediate Id-1 of Example **I-1** was followed, except that 4-bromo-2-(methylamino)pyridine was replaced with 4-bromo-2-(dimethylamino)pyridine. Compound **I-17** was obtained as a dark yellow solid in 11% yield. ESI-MS (positive): 433.0 [M + 1]⁺. ¹H NMR (400 MHz, DMSO-d₆) δ 11.91 (s, 1H), 9.83 (s, 1H), 8.74 (d, J = 2.5 Hz, 1H), 8.13 (dd, J = 9.0, 2.6 Hz, 1H), 7.78 (d, J = 8.7 Hz, 1H), 7.70 - 7.64 (m, 1H), 7.38 (d, J = 2.5 Hz, 1H), 7.02 - 6.90 (m, 2H), 6.66 (dd, J = 7.4, 2.4 Hz, 1H), 5.96 (s, 1H), 3.87 - 3.80 (m, 4H), 3.79 - 3.76 (m, 4H), 3.13 (s, 6H).

### Example I-18: Preparation of Compound I-18

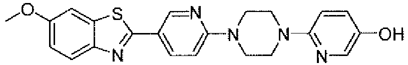

The synthetic method for Intermediate Ic-1 of Example **I-1** was followed, except that 4-bromo-2-(methylamino)pyridine was replaced with 2-bromo-5-hydroxypyridine. Compound **I-18** was obtained as a yellow solid in 35% yield. ESI-MS (positive): 420.1 [M + 1]⁺. ¹H NMR (400 MHz, DMSO-d₆) δ 8.77 (s, 1H), 8.15 (d, J = 8.9 Hz, 1H), 7.91 - 7.83 (m, 1H), 7.72 - 7.64 (m, 2H), 7.43 (d, J = 9.3 Hz, 1H), 7.11 (d, J = 9.2 Hz, 2H), 7.05 (d, J = 9.2 Hz, 1H), 3.85 (s, 3H), 3.84 - 3.80 (m, 4H), 3.61 - 3.56 (m, 4H).

### Example I-19: Preparation of Compound I-19

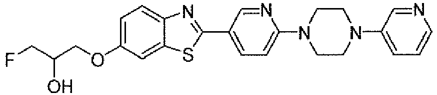

The synthetic method of Example **I-1** was followed, except that 4-bromo-2-(methylamino)pyridine was replaced with 3-bromopyridine. Compound **I-19** was obtained as a yellow solid in 21% yield. ESI-MS (positive): 466.1 [M + 1]⁺. ¹H NMR (400 MHz, DMSO-d₆) δ 8.81 - 8.74 (m, 1H), 8.48 (s, 1H), 8.23 - 8.11 (m, 2H), 8.01 (d, J = 8.7 Hz, 1H), 7.91 - 7.83 (m, 1H), 7.82 - 7.73 (m, 1H), 7.73 - 7.68 (m, 1H), 7.21 - 7.01 (m, 2H), 4.64 - 4.40 (m, 2H), 4.13 - 4.01 (m, 3H), 3.86 (s, 4H), 3.56 (s, 4H).

### Example I-20: Preparation of Compound I-20

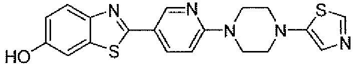

The synthetic method for Intermediate Id-1 of Example **I-1** was followed, except that 4-bromo-2-(methylamino)pyridine was replaced with 5-bromothiazole. Compound **I-20** was obtained as a yellow solid in 17% yield. ESI-MS (positive): 396.2 [M + 1]⁺. ¹H NMR (400 MHz, DMSO-d₆) δ 9.80 (s, 1H), 8.77 - 8.69 (m, 1H), 8.45 (s, 1H), 8.11 (dd, J = 9.0, 2.5 Hz, 1H), 7.78 (d, J = 8.8 Hz, 1H), 7.38 (d, J = 2.5 Hz, 1H), 7.19 (s, 1H), 7.06 (d, J = 9.0 Hz, 1H), 6.95 (dd, J = 8.8, 2.5 Hz, 1H), 3.81 (t, J = 5.0 Hz, 4H), 3.21 (t, J = 5.0 Hz, 4H).

### Example I-21: Preparation of Compound 1-21

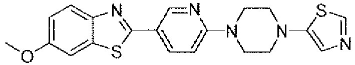

The synthetic method for Intermediate Ic-1 of Example I-1 was followed, except that 4-bromo-2-(methylamino)pyridine was replaced with 5-bromothiazole. Compound **I-21** was obtained as a yellow solid in 35% yield. ESI-MS (positive): 410.1 [M + 1]⁺. ¹H NMR (400 MHz, Chloroform-d) δ 8.72 (s, 1H), 8.28 (s, 1H), 8.13 (d, J = 9.0 Hz, 1H), 7.82 (d, J = 9.0 Hz, 1H), 7.18 (s, 1H), 7.10 (s, 1H), 7.03 - 6.95 (m, 1H), 6.70 (d, J = 9.0 Hz, 1H), 3.84 - 3.75 (m, 8H), 3.23 - 3.12 (m, 3H).

### Example I-22: Preparation of Compound 1-22

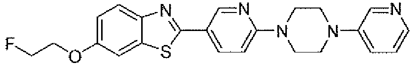

The synthetic method of Example **I-1** was followed, except that 4-bromo-2-(methylamino)pyridine was replaced with 3-bromopyridine, and epifluorohydrin was replaced with 1-bromo-2-fluoroethane. Compound **I-22** was obtained as a yellow solid in 25% yield. ESI-MS (positive): 436.1 [M + 1]⁺. ¹H NMR (400 MHz, DMSO-d₆) δ 8.79 (s, 1H), 8.46 (s, 1H), 8.24 - 8.11 (m, 2H), 7.97 - 7.82 (m, 2H), 7.75 - 7.60 (m, 2H), 7.18 - 7.01 (m, 2H), 4.92 - 4.56 (m, 2H), 4.43 - 4.10 (m, 2H), 3.88 - 3.82 (m, 4H), 3.56 - 3.45 (m, 4H).

### Example 1-23: Preparation of Compound I-23

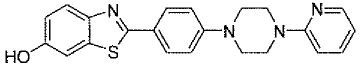

The synthetic method for Intermediate Id-1 of Example **1-1** was followed, except that 2-fluoro-5-pyridineboronic acid was replaced with 4-fluorophenylboronic acid, and 4-bromo-2-(methylamino)pyridine was replaced with 2-bromopyridine. Compound **1-23** was obtained as a yellow solid in 19% yield. ESI-MS (positive): 388.9 [M + 1]⁺. ¹H NMR (400 MHz, DMSO-d₆) δ 9.78 (s, 1H), 8.11 (dd, J = 5.7, 1.9 Hz, 1H), 7.94 - 7.78 (m, 3H), 7.76 (d, J = 8.8 Hz, 1H), 7.36 (d, J = 2.4 Hz, 1H), 7.20 (d, J = 8.8 Hz, 1H), 7.10 (d, J = 8.9 Hz, 2H), 6.94 (dd, J = 8.8, 2.4 Hz, 1H), 6.84 (t, J = 6.4 Hz, 1H), 3.76 (t, J = 5.0 Hz, 4H), 3.50 (t, J = 5.3 Hz, 4H).

### Example 1-24: Preparation of Compound 1-24

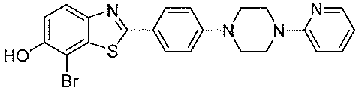

The synthetic method for Intermediate Id-1 of Example **I-1** was followed, except that 2-fluoro-5-pyridineboronic acid was replaced with 4-fluorophenylboronic acid, and 4-bromo-2-(methylamino)pyridine was replaced with 2-bromopyridine. Compound **I-24** was obtained as a yellow solid in 2% yield. ESI-MS (positive): 466.9 [M + I ]⁺, ¹H NMR (400 MHz, DMSO-d₆) δ 10.63 (s, 1H), 8.10 (dd, J = 5.6, 1.8 Hz, 1H), 7.90 (d, J = 8.6 Hz, 2H), 7.86 - 7.76 (m, 2H), 7.25 - 7.17 (m, 1H), 7.16 - 7.07 (m, 3H), 6.85 (t, J = 6.4 Hz, 1H), 3.77 (t, J = 5.0 Hz, 4H), 3.53 (t, J = 5.2 Hz, 4H).

### Example I-25: Preparation of Compound 1-25

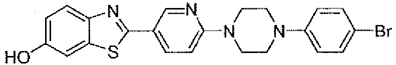

The synthetic method for Intermediate Id-1 of Example **I-1** was followed, except that 4-bromo-2-(methylamino)pyridine was replaced with 4-bromophenylboronic acid. Compound **1-25** was obtained as a yellow solid in 19% yield. ESI-MS (positive): 466.8 [M + 1]⁺. ¹H NMR (400 MHz, DMSO-d₆) δ 9.80 (s, 1H), 8.73 (d, J = 2.5 Hz, 1H), 8.11 (dd, J = 9.0, 2.5 Hz, 1H), 7.78 (d, J = 8.7 Hz, 1H), 7.47 - 7.30 (m, 3H), 7.04 (d, J = 9.1 Hz, 1H), 7.01 - 6.90 (m, 3H), 3.79 (t, J = 5.3 Hz, 4H), 3.28 (t, J = 5.1 Hz, 4H).

### Example I-26: Preparation of Compound 1-26

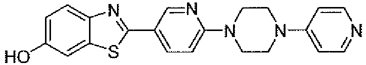

The synthetic method for Intermediate Id-1 of Example 1-1 was followed, except that 4-bromo-2-(methylamino)pyridine was replaced with 4-bromopyridine. Compound **I-26** was obtained as a yellow solid in 22% yield. ESI-MS (positive): 489.9 [M + 1]⁺. ¹H NMR (400 MHz, DMSO-d₆) δ 13.42 (s, 1H), 9.82 (s, 1H), 8.75 (d, J = 2.5 Hz, 1H), 8.29 (d, J = 7.0 Hz, 2H), 8.14 (dd, J = 9.0, 2.5 Hz, 1H), 7.78 (d, J = 8.8 Hz, 1H), 7.3 8 (d, J = 2.4 Hz, 1H), 7.22 (d, J = 7.1 Hz, 2H), 7.08 - 6.89 (m, 2H), 3.88 (s, 8H).

### Example I-27: Preparation of Compound 1-27

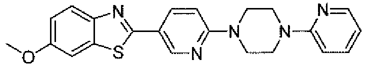

The synthetic method for Intermediate Ic-1 of Example **I-1** was followed, except that 4-bromo-2-(methylamino)pyridine was replaced with 2-bromopyridine. Compound **I-27** was obtained as a yellow solid in 40% yield. ESI-MS (positive): 403.9 [M + 1]⁺. ¹H NMR (400 MHz, Chloroform-d) δ 8.80 (s, 1H), 8.29 - 8.14 (m, 2H), 7.89 (d, J = 8.9 Hz, 1H), 7.54 (t, J = 8.0 Hz, 1H), 7.34 (s, 1H), 7.07 (d, J = 8.7, 2.2 Hz, 1H), 6.79 - 6.63 (m, 3H), 3.89 (s, 3H), 3.87 - 3.81 (m, 4H), 3.76 - 3.71 (m, 4H).

### Example 1-28: Preparation of Compound I-28

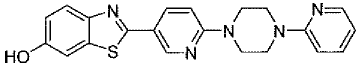

The synthetic method for Intermediate Id-1 of Example **I-1** was followed, except that 4-bromo-2-(methylamino)pyridine was replaced with 2-bromopyridine. Compound **I-28** was obtained as a yellow solid in 35% yield. ESI-MS (positive): 389.9 [M + 1]⁺. ¹H NMR (400 MHz, DMSO-d₆) δ 8.74 (d, J = 2.4 Hz, 1H), 8.16 (dd, J = 9.1, 2.4 Hz, 1H), 8.07 (d, J = 6.1 Hz, 1H), 8.02 (t, J = 8.3 Hz, 1H), 7.79 (d, J = 8.8 Hz, 1H), 7.42 - 7.37 (m, 2H), 7.06 (d, J = 9.1 Hz, 1H), 7.00 - 6.92 (m, 2H), 3.93 - 3.81 (m, 8H).

### Example I-29: Preparation of Compound 1-29

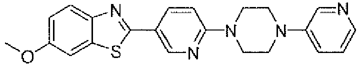

The synthetic method for Intermediate Ic-1 of Example **I-1** was followed, except that 4-bromo-2-(methylamino)pyridine was replaced with 3-bromopyridine. Compound **I-29** was obtained as a yellow solid in 41% yield. ESI-MS (positive): 403.9 [M + 1]⁺. ¹H NMR (400 MHz, DMSO-d₆) δ 8.78 (d, J = 2.4 Hz, 1H), 8.49 (d, J = 2.8 Hz, 1H), 8.22 - 8.12 (m, 2H), 7.99 (dd, J = 8.9, 2.8 Hz, 1H), 7.88 (d, J = 8.9 Hz, 1H), 7.76 (dd, J = 8.9, 5.1 Hz, 1H), 7.69 (d, J = 2.5 Hz, 1H), 7.19 - 6.90 (m, 2H), 3.90 - 3.81 (m, 7H), 3.54 (t, 4H).

### Example 1-30: Preparation of Compound I-30

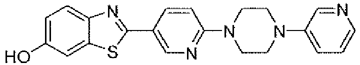

The synthetic method for Intermediate Id-1 of Example **I-1** was followed, except that 4-bromo-2-(methylamino)pyridine was replaced with 3-bromopyridine. Compound **I-30** was obtained as a yellow solid in 40% yield. ESI-MS (positive): 389.9 [M + 1]⁺. ¹H NMR (400 MHz, DMSO-d₆) δ 9.84 (s, 1H), 8.75 (d, J = 2.5 Hz, 1H), 8.49 (d, J = 2.8 Hz, 1H), 8.21 (d, J = 5.2 Hz, 1H), 8.13 (dd, J = 9.0, 2.5 Hz, 1H), 8.04 (dd, J = 9.2, 2.7 Hz, 1H), 7.83 - 7.75 (m, 2H), 7.38 (d, J = 2.4 Hz, 1H), 7.07 (d, J = 9.1 Hz, 1H), 6.96 (dd, J = 8.8, 2.4 Hz, 1H), 3.93 - 3.75 (m, 4H), 3.64 - 3.53 (m, 4H).

### Example 1-31: Preparation of Compound 1-31

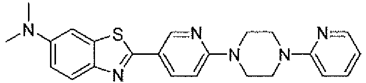

The synthetic method for Intermediate Ic-1 of Example **I-1** was followed, except that 6-methoxy-2-methylbenzothiazole was replaced with 6-(dimethylamino)-2-methylbenzothiazole, and 4-bromo-2-(methylamino)pyridine was replaced with 2-bromopyridine. Compound **I-31** was obtained as a yellow solid in 37% yield. ESI-MS (positive): 417.0 [M + 1]⁺. ¹H NMR (400 MHz, DMSO-d₆) δ 8.73 (d, J = 2.5 Hz, 1H), 8.15 - 8.04 (m, 2H), 7.86 (t, J = 8.1 Hz, 1H), 7.78 (d, J = 9.0 Hz, 1H), 7.33 (d, J = 2.6 Hz, 1H), 7.22 (d, J = 9.0 Hz, 1H), 7.05 - 6.95 (m, 2H), 6.86 (t, J = 6.5 Hz, 1H), 3.87 - 3.81 (m, 4H), 3.79 - 3.74 (m, 4H), 2.99 (s, 6H).

### Example 1-32: Preparation of Compound 1-32

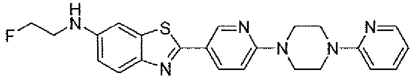

The synthetic route for Compound I-32 is as follows:

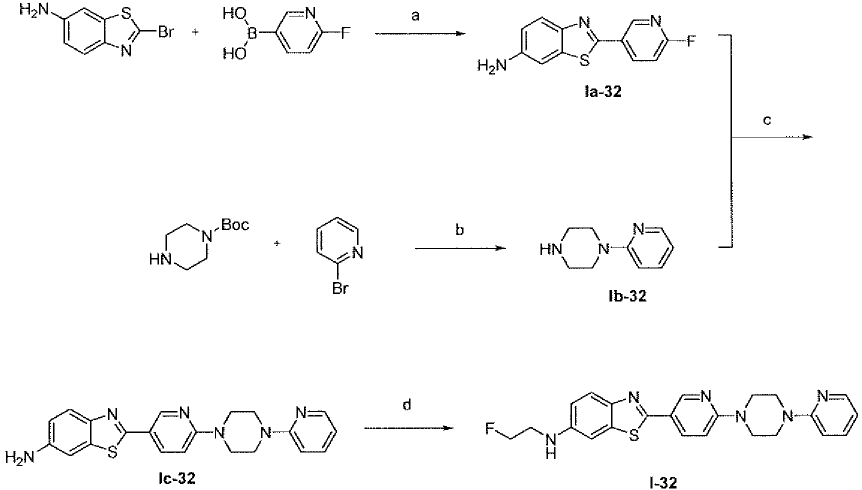

### Preparation of Intermediate Ic-32:

Intermediate Ic-32 was prepared according to the synthetic method for Intermediate Ic-1 of Example **I-1,** except that 6-methoxy-2-bromobenzothiazole was replaced with 6-amino-2-bromobenzothiazole, and 4-bromo-2-(methylamino)pyridine was replaced with 3-bromopyridine. Compound Ic-32 was obtained as a yellow solid in 31% yield. ESI-MS (positive): 389.1 [M + 1]⁺.

### Preparation of Compound I-32:

Intermediate Ic-32 (73 mg, 0.2 mmol) was dissolved in 2 mL of NMP. Cs₂CO₃ (195 mg, 0.6 mmol) and 1-bromo-2-fluoroethane (35 µL, 0.4 mmol) were added. The mixture was reacted at 100 °C for 12 h. After the reaction, the reaction solution was poured into 5 mL of water, extracted with 20 mL of ethyl acetate, washed three times with brine, dried over anhydrous sodium sulfate, and purified by column chromatography to give Compound **1-32** as a yellow solid in 1.8% yield. ESI-MS (positive): 435.0 [M + 1]⁺. ¹H NMR (400 MHz, DMSO-d₆) δ 8.88 - 8.67 (m, 1H), 8.21 - 8.05 (m, 2H), 7.87 - 7.60 (m, 2H), 7.29 - 6.74 (m, 6H), 4.81 - 4.46 (m, 2H), 4.01 - 3.66 (m, 8H), 3.60 - 3.31 (m, 2H).

### Example 1-33: Preparation of Compound 1-33

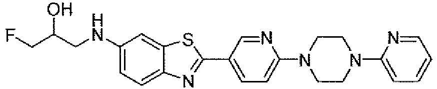

The synthetic method of Example **1-32** was followed, except that 1-bromo-2-fluoroethane was replaced with epifluorohydrin. Compound **I-33** was obtained as a yellow solid in 5% yield. ESI-MS (positive): 465.1 [M + 1]⁺. ¹H NMR (400 MHz, DMSO-d₆) δ 8.71 (s, 1H), 8.10 (d, J = 6.8 Hz, 2H), 7.86 (t, J = 8.1 Hz, 1H), 7.68 (d, J = 8.8 Hz, 1H), 7.22 (d, J = 9.2 Hz, 1H), 7.14 (s, 1H), 7.01 (d, J = 8.9 Hz, 1H), 6.91 - 6.80 (m, 2H), 4.55 - 4.44 (m, 1H), 4.43 - 4.30 (m, 1H), 3.97 - 3.92 (m, 1H), 3.86 - 3.83 (m, 4H), 3.79 - 3.75 (m, 4H), 3.21 (dd, J = 13.3, 5.8 Hz, 1H), 3.09 (dd, J = 13.2, 6.3 Hz, 1H).

### Example I-34: Preparation of Compound 1-34

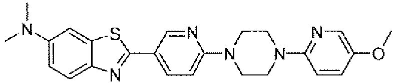

The synthetic method for Intermediate Ic-1 of Example **I-1** was followed, except that 6-methoxy-2-bromobenzothiazole was replaced with 6-(dimethylamino)-2-bromobenzothiazole, and 4-bromo-2-(methylamino)pyridine was replaced with 2-bromo-5-methoxypyridine. Compound 1-34 was obtained as a yellow solid in 9% yield. ESI-MS (positive): 447.1 [M + 1]⁺. ¹H NMR (400 MHz, DMSO-d₆) δ 8.72 (s, 1H), 8.14 - 8.07 (m, 1H), 7.87 (s, 1H), 7.78 (d, J = 8.9 Hz, 1H), 7.44 - 7.38 (m, 1H), 7.33 (s, 1H), 7.04 - 6.96 (m, 3H), 3.76 (s, 3H), 3.59 - 3.53 (m, 4H), 3.08 - 3.01 (m, 4H), 2.99 (s, 6H).

### Example 1-35: Preparation of Compound I-35

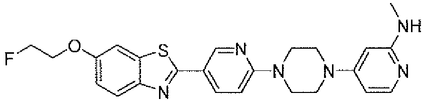

The synthetic method of Example **1-1** was followed, except that epifluorohydrin was replaced with 1-bromo-2-fluoroethane. Compound **1-35** was obtained as a yellow solid in 12% yield. ESI-MS (positive): 465.2 [M + 1]⁺. ¹H NMR (400 MHz, DMSO-d₆) δ 8.78 (d, J = 2.5 Hz, 1H), 8.15 (dd, J = 8.9, 2.5 Hz, 1H), 7.88 (d, J = 8.9 Hz, 1H), 7.75 - 7.65 (m, 2H), 7.27 - 7.10 (m, 2H), 7.00 (d, J = 9.1 Hz, 1H), 6.50 (dd, J = 7.1, 2.4 Hz, 1H), 5.90 (d, J = 2.4 Hz, 1H), 4.87 - 4.82 (m, 1H), 4.75 - 4.71 (m, 1H), 4.37 (t, J = 3.8 Hz, 1H), 4.31 - 4.27 (m, 1H), 3.87 - 3.77 (m, 4H), 3.70 - 3.60 (m, 4H), 2.83 (d, J = 4.8 Hz, 3H).

### Example 1-36: Preparation of Compound I-36

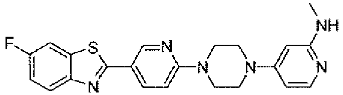

The synthetic method for Intermediate Ic-1 of Example **I-1** was followed, except that 6-methoxy-2-bromobenzothiazole was replaced with 6-fluoro-2-methylbenzothiazole. Compound **1-36** was obtained as a yellow solid in 20% yield. ESI-MS (positive): 421.3 [M + 1]⁺. ¹H NMR (400 MHz, DMSO-d₆) δ 12.07 (s, 1H), 8.82 (d, J = 2.5 Hz, 1H), 8.19 (dd, J = 9.0, 2.6 Hz, 1H), 8.10 - 7.92 (m, 2H), 7.72 - 7.64 (m, 1H), 7.58 (d, J = 5.3 Hz, 1H), 7.44 - 7.32 (m, 1H), 7.00 (d, J = 9.0 Hz, 1H), 6.61 (dd, J = 7.5, 2.5 Hz, 1H), 5.92 (s, 1H), 3.89 - 3.84 (m, 4H), 3.77 - 3.71 (m, 4H), 2.86 (d, J = 4.8 Hz, 3H).

### Example I-37: Preparation of Compound I-37

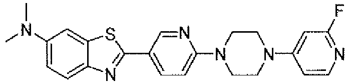

The synthetic method for Intermediate Ic-1 of Example **I-1** was followed, except that 6-methoxy-2-bromobenzothiazole was replaced with 6-(dimethylamino)-2-bromobenzothiazole, and 4-bromo-2-(methylamino)pyridine was replaced with 4-bromo-2-fluoropyridine. Compound **I-37** was obtained as a yellow solid in 34% yield. ESI-MS (positive): 435.2 [M + 1]⁺. ¹H NMR (400 MHz, DMSO-d₆) δ 8.73 (s, 1H), 8.12 (d, J = 8.9 Hz, 1H), 7.91 - 7.74 (m, 2H), 7.36 (s, 1H), 7.07 - 6.95 (m, 2H), 6.95 - 6.79 (m, 1H), 6.53 (s, 1H), 3.82 - 3.78 (m, 4H), 3.31 - 3.29 (m, 4H), 3.00 (s, 6H).

### Example 1-38: Preparation of Compound I-38

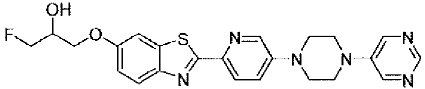

The synthetic method of Example **I-1** was followed, except that 4-bromo-2-(methylamino)pyridine was replaced with 5-bromopyrimidine. Compound 1-38 was obtained as a yellow solid in 16% yield. ESI-MS (positive): 467.3 [M + 1]⁺. ¹H NMR (400 MHz, DMSO-d₆) δ 8.64 (s, 1H), 8.59 (s, 2H), 8.48 (d, J = 2.9 Hz, 1H), 8.10 (d, J = 8.8 Hz, 1H), 7.89 (d, J = 8.9 Hz, 1H), 7.70 (d, J = 2.6 Hz, 1H), 7.57 (dd, J = 8.9, 2.9 Hz, 1H}, 7.12 (dd, J = 8.9, 2.6 Hz, 1H), 4.63 - 4.52 (m, 1H), 4.51 - 4.41 (m, 1H), 4.13 - 4.01 (m, 3H), 3.62 - 3.53 (m, 4H), 3.49 - 3.44 (m, 4H).

### Example I-39: Preparation of Compound 1-39

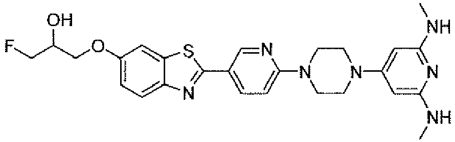

The synthetic method of Example **I-1** was followed, except that 4-bromo-2-(methylamino)pyridine was replaced with 4-bromo-N²,N⁶-dimethylpyridine-2,6-diamine. Compound **1-39** was obtained as a yellow solid in 10% yield. ESI-MS (positive): 524.3 [M + 1]⁺. ¹H NMR (400 MHz, DMSO-d₆) δ 10.97 (s, 1H), 8.77 (d, J = 2.5 Hz, 1H), 8.16 (dd, J = 9.0, 2.6 Hz, 1H), 7.87 (d, J = 8.9 Hz, 1H), 7.71 (d, J = 2.5 Hz, 1H), 7.12 (dd, J = 8.9, 2.6 Hz, 1H), 7.03 - 6.86 (m, 3H), 5.34 (s, 2H), 4.64 - 4.52 (m, 1H), 4.51 - 4.41 (m, 1H), 4.15 - 3.98 (m, 3H), 3.86 - 3.79 (m, 4H), 3.71 - 3.67 (m, 4H), 2.80 (d, J = 4.5 Hz, 6H).

### Example 1-40: Preparation of Compound I-40

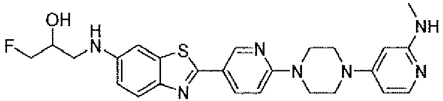

The synthetic method of Example **I-32** was followed, except that 1-bromo-2-fluoroethane was replaced with epifluorohydrin, and 4-bromo-2-(methylamino)pyridine was replaced with 2-bromopyridine. Compound **I-40** was obtained as a yellow solid in 7% yield. ESI-MS (positive): 494.2 [M + 1]⁺. ¹H NMR (400 MHz, DMSO-d₆) δ 12.17 - 11.96 (m, 1H), 8.79 - 8.66 (m, 1H), 8.14 - 8.07 (m, 1H), 7.72 - 7.62 (m, 2H), 7.61 - 7.54 (m, 1H), 7.15 - 7.10 (m, 1H), 7.02 - 6.94 (m, 1H), 6.86 (dd, J = 8.9, 2.3 Hz, 1H), 6.62 (dd, J = 7.7, 2.4 Hz, 1H), 5.92 (s, 1H), 4.56 - 4.43 (m, 1H), 4.43 - 4.31 (m, 1H), 3.97 - 3.79 (m, 3H), 2.86 (d, J = 4.7 Hz, 3H).

### Example 1-41: Preparation of Compound I-41

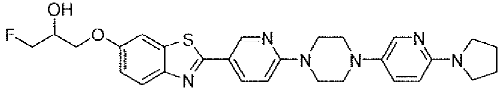

The synthetic method of Example **I-1** was followed, except that 4-bromo-2-(methylamino)pyridine was replaced with 5-bromo-2-(pyrrolidin-1-yl)pyridine. Compound **I-41** was obtained as a yellow solid in 35% yield. ESI-MS (positive): 535.2 [M + 1]⁺. ¹H NMR (400 MHz, DMSO-d₆) δ 8.76 (d, J = 2.4 Hz, 1H), 8.41 (s, 1H), 8.13 (dd, J = 8.9, 2.3 Hz, 1H), 8.00 (dd, J = 9.7, 2.5 Hz, 1H), 7.88 (d, J = 8.9 Hz, 1H), 7.73 (d, J = 2.5 Hz, 1H), 7.25 (d, J = 2.6 Hz, 1H), 7.14 - 6.97 (m, 3H), 4.62 - 4.53 (m, 1H), 4.50 - 4.40 (m, 1H), 4.13 - 4.00 (m, 3H), 3.81 (t, J = 4.9 Hz, 4H), 3.12 (t, J = 4.9 Hz, 4H), 2.07 - 1.99 (m, 1H).

### Example I-42: Preparation of Compound 1-42

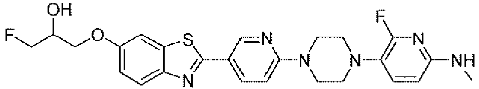

The synthetic method of Example **I-1** was followed, except that 4-bromo-2-(methylamino)pyridine was replaced with 5-bromo-6-fluoro-2-(methylamino)pyridine. Compound **I-42** was obtained as a yellow solid in 24% yield. ESI-MS (positive): 513.2 [M + 1]⁺. ¹H NMR (400 MHz, DMSO-d₆) δ 8.79 (d, J = 2.5 Hz, 1H), 8.43 (s, 1H), 8.12 (dd, J = 9.0, 2.5 Hz, 1H), 8.00 (d, J = 9.8 Hz, 1H), 7.86 (d, J = 8.9 Hz, 1H), 7.73 (d, J = 2.6 Hz, 1H), 7.12 - 6.97 (m, 3H), 4.61 - 4.52 (m, 1H), 4.50 - 4.40 (m, 1H), 4.15 - 4.00 (m, 3H), 3.81 (t, J = 4.9 Hz, 4H), 3.12 (t, J = 4.9 Hz, 4H), 2.94 (s, 3H).

### Example 1-43: Preparation of Compound I-43

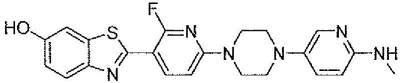

The synthetic method for Intermediate Id-1 of Example **I-1** was followed, except that 4-bromo-2-(methylamino)pyridine was replaced with 5-bromo-2-(methylamino)pyridine, and 2-fluoro-5-pyridineboronic acid was replaced with (2,6-difluoropyridin-3-yl)boronic acid. Compound **1-43** was obtained as a yellow solid in 27% yield. ESI-MS (positive): 437.1 [M + 1]⁺. ¹H NMR (400 MHz, DMSO-d₆) δ 9.83 (s, 1H), 8.41 (s, 1H), 8.14 (d, J = 9.0 Hz, 1H), 8.01 (dd, J = 9.8, 2.7 Hz, 1H), 7.85 (d, J = 8.9 Hz, 1H), 7.70 (d, J = 2.6 Hz, 1H), 7.25 (d, J = 2.7 Hz, 1H), 7.17 - 7.03 (m, 3H), 4.61 - 4.52 (m, 1H), 4.51 - 4.45 (m, 1H), 4.13 - 4.01 (m, 3H), 3.81 (t, J = 5.1 Hz, 4H), 3.23 (t, J = 5.1 Hz, 4H), 2.97 (s, 3H).

### Example I-44: Preparation of Compound I-44

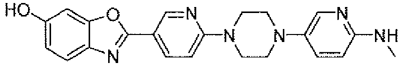

The synthetic method for Intermediate Id-1 of Example **1-1** was followed, except that 6-methoxy-2-bromobenzothiazole was replaced with 6-methoxy-2-bromobenzoxazole, and 4-bromo-2-(methylamino)pyridine was replaced with 5-bromo-2-(methylamino)pyridine. Compound **I-44** was obtained as a yellow solid in 22% yield. ESI-MS (positive): 403.2 [M + 1]⁺. ¹H NMR (400 MHz, DMSO-d₆) δ 9.93 (s, 1H), 8.41 (s, 1H), 8.13 - 8.00 (m, 2H), 7.87 - 7.80 (m, 1H), 7.72 (d, J = 2.6 Hz, 1H), 7.23 (d, J = 2.6 Hz, 1H), 7.15 - 7.05 (m, 3H), 3.82 (t, J = 5.1 Hz, 4H), 3.21 (t, J = 5.1 Hz, 4H), 2.93 (s, 3H).

### Example I-45: Preparation of Compound 1-45

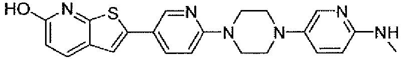

The synthetic method for Intermediate Id-1 of Example **I-1** was followed, except that 6-methoxy-2-bromobenzothiazole was replaced with 2-bromo-6-methoxythieno[2,3-b]pyridine, and 4-bromo-2-(methylamino)pyridine was replaced with 5-bromo-2-(methylamino)pyridine. Compound **I-45** was obtained as a yellow solid in 13% yield. ESI-MS (positive): 419.2 [M + 1]⁺. ¹H NMR (400 MHz, DMSO-d₆) δ 10.98 (s, 1H), 8.45 (s, 1H), 7.78 - 7.66 (m, 3H), 7.17 - 7.07 (m, 2H), 6.82 - 6.54 (m, 4H), 3.81 (t, J = 5.0 Hz, 4H), 3.25 (t, J = 5.0 Hz, 4H), 2.90 (s, 3H).

### Example 1-46: Preparation of Compound I-46

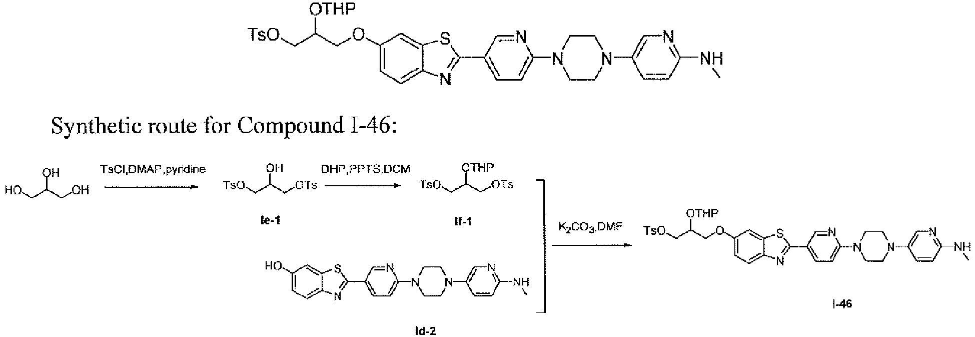

### Step 1: Preparation of Intermediate Ie-1

Glycerol (1 g, 11 mmol) and 4-dimethylaminopyridine (DMAP) (335 mg, 2.75 mmol) were dissolved in pyridine (10 mL). p-Toluenesulfonyl chloride (TsCl) (4.4 g, 23 mmol) dissolved in pyridine (15 mL) was added dropwise at 0 °C. The mixture was reacted at room temperature overnight. After the reaction was complete, the reaction solution was diluted in 100 mL of water, concentrated HCl was added dropwise to adjust the pH to acidic, and the mixture was extracted with DCM (100 mL × 5). The organic phase was washed with IN HCl (100 mL), then with water (100 mL), and finally purified by flash silica gel column chromatography (PE/EA) to give the product Ie-1 (2.8 g, 7 mmol) as a colorless oily liquid in 64% yield. MS-ESI: m/z 401 [M + H]⁺.

### Step 2: Preparation of Intermediate If-1

Ie-1 (1.2 g, 3 mmol) and 3,4-dihydropyran (DHP) (5.5 mL, 60 mmol) were dissolved in DCM (10 mL). Pyridine p-toluenesulfonate (PPTS) (150 mg, 0.6 mmol) was added, and the mixture was reacted at 40 °C overnight. After the reaction was complete, the reaction solution was concentrated and then purified by flash silica gel column chromatography (PE/EA) to give the product If-1 (1.3 g, 2.7 mmol) as a light yellow oily liquid in 89% yield. MS-ESI: m/z 485 [M + H]⁺.

### Step 3: Preparation of Product I-46

Id-2 (0.06 mmol), If-1 (0.29 mmol), and potassium carbonate (16 mg, 0.12 mmol) were added to DMF (0.5 mL) and reacted at 80 °C for 2 h. After the reaction was complete, the mixture was purified by flash silica gel column chromatography (PE/EA) to give the product **I-46** (3.8 mg, 0.006 mmol) as a yellow solid in 69% yield. MS-ESI: m/z 731.2 [M + H]⁺.

### Example I-47: Preparation of Compound 1-47

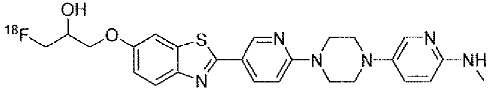

### Method 1:

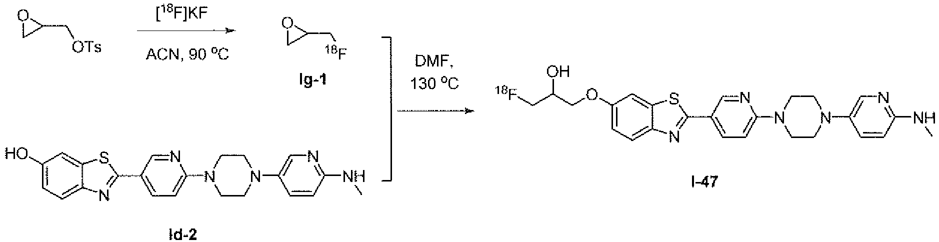

150 mCi of ¹⁸F was trapped on a QMA column. The ¹⁸F was eluted into the reaction vial with 0.6 mL of eluent (150 mg Kryptofix 222, 15 mg K₂CO₃ dissolved in 9 mL acetonitrile and 1 mL water). The solvent was dried under high-purity nitrogen by heating at 110°C for 20 min. 0.2 mg of oxiran-2-ylmethyl 4-methylbenzenesulfonate and 0.2 mL of acetonitrile were added to the reaction vial, and the mixture was reacted at 90 °C for 10 min. The acetonitrile was evaporated, and 0.2 mL of DMF and 0.5 mg of Id-2 were added, followed by reaction at 130 °C for 10 min. The mixture was separated and purified by HPLC (HPLC conditions: CAPCELL PAK C18 UG80 10mm×250mm, acetonitrile: water = 26:74, flow rate: 3 mL/min). The product was collected and subjected to solid-phase extraction using a C18 column, and eluted with ethanol to obtain the final product I-47, with a labeling efficiency of 8%.

### Method 2:

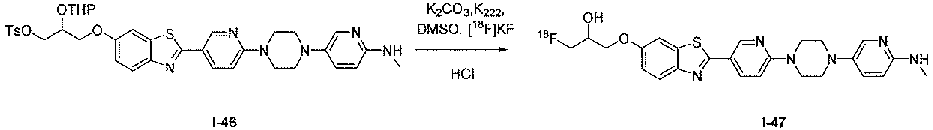

150 mCi of ¹⁸F was trapped on a QMA column. The ¹⁸F was eluted into the reaction vial with 0.6 mL of eluent (150 mg Kryptofix 222, 15 mg K₂CO₃ dissolved in 9 mL acetonitrile and 1 mL water). The solvent was dried under high-purity nitrogen by heating at 110 °C for 20 min. 0.5 mg of I-46 was added to the reaction vial and reacted at 140 °C for 10 min. 0.2 mL of 2N HCl was then added, and the mixture was reacted at 105 °C for 8 min to remove the THP protecting group. The reaction solution was separated and purified by HPLC (HPLC conditions: CAPCELL PAK C18 UG80 10mm×250mm, acetonitrile: water = 26:74, flow rate: 3 mL/min). The product was collected and subjected to solid-phase extraction using a C18 column, and eluted with ethanol to obtain the final product **1-47,** with a labeling efficiency of 10%.

### Example 1-48: Preparation of Compound I-48

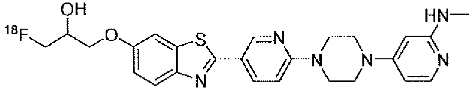

The synthetic method of Example **1-47** was followed, except that the intermediate was replaced with the corresponding starting material.

### Example I-49: Preparation of Compound I-49

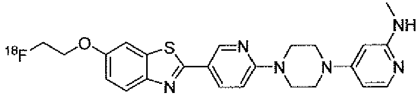

### Synthetic route for Compound 1-49:

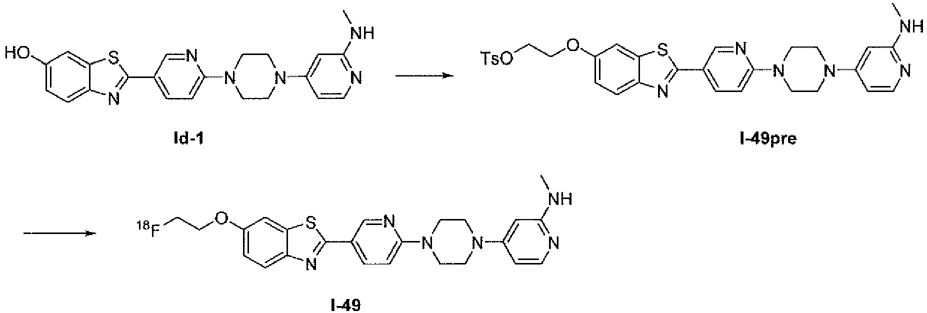

### Step 1: Synthesis of Precursor I-49pre

Id-1 (0.06 mmol), diethylene glycol ditosylate (0.29 mmol), and potassium carbonate (16 mg, 0.12 mmol) were added to DMF (0.5 mL) and reacted at 80 °C for 2 h. After the reaction was complete, the mixture was purified by flash silica gel column chromatography (PE/EA) to give the product I-49pre (3.8 mg, 0.006 mmol) as a yellow solid in 83% yield. MS-ESI: m/z 617.2 [M + H]⁺.

### Step 2: Radiolabeling of I-49

150 mCi of ¹⁸F was trapped on a QMA column. The ¹⁸F was eluted into the reaction vial with 0.6 mL of eluent (150 mg Kryptofix 222, 15 mg K₂CO₃ dissolved in 9 mL acetonitrile and 1 mL water). The solvent was dried under high-purity nitrogen by heating at 110 °C for 20 min. 0.5 mg of I-49pre was added to the reaction vial and reacted at 140 °C for 10 min. The reaction solution was separated and purified by HPLC (HPLC conditions: CAPCELL PAK C18 UG80 10mm×250mm, acetonitrile: water = 29:71, flow rate: 3 mL/min). The product was collected and subjected to solid-phase extraction using a C18 column, and eluted with ethanol to obtain the final product 1-49, with a labeling efficiency of 18%.

### Example I-50: Preparation of Compound I-50

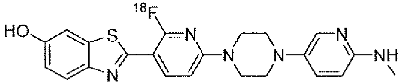

### Synthetic route for Compound I-50:

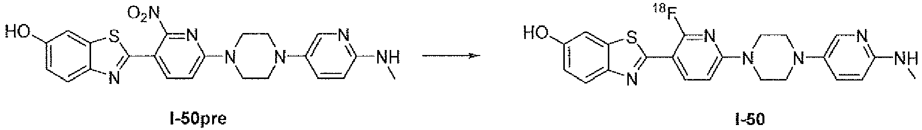

### Step 1: Synthesis of Precursor I-50pre

The synthetic method was the same as that of Example 1-43, except that 2-fluoro-5-boronic acid pyridine was replaced with 2-fluoro-5-boronic acid-6-fluoro-pyridine. MS-ESI: m/z 464.1 [M + H]⁺.

### Step 2: Radiolabeling of I-50

150 mCi of ¹⁸F was trapped on a QMA column. The ¹⁸F was eluted into the reaction vial with 0.6 mL of eluent (150 mg Kryptofix 222, 15 mg K₂CO₃ dissolved in 9 mL acetonitrile and 1 mL water). The solvent was dried under high-purity nitrogen by heating at 110 °C for 20 min. 0.7 mg of I-50pre was added to the reaction vial and reacted at 140 °C for 10 min. The reaction solution was separated and purified by HPLC (HPLC conditions: CAPCELL PAK C18 UG80 10mm×250mm, acetonitrile: water = 20:80, flow rate: 3 mL/min). The product was collected and subjected to solid-phase extraction using a C18 column, and eluted with ethanol to obtain the final product **I-50,** with a labeling efficiency of 5%.

### Example 1-51: Preparation of Compound I-51

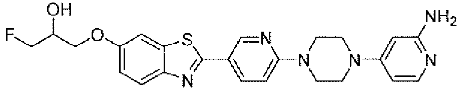

The synthetic method of Example **1-1** was followed, except that 4-bromo-2-(methylamino)pyridine was replaced with 4-bromopyridin-2-amine. Compound I-51 was obtained as a yellow solid in 12% yield. ESI-MS (positive): 481.2 [M + 1]⁺. ¹H NMR (400 MHz, DMSO-d₆) δ 12.08 (s, 1H), 8.77 (s, 1H), 8.16 (d, J = 9.0 Hz, 1H), 7.87 (d, J = 8.9 Hz, 1H), 7.77 - 7.62 (m, 2H), 7.42 - 6.91 (m, 4H), 6.77 - 6.57 (m, 1H), 6.17 - 5.94 (m, 1H), 4.66 - 4.51 (m, 1H), 4.51 - 4.37 (m, 1H), 4.15 - 4.01 (m, 3H), 3.87 - 3.83 (m, 4H), 3.69 - 3.66 (m, 4H).

### Example II-1: Preparation of Compound 11-1

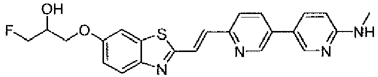

### Synthetic route for Compound II-1:

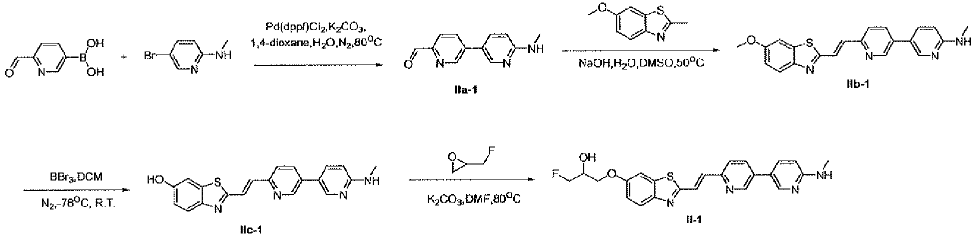

### Step 1: Preparation of Intermediate IIa-1

(6-Formylpyridin-3-yl)boronic acid (48 mg, 0.32 mmol), 5-bromo-2-(methylamino)pyridine (60 mg, 0.32 mmol), 1,1'-bis(diphenylphosphino)ferrocene]dichloropalladium(II) (Pd(dppf)Cl₂) (23 mg, 0.032 mmol), and potassium carbonate (138 mg, 0.96 mmol) were dissolved in 1,4-dioxane (2.4 mL) and water (0.6 mL). The mixture was reacted at 80 °C overnight under N₂ protection. After the reaction was complete, the mixture was purified by flash silica gel column chromatography (petroleum ether (PE)/ethyl acetate (EA)) to give the product **IIa-1** (42 mg, 0.2 mmol) as a yellow solid in 61% yield. MS-ESI: m/z 214 [M + H]⁺.

### Step 2: Preparation of Intermediate IIb-1

**IIa-1** (42 mg, 0.2 mmol) and 6-methoxy-2-methylbenzothiazole (36 mg, 0.2 mmol) were dissolved in dimethyl sulfoxide (DMSO) (2 mL). An aqueous solution of NaOH (1 g/mL) (32 µL, 0.8 mmol) was added. The mixture was reacted at 50 °C for 2 h. After the reaction was complete, the mixture was purified by flash silica gel column chromatography (PE/EA) and flash C18 column chromatography (acetonitrile/water) to give the product **IIb-1** (4 mg, 0.01 mmol) as an orange solid in 5% yield. MS-ESI: m/z 375 [M + H]⁺.

### Step 3: Preparation of Intermediate IIc-1

**IIb-1** (4 mg, 0.01 mmol) was dissolved in dichloromethane (DCM) (0.03 mL). Under N₂ protection, a solution of boron tribromide in dichloromethane (1 M) (0.03 mL, 0.03 mmol) was added dropwise at -78 °C. The mixture was stirred for 2 h, then allowed to warm to room temperature and stirred overnight. The reaction solution was quenched by dropwise addition into saturated aqueous sodium bicarbonate solution (50 mL) and extracted with ethyl acetate (EA) (50 mL). The EA phase was washed with water (50 mL × 2), washed with saturated aqueous sodium chloride solution (50 mL), and dried over anhydrous sodium sulfate to give the product **IIc-1** (3.6 mg, 0.01 mmol) as an orange solid in 93% yield. MS-ESI: m/z 361 [M + H]⁺. ¹H NMR (400 MHz, DMSO-*d*₆) δ 9.86 (s, 1H), 8.57 (d, *J* = 2.4 Hz, 1H), 8.37 (d, *J* = 4.8, 1.9 Hz, 1H), 8.09 (dd, *J* = 9.0, 2.4 Hz, 1H), 7.82 - 7.72 (m, 2H), 7.47 (s, 2H), 7.41 - 7.34 (m, 2H), 7.25 (d, *J* = 8.8 Hz, 1H), 7.08 (dd, *J* = 7.2, 4.9 Hz, 1H), 6.96 (dd, *J* = 8.8, 2.5 Hz, 1H), 3.57 (s, 3H).

### Step 4: Preparation of Product II-1

**IIc-1** (3.6 mg, 0.01 mmol) and potassium carbonate (4 mg, 0.03 mmol) were added to N,N-dimethylformamide (DMF) (0.1 mL). Epifluorohydrin (1.5 µL, 0.02 mmol) was added dropwise, and the mixture was reacted at 80 °C for 4 h. After the reaction was complete, the mixture was purified by HPLC (acetonitrile/water) to give the product **II-1** (0.9 mg, 0.002 mmol) as an orange solid in 21 % yield. MS-ESI: m/z 437 [M + H]⁺. ¹H NMR (400 MHz, DMSO-*d*₆) δ 8.98 (d, *J* = 2.4 Hz, 1H), 8.42 (s, 1H), 8.23 - 8.12 (m, 2H), 7.92 (s, 1H), 7.89 (d, *J* = 8.5 Hz, 1H), 7.84 (d, *J* = 8.2 Hz, 1H), 7.74 (d, *J =* 2.5 Hz, 1H), 7.65 (d, *J* = 15.8 Hz, 1H), 7.16 (dd, *J* = 8.9, 2.6 Hz, 1H), 7.00 - 6.86 (m, 1H), 4.63 - 4.52 (m, 1H), 4.52 - 4.41 (m, 1H), 4.17 - 4.02 (m, 3H), 2.94 (s, 3H).

### Resolution and Characterization of Isomers of Compound II-1

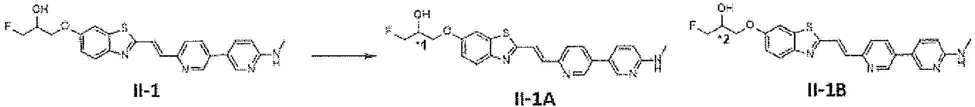

Compound II-1 (100 mg, 0.23 mmol) was dissolved in MeOH:DCM = 1:1 (80 mL) and purified by chiral SFC (Column: ChiralPak IH, 250 × 40 mm I.D., 10 µm; Mobile phase: A: CO₂, B: MeOH (0.1% NH₃·H₂O); Flow rate: 140 mL/min) to give yellow solid **II-1A** (shorter retention time, 40 mg, MS-ESI: m/z 437.1 [M + H]⁺) and yellow solid **II-1B** (longer retention time, 40 mg, MS-ESI: m/z 437.0 [M + H]⁺).

### Single configuration compound I-1A (shorter retention time):

SFC analysis: Retention time: 4.578 min, ee > 99.9% (Column: Chiral Pak IH, 100 × 3 mm I.D., 3 µm; Mobile phase: A: CO₂, B: MeOH (0.1% DEA); Flow rate: 2.0 mL/min).

¹HNMR (400 MHz, DMSO-d6) δ 8.93 (d, *J* = 2.3 Hz, 1H), 8.48 (d, *J =* 2.5 Hz, 1H), 8.06 (dd, *J* = 8.2, 2.4 Hz, 1H), 7.90 (d, *J* = 8.9 Hz, 1H), 7.89 - 7.80 (m, 2H), 7.76 (d, *J* = 8.2 Hz, 1H), 7.73 (d, *J* = 2.6 Hz, 1H), 7.61 (d, *J =* 15.9 Hz, 1H), 6.57 (d, *J* = 8.8 Hz, 1H), 5.53 (d, *J=* 5.1 Hz, 1H), 4.58 (qd, *J* = 9.6, 4.2 Hz, 1H), 4.52 - 4.40 (m, 1H), 4.18 - 3.96 (m, 3H), 2.83 (d, *J* = 4.8 Hz, 3H).

### Single configuration compound I-1B (longer retention time):

SFC analysis: Retention time: 5.744 min, ee: 99.59% (Column: Chiral Pak IH, 100 × 3 mm I.D., 3 µm; Mobile phase: A: CO₂, B: MeOH (0.1% DEA); Flow rate: 2.0 mL/min).

¹HNMR (400 MHz, DMSO-*d6*) δ 8.93 (d, *J* = 2.3 Hz, 1H), 8.48 (d, *J =* 2.5 Hz, 1H), 8.06 (dd, *J* = 8.2, 2.4 Hz, 1H), 7.90 (d, *J* = 8.9 Hz, 1H), 7.89 - 7.80 (m, 2H), 7.76 (d, *J* = 8.2 Hz, 1H), 7.73 (d, *J* = 2.6 Hz, 1H), 7.61 (d, *J =* 15.9 Hz, 1H), 6.57 (d, *J* = 8.8 Hz, 1H), 5.53 (d, *J* = 5.1 Hz, 1H), 4.58 (qd, *J =* 9.6, 4.2 Hz, 1H), 4.52 - 4.40 (m, 1H), 4.18 - 3.96 (m, 3H), 2.83 (d, *J* = 4.8 Hz, 3H).

### Example II-2: Preparation of Compound II-2

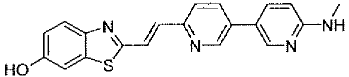

Example **II-2** is Intermediate **IIc-1**, the synthesis of which is described in Example **II-1**.

### Example II-3: Preparation of Compound II-3

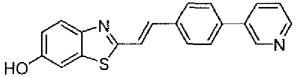

The synthetic method of Example 3 was the same as that of Intermediate **IIc-1**, except that (6-formylpyridin-3-yl)boronic acid was replaced with 4-boronobenzaldehyde, and 5-bromo-2-(methylamino)pyridine was replaced with 3-bromopyridine. The product **II-3** was obtained as a yellow solid in 26% yield. LC-MS (ESI) [M + H]⁺: 331.0. ¹H NMR (400 MHz, DMSO-*d*₆) δ 9.91 (s, 1H), 9.09 (d, *J* = 2.4 Hz, 1H), 8.73 - 8.66 (m, 1H), 8.41 (d, *J* = 8.0 Hz, 1H), 7.98 - 7.83 (m, 4H), 7.79 (d, *J* = 8.8 Hz, 1H), 7.72 (dd, *J* = 8.0, 5.0 Hz, 1H), 7.65 (d, *J =* 16.2 Hz, 1H), 7.57 (d, *J =* 16.3 Hz, 1H), 7.38 (d, *J* = 2.5 Hz, 1H), 6.98 (dd, *J =* 8.8, 2.5 Hz, 1H).

### Example II-4: Preparation of Compound II-4

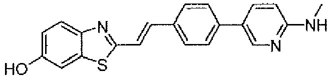

The synthetic method of Example II-4 was the same as that of Intermediate **IIc-1**, except that (6-formylpyridin-3-yl)boronic acid was replaced with 4-boronobenzaldehyde. The product **II-4** was obtained as a yellow solid in 31% yield. LC-MS (ESI) [M + H]⁺: 360.1. ¹H NMR (400 MHz, DMSO-*d*₆) δ 9.90 (s, 1H), 8.56 - 8.11 (m, 3H), 7.84 (s, 2H), 7.80 - 7.69 (m, 3H), 7.57 (dd, *J =* 16.1, 4.2 Hz, 2H), 7.37 (s, 1H), 7.08 - 6.88 (m, 2H), 2.96 (d, *J* = 4.1 Hz, 3H).

### Example II-5: Preparation of Compound II-5

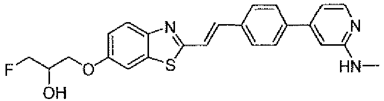

The synthetic method of Example **II-5** was the same as that of Example II-1, except that (6-formylpyridin-3-yl)boronic acid was replaced with 4-boronobenzaldehyde, and 5-bromo-2-(methylamino)pyridine was replaced with 4-bromo-2-(methylamino)pyridine. The product **II-5** was obtained as a yellow solid in 12% yield. LC-MS (ESI) [M + H]⁺: 436.0. ¹H NMR (400 MHz, DMSO-*d*₆) δ 8.03 (d, *J* = 6.6 Hz, 1H), 7.97 (d, *J =* 8.2 Hz, 2H), 7.94 - 7.86 (m, 3H), 7.77 - 7.71 (m, 2H), 7.65 (d, *J =* 16.2 Hz, 1H), 7.26 (d, *J* = 8.3 Hz, 2H), 7.15 (dd, *J* = 8.9, 2.6 Hz, 1H), 4.64 - 4.53 (m, 1H), 4.51 - 4.39 (m, 1H), 4.16 - 4.02 (m, 3H), 3.00 (s, 3H).

### Example II-6: Preparation of Compound II-6

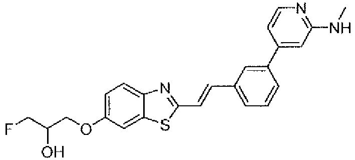

The synthetic method of Example **II-6** was the same as that of Example II-1, except that (6-formylpyridin-3-yl)boronic acid was replaced with 3-boronobenzaldehyde, and 5-bromo-2-(methylamino)pyridine was replaced with 4-bromo-2-(niethylamino)pyridine. The product **II-6** was obtained as a yellow solid in 12% yield. LC-MS (ESI) [M + H]⁺: 436.0. ¹H NMR (400 MHz, DMSO-*d*₆) δ 8.23 (s, 1H), 8.07 (d, *J* = 6.8 Hz, 1H), 7.96 (d, *J =* 7.3 Hz, 1H), 7.88 (d, *J =* 8.9 Hz, 1H), 7.85 - 7.78 (m, 2H), 7.77 - 7.69 (m, 2H), 7.67 - 7.62 (m, 1H), 7.55 - 7.46 (m, 1H), 7.34 - 7.29 (m, 1H), 7.15 (dd, *J* = 8.9, 2.5 Hz, 1H), 7.10 - 7.00 (m, 1H), 4.62 - 4.50 (m, 1H), 4.48 - 4.37 (m, 1H), 4.14 - 3.92 (m, 3H), 3.02 (s, 3H).

### Example II-7: Preparation of Compound II-7

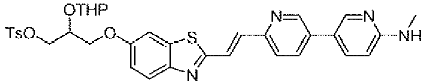

### Synthetic route for Compound II-7:

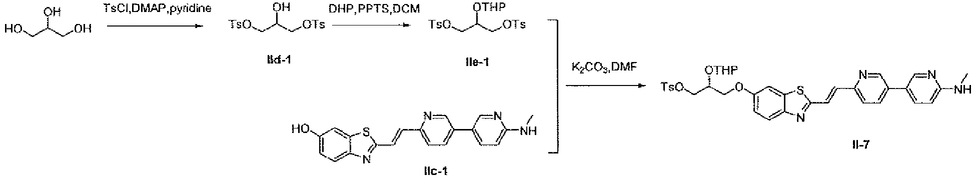

### Step 1: Preparation of Intermediate IId-1

Glycerol (1 g, 11 mmol) and 4-dimethylaminopyridine (DMAP) (335 mg, 2.75 mmol) were dissolved in pyridine (10 mL). p-Toluenesulfonyl chloride (TsCl) (4.4 g, 23 mmol) dissolved in pyridine (15 mL) was added dropwise at 0 °C. The mixture was reacted at room temperature overnight. After the reaction was complete, the reaction solution was diluted in 100 mL of water, concentrated HCl was added dropwise to adjust the pH to acidic, and the mixture was extracted with DCM (100 mL × 5). The organic phase was washed with IN HCl (100 mL), then with water (100 mL), and finally purified by flash silica gel column chromatography (PE/EA) to give the product **IId-1** (2.8 g, 7 mmol) as a colorless oily liquid in 64% yield. MS-ESI: m/z 401 [M + H]⁺.

### Step 2: Preparation of Intermediate IIe-1

**IId-1** (1.2 g, 3 mmol) and 3,4-dihydropyran (DHP) (5.5 mL, 60 mmol) were dissolved in DCM (10 mL). Pyridine p-toluenesulfonate (PPTS) (150 mg, 0.6 mmol) was added, and the mixture was reacted at 40 °C overnight. After the reaction was complete, the reaction solution was concentrated and then purified by flash silica gel column chromatography (PE/EA) to give the product **IIe-1** (1.3 g, 2.7 mmol) as a light yellow oily liquid in 89% yield. MS-ESI: m/z 485 [M + H]⁺.

### Step 3: Preparation of Product II-7

**IIc-1** (21 mg, 0.06 mmol), **IIe-1** (140 mg, 0.29 mmol), and potassium carbonate (16 mg, 0.12 mmol) were added to DMF (0.5 mL) and stirred at 80 °C for 2 h. After the reaction was complete, the mixture was purified by flash silica gel column chromatography (PE/EA) to give the product **II-7** (3.8 mg, 0.006 mmol) as a yellow solid in 10% yield. MS-ESI: m/z 673 [M + H]⁺.

### Example II-8: Preparation of Compound II-8

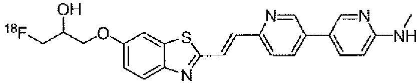

### Method 1:

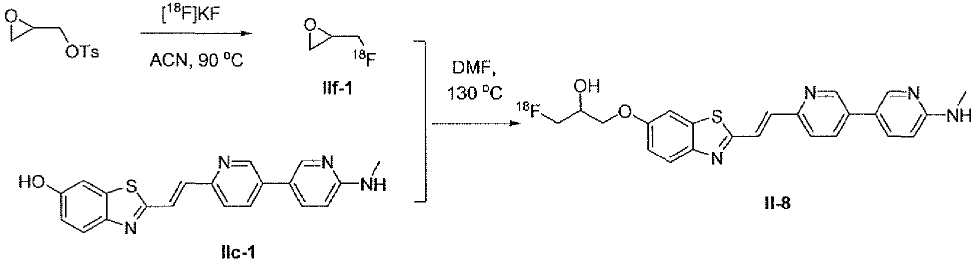

150 mCi of ¹⁸F was trapped on a QMA column. The ¹⁸F was eluted into the reaction vial with 0.6 mL of eluent (150 mg Kryptofix^{®} 222, 15 mg K₂CO₃ dissolved in 9 mL acetonitrile and 1 mL water). The solvent was dried under high-purity nitrogen by heating at 110 °C for 20 min. 0.2 mg of oxiran-2-ylmethyl 4-methylbenzenesulfonate and 0.2 mL of acetonitrile were added to the reaction vial, and the mixture was reacted at 90 °C for 10 min. The acetonitrile was evaporated, and 0.2 mL of DMF and 0.5 mg of **IIc-1** were added, followed by reaction at 130 °C for 10 min. The mixture was separated and purified by HPLC (HPLC conditions: CAPCELL PAK C18 UG80 10mm×250mm, acetonitrile: water = 26:74, flow rate: 3 mL/min, peak time: 25 min. The product was collected and subjected to solid-phase extraction using a C18 column, and eluted with ethanol to obtain the final product **II-8**, with a labeling efficiency of 15%.

### Method 2:

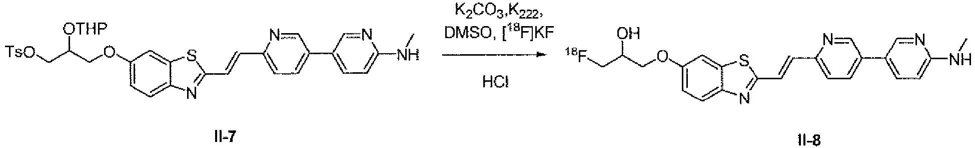

150 mCi of ¹⁸F was trapped on a QMA column. The ¹⁸F was eluted into the reaction vial with 0.6 mL of eluent (150 mg Kryptofix 222, 15 mg K₂CO₃ dissolved in 9 mL acetonitrile and 1 mL water). The solvent was dried under high-purity nitrogen by heating at 110 °C for 20 min. 0.5 mg of **II-7** was added to the reaction vial and stirred at 140 °C for 10 min. 0.2 mL of 2N HCl was then added, and the mixture was reacted at 105 °C for 8 min to remove the THP protecting group. The reaction solution was separated and purified by HPLC (HPLC conditions: CAPCELL PAK C18 UG80 10mm×250mm, acetonitrile: water = 26:74, flow rate: 3 mL/min, peak time: 25 min. The product was collected and subjected to solid-phase extraction using a C18 column, and eluted with ethanol to obtain the final product II**-8,** with a labeling efficiency of 13%.

Following a method analogous to Example 11-1, e.g., using a ChiralPak IH column, a pair of enantiomers of Example II-8 (Enantiomer 1 and Enantiomer 2) can be resolved.

### Example II-9: Preparation of Compound II-9

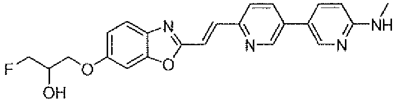

The synthetic method of Example **II-9** was the same as that of Example **II-1**, except that 6-methoxy-2-methylbenzothiazole was replaced with 6-methoxy-2-methylbenzoxazole. The product **II-9** was obtained as a yellow solid in 10% yield. LC-MS (ESI) [M + H]⁺: 421.2. ¹H NMR (400 MHz, DMSO-*d*₆) δ 9.01 (d, *J* = 2.4 Hz, 1H), 8.41 (s, 1H), 8.25 - 8.14 (m, 2H), 7.89 (d, *J* = 8.2 Hz, 1H), 7.80 (d, *J* = 16.0 Hz, 1H), 7.67 (d, *J* = 8.7 Hz, 1H), 7.59 (d, *J =* 15.9 Hz, 1H), 7.40 (d, *J* = 2.4 Hz, 1H), 7.09 - 6.95 (m, 2H), 4.65 - 4.52 (m, 1H), 4.51 - 4.39 (m, 1H), 4.15 - 4.01 (m, 3H), 2.96 (s, 3H).

### Example II-10: Preparation of Compound II-10

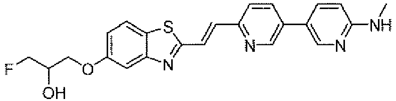

The synthetic method of Example **II-10** was the same as that of Example II-1, except that 6-methoxy-2-methylbenzothiazole was replaced with 7-methoxy-2-methylbenzothiazole. The product **II-10** was obtained as a yellow solid in 19% yield. LC-MS (ESI) [M + H]⁺: 437.2. ¹H NMR (400 MHz, DMSO-*d*₆) δ 9.01 (d, *J* = 2.4 Hz, 1H), 8.40 (s, 1H), 8.26 (d, *J* = 9.2 Hz, 1H), 8.19 (dd, *J =* 8.2, 2.5 Hz, 1H), 8.01 (d, *J* = 8.8 Hz, 1H), 7.93 (d, *J* = 15.9 Hz, 1H), 7.88 (d, *J* = 8.2 Hz, 1H), 7.73 (d, *J* = 15.9 Hz, 1H), 7.58 (d, *J* = 2.5 Hz, 1H), 7.14 (dd, *J =* 8.8, 2.5 Hz, 1H), 7.04 (d, *J* = 9.3 Hz, 1H), 4.64 - 4.53 (m, 1H), 4.52 - 4.40 (m, 1H), 4.16 - 4.04 (m, 3H), 2.97 (s, 3H).

### Example II-11: Preparation of Compound II-11

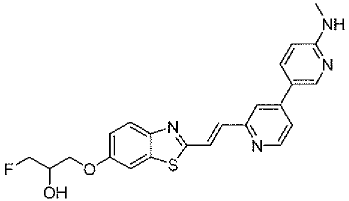

The synthetic method of Example **II-11** was the same as that of Example II-1, except that (6-formylpyridin-3-yl)boronic acid was replaced with (6-formylpyridin-4-yl)boronic acid. The product **II-11** was obtained as a yellow solid in 17% yield. LC-MS (ESI) [M + H]⁺: 437.2. ¹H NMR (400 MHz, DMSO-*d*₆) δ 8.70 (d, *J* = 5.4 Hz, 1H), 8.58 (s, 1H), 8.28 (d, *J* = 9.0 Hz, 1H), 8.24 (s, 1H), 8.04 (d, *J* = 16.0 Hz, 1H), 7.93 (d, *J* = 8.9 Hz, 1H), 7.81 - 7.74 (m, 2H), 7.65 (d, *J* = 16.0 Hz, 1H), 7.18 (dd, *J* = 8.9, 2.5 Hz, 1H), 6.97 (d, *J* = 9.1 Hz, 1H), 4.64 - 4.53 (m, 1H), 4.51 - 4.41 (m, 1H), 4.14 - 4.03 (m, 3H), 2.97 (s, 3H).

### Example II-12: Preparation of Compound II-12

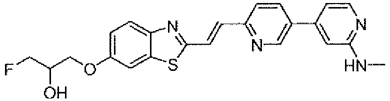

The synthetic method of Example **II-12** was the same as that of Example II-1, except that 5-bromo-2-(methylamino)pyridine was replaced with 4-bromo-2-(methylamino)pyridine. The product **II-12** was obtained as a yellow solid in 13% yield. LC-MS (ESI) [M + H]⁺: 437.1. ¹H NMR (400 MHz, DMSO-*d*₆) δ 9.11 (s, 1H), 8.36 - 8.28 (m, 1H), 8.08 (d, *J* = 6.5 Hz, 1H), 8.03 - 7.89 (m, 3H), 7.75 (d, *J* = 2.6 Hz, 1H), 7.70 (d, *J* = 15.8 Hz, 1H), 7.36 - 7.26 (m, 2H), 7.17 (dd, *J* = 8.9, 2.6 Hz, 1H), 4.64 - 4.51 (m, 1H), 4.51 - 4.41 (m, 1H), 4.16 - 4.03 (m, 3H), 3.00 (s, 3H).

### Example II-13: Preparation of Compound II-13

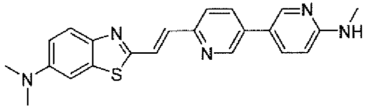

The synthetic method of Example **II-13** was the same as that of Intermediate **IIb-1,** except that 6-methoxy-2-methylbenzothiazole was replaced with 6-(dimethylamino)-2-methylbenzothiazole. The product **II-13** was obtained as a yellow solid in 12% yield. LC-MS (ESI) [M + H]⁺: 388.2.

### Example II-14: Preparation of Compound II-14

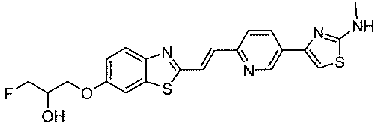

The synthetic method of Example **II-14** was the same as that of Example II-1, except that 5-bromo-2-(methylamino)pyridine was replaced with 4-bromo-2-(methylamino)thiazole. The product **II-14** was obtained as a yellow solid in 18% yield. LC-MS (ESI) [M + H]⁺: 443.1. ¹H NMR (400 MHz, DMSO-*d*₆) δ 9.12 (d, *J* = 2.2 Hz, 1H), 8.25 (dd, *J* = 8.1, 2.3 Hz, 1H), 7.96 - 7.69 (m, 5H), 7.61 (d, *J* = 15.9 Hz, 1H), 7.37 (s, 1H), 7.16 (dd, *J* = 8.9, 2.6 Hz, 1H), 4.64 - 4.52 (m, 1H), 4.51 - 4.40 (m, 1H), 4.12 - 4.03 (m, 3H), 2.91 (s, 3H).

### Example II-15: Preparation of Compound II-15

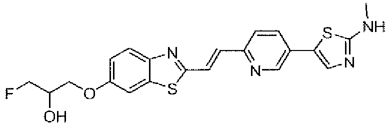

The synthetic method of Example **II-15** was the same as that of Example II-1, except that 5-bromo-2-(methylamino)pyridine was replaced with 5-bromo-2-(methylamino)thiazole. The product II-15 was obtained as a yellow solid in 22% yield. LC-MS (ESI) [M + H]⁺: 443.0. ¹H NMR (400 MHz, DMSO-*d*₆) δ 9.80 (s, 2H), 8.87 (d, *J* = 2.4 Hz, 1H), 8.17 (s, 1H), 8.04 (dd, *J =* 8.1, 2.5 Hz, 1H), 7.94 - 7.81 (m, 3H), 7.74 (d, *J* = 2.6 Hz, 1H), 7.64 (d, *J* = 15.8 Hz, 1H), 7.16 (dd, *J* = 8.9, 2.6 Hz, 1H), 4.65 - 4.53 (m, 1H), 4.51 - 4.39 (m, 1H), 4.15 - 4.01 (m, 3H), 3.65 (s, 3H).

### Example II-16: Preparation of Compound 11-16

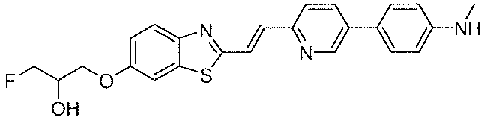

The synthetic method of Example **II-16** was the same as that of Example II-1, except that 5-bromo-2-(methylamino)pyridine was replaced with 4-bromo-N-methylaniline. The product **II-16** was obtained as a yellow solid in 7% yield. LC-MS (ESI) [M + H]⁺: 436.1. ¹H NMR (400 MHz, DMSO-*d*₆) δ 8.92 (d, *J* = 2.6 Hz, 1H), 8.40 (s, 1H), 8.25 - 8.10 (m, 2H), 7.90 (s, 1H), 7.82 (d, *J =* 8.5 Hz, 1H), 7.80 (d, *J =* 8.2 Hz, 1H), 7.73 (d, *J =* 2.3 Hz, 2H), 7.65 (d, *J =* 15.6 Hz, 2H), 7.16 (dd, *J =* 8.9, 2.6 Hz, 1H), 7.00 - 6.86 (m, 1H), 4.63 - 4.48 (m, 2H), 4.15 - 4.03 (m, 3H), 2.96 (s, 3H).

### Example II-17: Preparation of Compound II-17

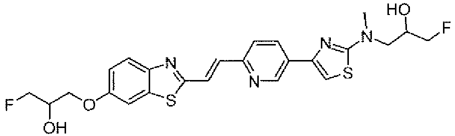

The synthetic method of Example **II-17** was the same as that of Example II-14, except that the amount of epifluorohydrin was increased threefold. The product II**-17** was obtained as a yellow solid in 4% yield. LC-MS (ESI) [M + H]⁺: 519.1. ¹H NMR (400 MHz, DMSO-*d*₆) δ 9.16 (s, 1H), 8.26 (s, 1H), 7.97 - 7.70 (m, 4H), 7.66 - 7.58 (m, 1H), 7.47 (d, *J* = 4.9 Hz, 1H), 7.17 (s, 1H), 4.62 - 4.30 (m, 4H), 4.20 - 4.01 (m, 6H), 3.20 - 3.13 (m, 3H).

### Example II-18: Preparation of Compound II-18

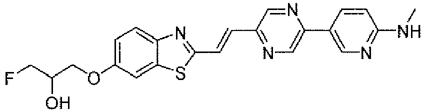

The synthetic method of Example **II-18** was the same as that of Example II-1, except that (6-formylpyridin-3-yl)boronic acid was replaced with (6-formylpyrazin-3-yl)boronic acid. The product **II-18** was obtained as a yellow solid in 7% yield. LC-MS (ESI) [M + H]⁺: 438.2. ¹H NMR (400 MHz, DMSO-*d*₆) δ 9.30 - 9.26 (m, 1H), 8.97 - 8.90 (m, 1H), 8.80 - 8.74 (m, 1H), 8.41 (d, *J* = 9.2 Hz, 1H), 7.99 - 7.88 (m, 2H), 7.80 - 7.65 (m, 2H), 7.17 (dd, *J* = 8.9, 2.6 Hz, 1H), 6.91 (d, *J* = 9.2 Hz, 1H), 4.64 - 4.54 (m, 1H), 4.51 - 4.40 (m, 1H), 4.15 - 4.03 (m, 3H), 2.95 (s, 3H).

### Example II-19: Preparation of Compound II-19

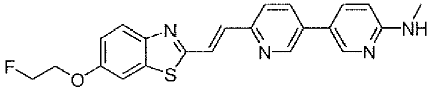

The synthetic method of Example **II-19** was the same as that of Example II-1, except that epifluorohydrin was replaced with 1-bromo-2-fluoroethane. The product **II-19** was obtained as a yellow solid in 27% yield. LC-MS (ESI) [M + H]⁺: 407.1. ¹H NMR (400 MHz, DMSO-*d*₆) δ 8.99 (s, 1H), 8.41 (s, 1H), 8.23 - 8.11 (m, 2H), 7.96 - 7.81 (m, 3H), 7.75 (s, 1H), 7.65 (d, *J* = 15.8 Hz, 1H), 7.18 (d, *J* = 9.1 Hz, 1H), 6.97 (d, *J* = 9.0 Hz, 1H), 4.91 - 4.83 (m, 1H), 4.80 - 4.72 (m, 1H), 4.44 - 4.35 (m, 1H), 4.34 - 4.29 (m, 1H), 2.95 (s, 3H).

### Example II-20: Preparation of Compound II-20

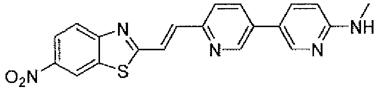

The synthetic method of Example **II-20** was the same as that of Intermediate **IIb-1,** except that 6-methoxy-2-methylbenzothiazole was replaced with 6-nitro-2-methylbenzothiazole. The product **II-20** was obtained as a yellow solid in 12% yield. LC-MS (ESI) [M + H]⁺: 390.1.

### Example II-21: Preparation of Compound II-21

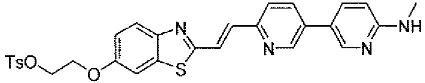

The synthetic method of Example **II-21** was the same as that of Example II-1, except that epifluorohydrin was replaced with 1-bromo-2-oxo-p-toluenesulfonylethane. The product **II-21** was obtained as a yellow solid in 21% yield. LC-MS (ESI) [M + H]⁺: 559.1.

### Example II-22: Preparation of Compound II-22

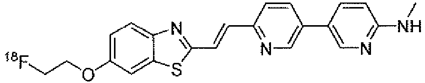

150 mCi of ¹⁸F was trapped on a QMA column. The ¹⁸F was eluted into the reaction vial with 0.6 mL of eluent (150 mg Kryptofix 222, 15 mg K₂CO₃ dissolved in 9 mL acetonitrile and 1 mL water). The solvent was dried under high-purity nitrogen by heating at 110 °C for 20 min. 0.5 mg of the precursor compound **II-21** was added to the reaction vial and stirred at 140 °C for 10 min. The reaction solution was separated and purified by HPLC. The product was collected and subjected to solid-phase extraction using a C18 column, and eluted with ethanol to obtain the final product **II-22,** with a labeling efficiency of 21%.

### Example II-23: Preparation of Compound II-23

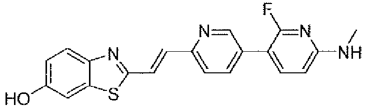

The synthetic method of Example **II-23** was the same as that of Example II-2, except that 5-bromo-2-(methylamino)pyridine was replaced with 5-bromo-6-fluoro-2-(methylamino)pyridine. The product **II-23** was obtained as a brown solid in 9% yield. LC-MS (ESI) [M + H]⁺: 379.1.

### Example II-24: Preparation of Compound II-24

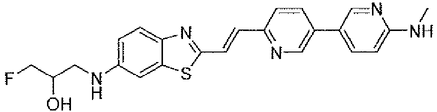

The synthetic method of Example **II-24** was the same as that of Example II-1, except that 6-methoxy-2-methylbenzothiazole was replaced with 6-amino-2-methylbenzothiazole. The product **II-24** was obtained as a yellow solid in 3% yield. LC-MS (ESI) [M + H]⁺: 437.2.

### Example II-25: Preparation of Compound II-25

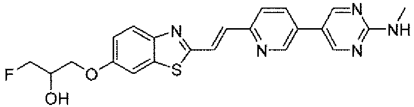

### Synthetic route for Compound II-25:

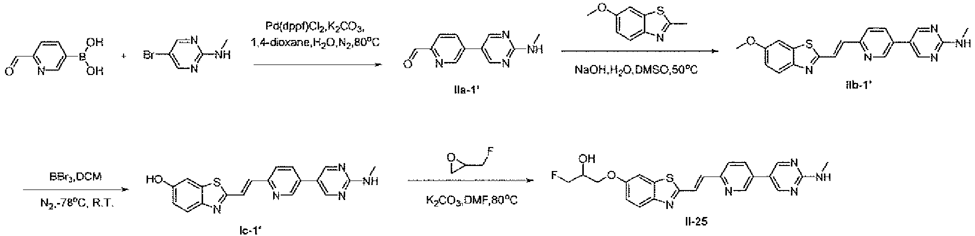

### Step 1: Preparation of Intermediate IIa-1'

(6-Formylpyridin-3-yl)boronic acid (48 mg, 0.32 mmol), 5-bromo-2-(methylamino)pyrimidine (60 mg, 0.32 mmol), 1,1'-bis(diphenylphosphino)ferrocene]dichloropalladium(II) (Pd(dppf)Cl₂) (23 mg, 0.032 mmol), and potassium carbonate (138 mg, 0.96 mmol) were dissolved in 1,4-dioxane (2.4 mL) and water (0.6 mL). The mixture was stirred at 80 °C overnight under N₂ protection. After the reaction was complete, the mixture was purified by flash silica gel column chromatography (petroleum ether (PE)/ethyl acetate (EA)) to give the product **IIa-1'** (42 mg, 0.2 mmol) as a yellow solid in 73% yield. MS-ESI: m/z 215.0 [M + H]⁺.

### Step 2: Preparation of Intermediate IIb-1'

**IIa-1'** (42 mg, 0.2 mmol) and 6-methoxy-2-methylbenzothiazole (36 mg, 0.2 mmol) were dissolved in dimethyl sulfoxide (DMSO) (2 mL). An aqueous solution of NaOH (1 g/mL) (32 µL, 0.8 mmol) was added. The mixture was reacted at 50 °C for 2 h. After the reaction was complete, the mixture was purified by flash silica gel column chromatography (PE/EA) and flash C18 column chromatography (acetonitrile/water) to give the product **IIb-1'** (4 mg, 0.01 mmol) as an orange solid in 11% yield. MS-ESI: m/z 376.1 [M + H]⁺.

### Step 3: Preparation of Intermediate IIc-1'

**IIb-1'** (4 mg, 0.01 mmol) was dissolved in dichloromethane (DCM) (0.03 mL). Under N₂ protection, a solution of boron tribromide in dichloromethane (1 M) (0.03 mL, 0.03 mmol) was added dropwise at -78 °C. The mixture was stirred for 2 h, then allowed to warm to room temperature and reacted overnight. The reaction solution was quenched by dropwise addition into saturated aqueous sodium bicarbonate solution (50 mL) and extracted with EA (50 mL). The EA phase was washed with water (50 mL × 2), then with saturated aqueous sodium chloride solution (50 mL), and dried over anhydrous sodium sulfate to give the product **IIc-1'** (3.6 mg, 0.01 mmol) as an orange solid in 90% yield. MS-ESI: m/z 362.1 [M + H]⁺.

### Step 4: Preparation of Product II-25

**IIc-1'** (3.6 mg, 0.01 mmol) and potassium carbonate (4 mg, 0.03 mmol) were added to N,N-dimethylformamide (DMF) (0.1 mL). Epifluorohydrin (1.5 µL, 0.02 mmol) was added dropwise, and the mixture was reacted at 80 °C for 4 h. After the reaction was complete, the mixture was purified by HPLC (acetonitrile/water) to give the product I-1 as a yellow solid in 7% yield. LC-MS (ESI) [M + H]⁺: 438.1. ¹H NMR (400 MHz, DMSO-*d*₆) δ 8.98 (d, *J =* 2.4 Hz, 1H), 8.79 (s, 2H), 8.17 (dd, *J* = 8.2, 2.4 Hz, 1H), 7.94 - 7.86 (m, 2H), 7.84 (d, *J =* 8.3 Hz, 1H), 7.74 (d, *J* = 2.6 Hz, 1H), 7.63 (d, *J* = 15.9 Hz, 1H), 7.49 (s, 1H), 7.16 (dd, *J* = 8.9, 2.5 Hz, 1H), 4.64 - 4.53 (m, 1H), 4.51 - 4.41 (m, 1H), 4.15 - 4.04 (m, 3H), 2.87 (s, 3H).

### Example II-26: Preparation of Compound II-26

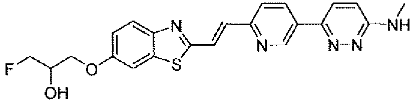

The synthetic method of Example **II-26** was the same as that of Example II-25, except that 5-bromo-2-(methylamino)pyrimidine was replaced with 6-bromo-3-(methylamino)pyridazine. The product II**-26** was obtained as a yellow solid in 5% yield. LC-MS (ESI) [M + H]⁺: 438.1. ¹H NMR (400 MHz, DMSO-*d*₆) δ 9.26 (d, *J* = 2.3 Hz, 1H), 8.42 (dd, *J* = 8.3, 2.4 Hz, 1H), 8.25 (d, *J* = 9.6 Hz, 1H), 8.00 - 7.87 (m, 3H), 7.75 (d, *J* = 2.5 Hz, 1H), 7.68 (d, *J* = 15.8 Hz, 1H), 7.36 (d, *J* = 9.5 Hz, 1H), 7.17 (dd, *J* = 8.9, 2.6 Hz, 1H), 4.63 - 4.53 (m, 1H), 4.51 - 4.42 (m, 1H), 4.18 - 4.02 (m, 3H), 3.05 - 2.98 (m, 3H).

### Example II-27: Preparation of Compound II-27

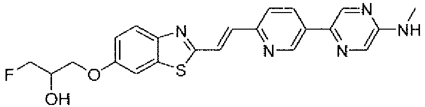

The synthetic method of Example II-27 was the same as that of Example II**-25,** except that 5-bromo-2-(methylamino)pyrimidine was replaced with 5-bromo-2-(methylamino)pyrazine. The product **II-27** was obtained as a yellow solid in 7% yield. LC-MS (ESI) [M + H]⁺: 438.1. ¹H NMR (400 MHz, DMSO-*d*₆) δ 9.22 (d, *J* = 2.3 Hz, 1H), 8.79 - 8.70 (m, 1H), 8.37 (dd, *J* = 8.3, 2.3 Hz, 1H), 8.07 - 8.04 (m, 1H), 7.93 - 7.79 (m, 3H), 7.74 (d, *J* = 2.6 Hz, 1H), 7.63 (d, *J* = 15.9 Hz, 1H), 7.43 (s, 1H), 7.16 (dd, *J* = 8.9, 2.6 Hz, 1H), 4.65 - 4.53 (m, 1H), 4.52 - 4.41 (m, 1H), 4.16 - 4.02 (m, 3H), 2.88 (s, 3H).

### Example II-28: Preparation of Compound II-28

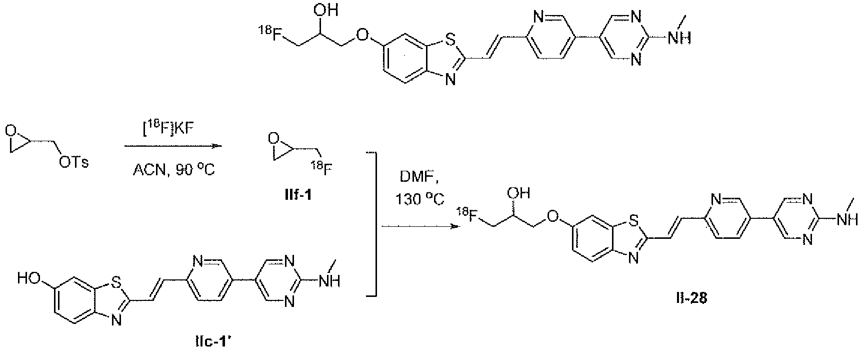

200 mCi of ¹⁸F was trapped on a QMA column. The ¹⁸F was eluted into the reaction vial with 0.7 mL of eluent (150 mg Kryptofix^{®} 222, 15 mg K₂CO₃, 9 mL acetonitrile, 1 mL water). High-purity nitrogen was introduced, and the solvent was evaporated by heating at 110 °C for 20 min. 0.2 mL of acetonitrile and 0.2 mg of oxiran-2-ylmethyl 4-methylbenzenesulfonate were added to the reaction vial, and the mixture was reacted at 90 °C for 10 min. The acetonitrile was evaporated using a rotary evaporator, and 0.2 mL of DMF solvent and 0.5 mg of **IIc-1'** were added, followed by reaction at 130 °C for 10 min. The mixture was separated and purified by HPLC (HPLC conditions: CAPCELL PAK C18 UG80 10mm×250mm, acetonitrile: water = 30:70, flow rate: 3 mL/min, peak time: 29 min. The product was collected and subjected to solid-phase extraction using a C18 column, and eluted with ethanol to obtain the final product **II-28,** with a labeling efficiency of 6%.

### Example II-29: Preparation of Compound II-29

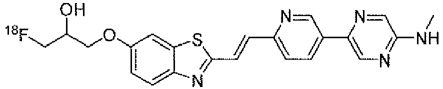

The labeling method was the same as that shown in Example II-28, except that the precursor **IIc-1'** was replaced with

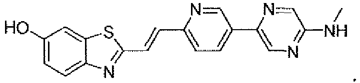

### Example III-1: Preparation of Compound III-1

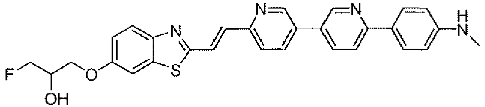

### Synthetic route for Compound III-1:

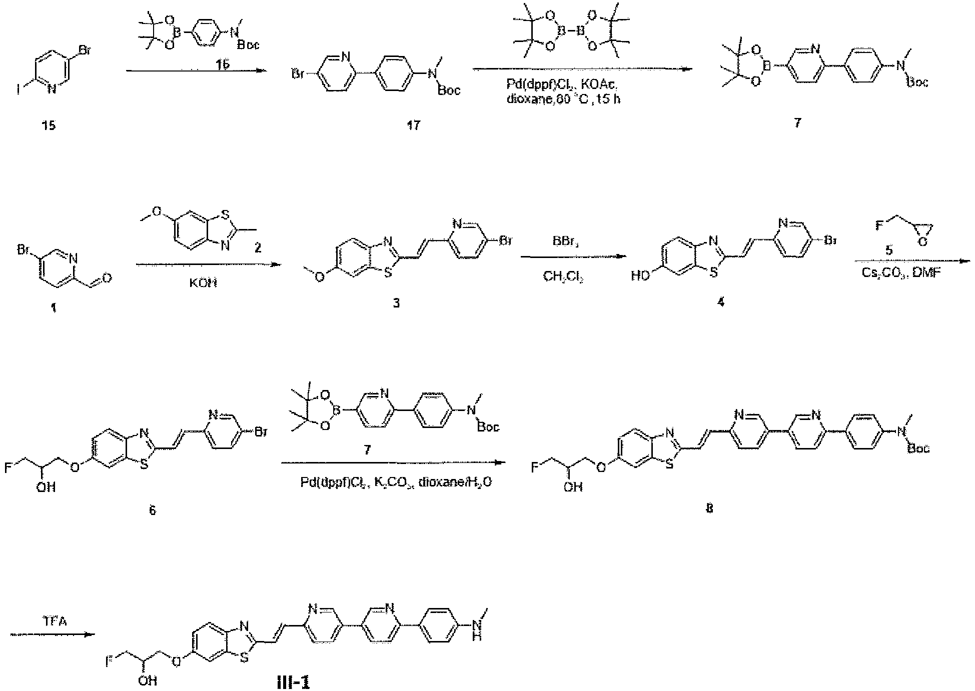

### Step 1: Preparation of Intermediate 17

To a mixture of Compound 15 (1.280 g, 4.51 mmol), Compound 16 (1.5 g, 4.51 mmol), cesium carbonate (4.41 g, 13.53 mmol), palladium acetate (101 mg, 0.45 mmol), and 1,1'-bis(diphenylphosphino)ferrocene (DPPF) (249 mg, 0.45 mmol) were added 1,4-dioxane (15 mL), ethanol (5 mL), and water (5 mL). The reaction mixture was reacted at 120 °C for 3 h under a N₂ atmosphere. The reaction mixture was then filtered and concentrated directly. The residue was purified by column chromatography (PE:EA = 3:1) to give Compound 17 (1.2 g, 73.5% yield) as a white solid. LCMS: m/z(ESI) = 363.6 [M + H]⁺.

### Step 2: Preparation of Intermediate 7

To a solution of Compound 17 (1.2 g, 3.31 mmol) in dioxane (25 mL) were added bis(pinacolato)diboron (1.68 g, 6.62 mmol), Pd(dppf)Cl₂ (242 mg, 0.33 mmol), and potassium acetate (973 mg, 9.93 mmol). The reaction mixture was stirred at 80 °C for 15 h. The reaction solution was then filtered and concentrated directly, and the residue was purified by column chromatography (PE:EA = 3:1) to give compound 7 (400 mg, 29.4% yield) as a gray solid.

### Step 3: Preparation of Intermediate 3

Compound 1 (5 g, 26.88 mmol) and compound 2 (4.8 g, 26.88 mmol) were dissolved in dimethyl sulfoxide (DMSO, 100 mL). A 50% aqueous potassium hydroxide solution (100 mL) was added slowly dropwise, and the mixture was stirred at room temperature overnight. After the reaction was complete, a yellow precipitate formed. The solid was collected by filtration and dried under reduced pressure to give compound 3 (3.2 g, 34% yield) as a white solid. LCMS: m/z(ESI) = 346.8/348.8 [M + H]⁺.

### Step 4: Preparation of Intermediate 4

To a solution of compound 3 (3.2 g, 9.25 mmol) in dichloromethane (100 mL) at -78 °C was added a 1M solution of boron tribromide (BBr₃) in dichloromethane (92.5 mL, 92.5 mmol) dropwise. After stirring at -78 °C for 2 h, the mixture was allowed to warm to room temperature and stirred for an additional 15 h. The reaction solution was poured into ice water (500 mL), and saturated NaHCO₃ solution was added slowly dropwise until the pH reached 8. The precipitate was collected by filtration to give compound 4 (2.4 g, 78% yield) as a yellow solid. ¹H NMR (400 MHz, DMSO) δ 8.76 (d, *J* = 2.4 Hz, 1H), 8.12 (dd, *J =* 8.4, 2.4 Hz, 1H), 7.84 (d, *J =* 15.9 Hz, 1H), 7.80 (d, *J =* 8.8 Hz, 1H), 7.70 (t, *J* = 13.1 Hz, 1H), 7.51 (d, *J* = 15.9 Hz, 1H), 7.35 (d, *J =* 2.3 Hz, 1H), 6.97 (dd, *J* = 8.8, 2.4 Hz, 1H).

### Step 5: Preparation of Intermediate 6

To a solution of compound 4 (2.4 g, 7.2 mmol) in N,N-dimethylformamide (DMF, 30 mL) were added cesium carbonate (Cs₂CO₃ 2.98 g, 21.6 mmol) and compound 5 (2.74 g, 36 mmol). The reaction mixture was heated to 80 °C and maintained for 4 h. After cooling to room temperature, the reaction mixture was poured into ice water (100 mL), stirred for 10 min, and filtered. The solid was collected and purified by flash column chromatography (DCM:MeOH = 10:1) to give compound 6 (1.5 g, 51% yield) as a yellow solid. LCMS: m/z(ESI) = 409.0 [M + H]⁺.

### Step 6: Preparation of Intermediate 8

To a mixture of Compound 6 (300 mg, 0.73 mmol), Compound 7 (300 mg, 0.73 mmol), Pd(dppf)Cl₂ (51 mg, 0.07 mmol), and K₂CO₃ (294 mg, 2.16 mmol) were added 1,4-dioxane (10 mL) and water (2 mL). The reaction mixture was stirred at 80 °C for 15 h under a N₂ atmosphere. The reaction solution was filtered, and the filtrate was concentrated. The residue was purified by column chromatography (PE:EA = 1:1) to give compound 8 (360 mg, 81% yield) as a yellow solid. LCMS: m/z(ESI) = 613.2 [M + H]⁺.

### Step 7: Preparation of Compound III-1

Trifluoroacetic acid (5 mL) was added to a solution of Compound 8 (360 mg, 0.59 mmol) in dichloromethane (10 mL). The reaction mixture was stirred at room temperature for 2 h. The mixture was concentrated and purified by preparative HPLC (containing 0.05% trifluoroacetic acid) to give the trifluoroacetate salt of Compound III-1 (60 mg, 16.7% yield) as a yellow solid. LCMS: m/z(ESI) = 513.2 [M + H]⁺. ¹H NMR (400 MHz, DMSO) δ 9.12 (d, *J =* 2.0 Hz, 1H), 9.01 (d, *J =* 2.0 Hz, 1H), 8.37 - 8.3 (m, 2H), 8.04 (d, *J* = 8.8 Hz, 1H), 7.98 - 7.89 (m, 5H), 7.75 (d, *J =* 3.5 Hz, 1H), 7.68 (d, *J* = 16 Hz, 1H), 7.17 (dd, *J* = 8.8, 2.4 Hz, 1H), 6.69 (d, *J =* 7.2 Hz, 2H), 4.60 - 4.58 (m, 1H), 4.57 - 4.50 (m, 1H), 4.12 - 4.08 (m, 3H), 2.77 (s, 3H).

### Example III-2: Preparation of Compound III-2

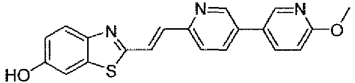

### Synthetic route for Compound III-2:

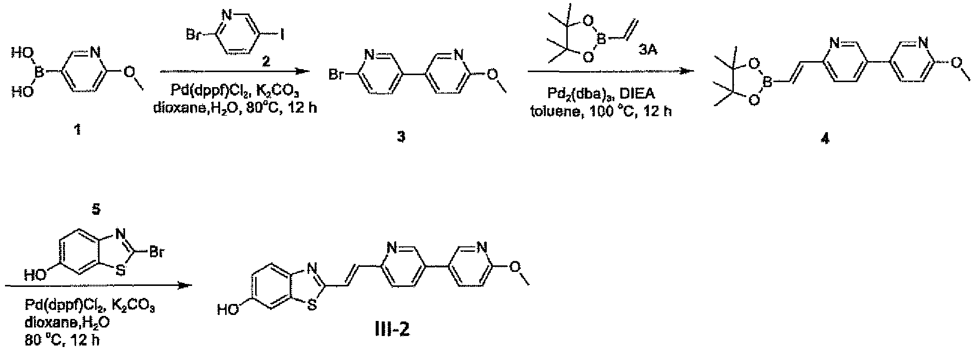

### Step 1: Preparation of Intermediate 3

Compound 1 (10.00 g, 65.33 mmol) and Compound 2 (22.11 g, 78.40 mmol) were dissolved in dioxane (80 mL). Water (20 mL), Pd(dppf)Cl₂ (1.00 g, 1.36 mmol), and K₂CO₃ (27.04 g, 196.00 mmol) were added. The reaction mixture was then stirred at 80 °C for 2 h. The reaction mixture was concentrated, and the residue was purified by flash silica gel column chromatography, eluting with (28% EtOAc in PE), to give compound 3 (5 g, 29.00% yield) as a yellow solid. LCMS: m/z(ESI) = 338.6 [M+H]⁺.

### Step 2: Preparation of Intermediate 4

To a solution of compound 3 (5.00 g, 18.94 mmol) and Compound 3A (14.58 g, 94.70 mmol) in toluene (50 mL) were added diisopropylethylamine (DIEA, 7.34 g, 56.82 mmol), tri-tert-butylphosphine tetrafluoroborate (1.09 g, 3.78 mmol), and Pd₂(dba)₃ (1.73 g, 1.89 mmol). The reaction mixture was then stirred at 100 °C for 12 h. The reaction mixture was concentrated, and the residue was purified by flash silica gel column chromatography, using (30% EA in PE) as eluent, to give compound 4 (0.80 g, 12.46% yield) as a yellow solid. LCMS: m/z(ESI) = 338.6 [M+H]⁺.

### Step 3: Preparation of Compound III-2

To a solution of Compound 4 (0.80 g, 2.36 mmol) and Compound 5 (0.64 g, 2.83 mmol) in dioxane (8 mL) and water (2 mL) were added Pd(dppf)Cl₂ (0.10 g, 0.14 mmol) and K₂CO₃ (0.97 g, 7.09 mmol). The reaction mixture was then stirred at 80 °C for 2 h. After this, the reaction mixture was concentrated to give a residue. The residue was purified by flash silica gel column chromatography, using (26% EtOAc in PE) as eluent, to give a crude product. This crude product was then purified by pre-HPLC (Instrument: Shimadzu LH-40 Liquid Handler, Shimadzu LC-20AP Pumps, Shimadzu SPD-20AP UV Detector; Column: Ultimate XB-C18, 50 × 250 mm × 10 µm; Mobile Phase A: H₂O with FA (10 mmol/L); Mobile Phase B: CH₃CN; Gradient: B increased from 12% to 42% over 30 min, then held at 100% B for 3 min; Flow rate: 20 mL/min; Retention time Rt = 15.0 min; Column temperature: 30°C; Wavelength: 214 nM, 254 nM) to give compound **III-2** (58.1 mg, 6.8% yield) as a yellow solid. LCMS: m/z(ESI) = 362.4 [M+H]⁺. ¹H NMR (400 MHz, DMSO) δ 9.96 (s, 1H), 8.99 (d, *J* = 2.0 Hz, 1H), 8.64 (d, *J* = 2.4 Hz, 1H), 8.19 - 8.14 (m, 2H), 7.87 (d, *J =* 16 Hz, 1H), 7.82 (d, *J* = 8.8 Hz, 2H), 7.59 (d, *J* = 16 Hz, 1H), 7.40 (d, *J* = 2.4 Hz, 1H), 7.00 - 6.96 (m, 2H), 3.92 (s, 3H).

### Example III-3: Preparation of Compound III-3

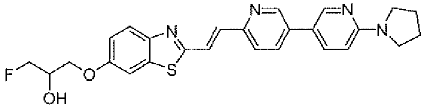

### Synthetic route for Compound III-3

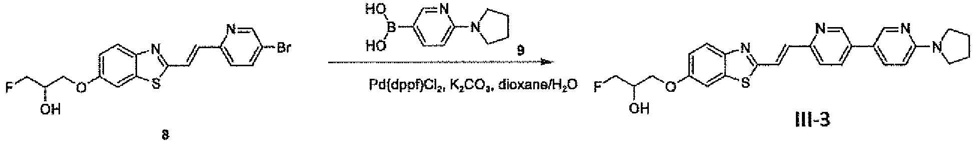

To a mixture of compound 8 (300 mg, 0.73 mmol), Compound 9 (300 mg, 140 mmol), Pd(dppf)Cl₂ (51 mg, 0.07 mmol), and K₂CO₃ (294 mg, 2.16 mmol) were added 1,4-dioxane (10 mL) and water (2 mL). The reaction mixture was heated to 80°C and maintained for 15 h under a N₂ atmosphere. The reaction solution was filtered and concentrated, and the resulting residue was purified by preparative HPLC (containing 0.05% formic acid) to give compound **III-3** (63.0 mg, 18.0% yield) as a gray solid. LCMS: m/z(ESI) = 477.2 [M + H]⁺. ¹H NMR (400 MHz, DMSO) δ 8.96 (d, *J* = 2.4 Hz, 1H), 8.58 (d, *J* = 6.8 Hz, 1H), 8.09 (dd, J = 8.2, 2.4 Hz, 1H), 7.96 (dd, *J* = 8.8, 2.4 Hz, 1H), 7.93 - 7.82 (m, 2H), 7.75 (dd, *J* = 14.4, 5.4 Hz, 2H), 7.62 (d, *J =* 15.8 Hz, 1H), 7.20 - 7.12 (m, 1H), 6.57 (d, *J =* 8.8 Hz, 1H), 5.51 (d, *J =* 9.2 Hz, 1H), 4.58 (d, *J =* 12.8 Hz, 1H), 4.47 (t, *J* = 8.2 Hz, 1H), 4.09 (d, *J =* 17.6 Hz, 3H), 3.46 (d, *J =* 6.6 Hz, 4H), 1.97 (t, *J =* 6.6 Hz, 4H).

### Example III-4: Preparation of Compound III-4

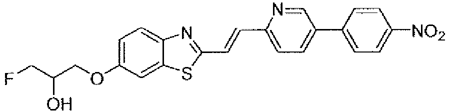

### Synthetic route for Compound III-4:

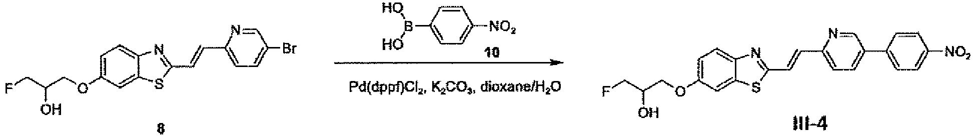

To a mixture of compound 8 (300 mg, 0.73 mmol), Compound 10 (122 mg, 0.73 mmol), Pd(dppf)Cl₂ (51 mg, 0.07 mmol), and K₂CO₃ (294 mg, 2.16 mmol) were added 1,4-dioxane (10 mL) and water (2 mL). The reaction mixture was heated to 80°C under a N₂ atmosphere and stirred for 15 h. The reaction mixture was then filtered and concentrated, and the resulting residue was purified by preparative HPLC (containing 0.05% formic acid) to give compound **III-4** (78.0 mg, 23.5% yield) as a yellow solid. LCMS: m/z(ESI) = 452.2 [M + H]⁺. 1H NMR (400 MHz, DMSO) δ 9.11 (d, *J* = 2.4 Hz, 1H), 8.38 - 8.31 (m, 3H), 8.12 (d, *J =* 8.8 Hz, 2H), 7.95 (t, *J =* 11.2 Hz, 3H), 7.72 (dd, *J* = 22.8, 9.2 Hz, 2H), 7.17 (dd, *J* = 8.8, 2.6 Hz, 1H), 4.61 - 4.43 (m, 3H), 4.13 - 4.05 (m, 3H).

### Example III-5: Preparation of Compound III-5

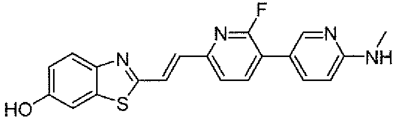

### Synthetic route for Compound III-5:

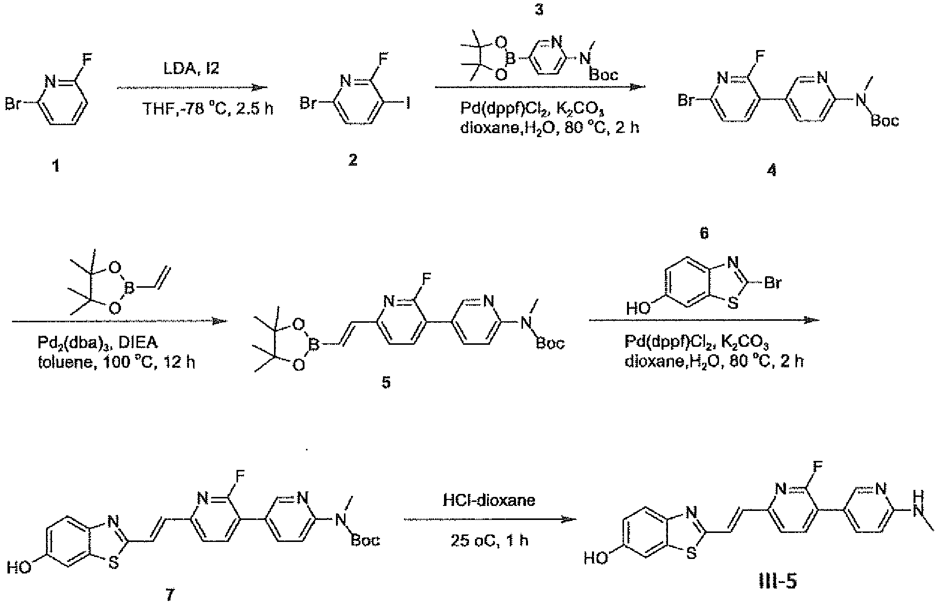

### Step 1: Preparation of Intermediate 2

To a solution of Compound 1 (25.00 g, 142.90 mmol) in tetrahydrofuran (450 mL) at -78°C was added LDA (157 mmol, 157 mmol), and the mixture was stirred at this temperature for 1 h. Then, a solution of iodine (142.90 g, 142.90 mmol) in anhydrous tetrahydrofuran (50 mL) was added, and stirring continued at -78°C for 1.5 h. At this temperature, the reaction mixture was quenched with saturated aqueous sodium thiosulfate solution and extracted with ethyl acetate (3 × 500 mL). The organic layers were combined, washed with saturated brine (100 mL), dried over sodium sulfate, filtered, and concentrated to give a residue. The residue was purified by flash silica gel column chromatography, eluting with (18% EtOAc in PE), to give compound 2 (16 g, 37.2% yield) as a white solid. ¹H NMR (400 MHz, DMSO) δ 8.31 (t, *J =* 8.0 Hz, 1H), 7.43 - 7.41 (d, *J* = 8.0 Hz, 1H).

### Step 2: Preparation of Intermediate 4

To a solution of Compound 2 (2.00 g, 6.64 mmol) and Compound 3 (2.66 g, 7.97 mmol) in dioxane (20 mL) and water (4 mL) at 25°C were added Pd(dppf)Cl₂ (0.20 g, 0.273 mmol) and K₂CO₃ (2.75 g, 19.9 mmol). The reaction mixture was then stirred at 80°C for 2 h. The reaction mixture was concentrated to give a residue. The residue was purified by flash silica gel column chromatography, eluting with (18% EtOAc in PE), to give Compound 4 (1.1 g, 43.47% yield) as a yellow solid. LCMS: m/z(ESI) = 326.0 [M-56+H]⁺.

### Step 3: Preparation of Intermediate 5

To a solution of Compound 4 (1.10 g, 2.88 mmol) and Compound 4A (2.22 g, 14.43 mmol) in toluene (20 mL) at 25°C were added tri-tert-butylphosphine tetrafluoroborate (0.16 g, 0.57 mmol), Pd₂(dba)₃ (0.26 g, 0.28 mmol), and diisopropylethylamine (DIEA, 1.11 g, 8.64 mmol). The reaction mixture was then stirred at 100°C for 12 h. The reaction mixture was concentrated to give a residue. The residue was purified by flash silica gel column chromatography, eluting with (30% EtOAc in PE), to give compound 5 (0.78 g, 59.54% yield) as a yellow solid. LCMS: m/z(ESI) = 400.4 [M-56+H]⁺.

### Step 4: Preparation of Intermediate 7

To a solution of Compound 5 (0.78 g, 1.71 mmol) and Compound 6 (0.47 g, 2.05 mmol) in dioxane (20 mL) and water (4 mL) at 25°C were added Pd(dppf)Cl₂ (0.20 g, 0.27 mmol) and K₂CO₃ (0.70 g, 5.13 mmol). The reaction mixture was then stirred at 80°C for 2 h. The reaction mixture was concentrated to give a residue. The residue was purified by flash silica gel column chromatography, eluting with (22% EA in PE), to give Compound 7 (0.50 g, 61.72% yield) as a yellow solid. LCMS: m/z(ESI) = 478.6 [M+H]⁺.

### Step 5: Preparation of Compound III-5

Compound 7 (0.50 g, 1.04 mmol) was dissolved in HCl-dioxane solution (5 mL, 4 mmol/L) and stirred at 25°C for 1 h. The reaction mixture was concentrated to give a crude product. The crude product was purified by preparative HPLC (Instrument: Shimadzu LH-40 Liquid Handler, Shimadzu LC-20AP Pumps, Shimadzu SPD-20AP UV Detector; Column: Ultimate XB-C18, 50 × 250 mm × 10 µm; Mobile Phase A: H₂O with formic acid (10 mmol/L); Mobile Phase B: Acetonitrile; Gradient: B increased from 18% to 48% over 30 min, then held at 100% B for 3 min; Flow rate: 20 mL/min; Retention time Rt = 12.5 min; Column temperature: 30°C; Detection wavelength: 214 nM, 254 nM) to give compound **III-5** (63.1 mg, 16.1% yield) as a yellow solid. LCMS: m/z(ESI) = 379.4 [M+H]⁺. ¹H NMR (400 MHz, DMSO) δ 9.99 (s, 1H), 8.33 (s, 1H), 8.14 - 8.10 (m, 1H), 7.86 - 7.68 (m, 4H), 7.54 (d, *J =* 16 Hz, 1H), 7.40 (d, *J* = 2.4 Hz, 1H), 7.00 - 6.98 (m, 1H), 6.90 - 6.86 (m, 1H), 6.57 (d, *J* = 8.8 Hz, 1H), 2.83 (d, *J =* 4.8 Hz, 3H).

### Example III-6: Preparation of Compound III-6

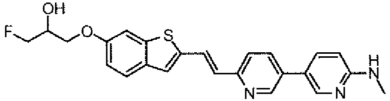

### Synthetic route for Compound III-6:

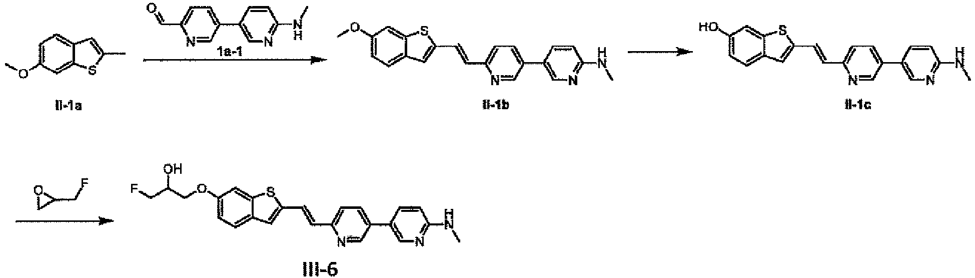

Referring to the synthetic method for II-1 in Example **II-1**, 6-methoxy-2-methylbenzothiazole was replaced with II-1a to give product **III-6** (54 mg) as a yellow solid. LC-MS(ESI): [M+1]⁺ = 536.1.

### Example III-7: Preparation of Compound III-7

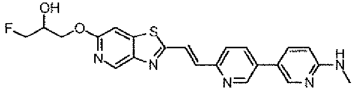

### Synthetic route for Compound III-7:

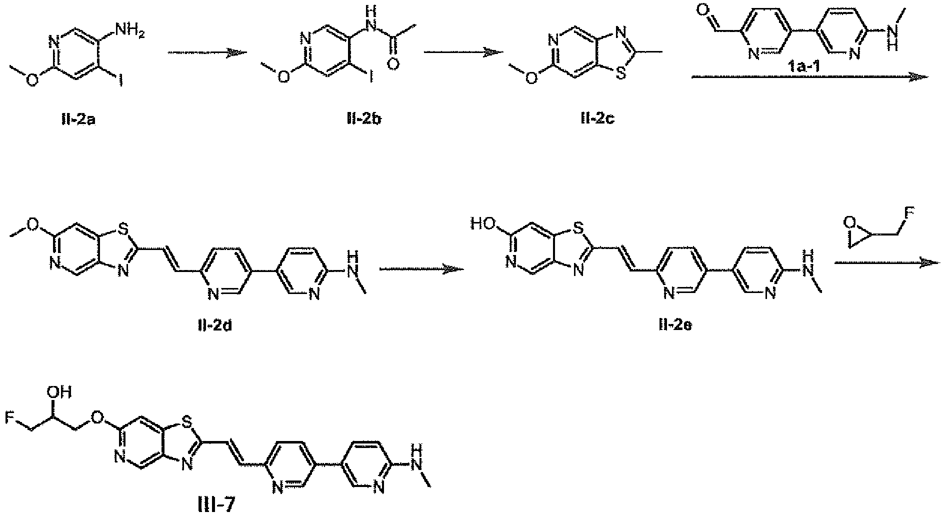

### Step 1: Preparation of Intermediate II-2b

4-Iodo-6-methoxypyridin-3-amine (5.10 g, 20.4 mmol) was dissolved in dichloromethane (50 mL). Triethylamine (4.27 mL, 30.6 mmol) and acetyl chloride (1.74 mL, 24.5 mmol) were added under ice-cooling, and the mixture was stirred at room temperature for 1 hour. The reaction mixture was washed successively with water and saturated brine, and dried over anhydrous sodium sulfate. The solvent was evaporated under reduced pressure, and the precipitated powder was washed with diisopropyl ether and collected by filtration to give Intermediate II-2b as a white solid (4.42 g, yield 74%). LC-MS(ESI): [M+1]⁺ = 293.0.

### Step 2: Preparation of Intermediate II-2c

II-2b (1.32 g, 4.51 mmol), potassium sulfide (1.49 g, 13.5 mmol), and copper(I) iodide (85.8 mg, 0.451 mmol) were dissolved in DMF (25 mL) at room temperature. The reaction solution was warmed to 90°C and stirred for 1 hour. The reaction solution was then quenched with 1N hydrochloric acid, extracted with ethyl acetate, washed with saturated brine, dried over anhydrous sodium sulfate, and filtered. The filtrate was concentrated under reduced pressure, and the resulting residue was purified by silica gel column chromatography (eluent: petroleum ether/ethyl acetate = 2:1) to give II-2c (354 mg) as a yellow solid in 43.5% yield. LC-MS(ESI): [M+1]⁺ = 181.0.

Steps 3, 4, 5: Referring to the synthetic method for II-1 in Example II-1, 6-methoxy-2-methylbenzothiazole was replaced with II-2c to give product III-7 (24 mg) as a yellow solid. LC-MS(ESI): [M+1]⁺ = 438.1.

### Example III-8: Preparation of Compound III-8

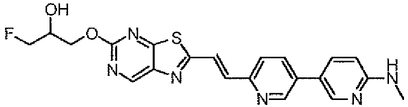

### Synthetic route for Compound III-8:

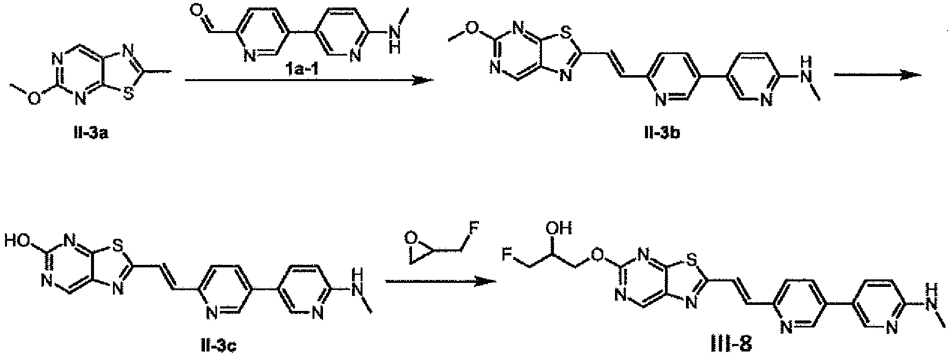

Referring to the synthetic method for II-1 in Example **II-1,** 6-methoxy-2-methylbenzothiazole was replaced with II-3a to give product **III-8** (28 mg) as a yellow solid. LC-MS(ESI): [M+1]⁺ = 439.1. The synthesis of II-3a was carried out according to the method described in Chem. Pharm. Bull. 1958, *6*, 334-338.

### Example III-9: Preparation of Compound III-9

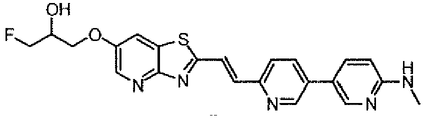

### Synthetic route for Compound III-9:

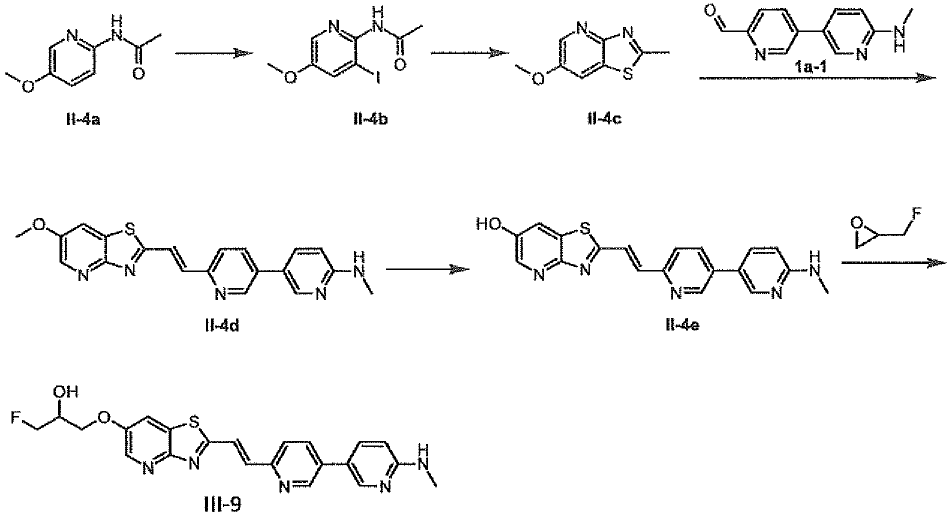

### Step 1: Preparation of Intermediate II-4b

Under nitrogen protection, II-4a (2.49 g, 15 mmol) dissolved in dry tetrahydrofuran (70 mL) was injected into a dry single-neck flask using a syringe. The solution was cooled to -78°C, and then 25 mL of tert-butyllithium (1.5 M) was added dropwise over 15 minutes. Afterwards, the temperature was allowed to rise to 0°C and stirring was maintained for 2 hours. The reaction mixture was cooled again to -78°C, and then iodine (9.52 g, 37.5 mmol) dissolved in 5 mL of dry tetrahydrofuran was added. The reaction mixture was then poured into aqueous ammonium chloride solution and extracted with ethyl acetate. The organic phase was washed with aqueous sodium thiosulfate solution, dried over magnesium sulfate, and concentrated under reduced pressure. The crude product was purified by silica gel chromatography (eluent: dichloromethane/methanol 100/0 to 95/5) to give Intermediate II-4b (1.5 g, yield 34%). LC-MS(ESI): [M+1]⁺ = 292.9.

Steps 2, 3, 4, 5: Referring to the synthetic method for III-7 in Example III-7, II-2b was replaced with II-4b to give product **III-9** (56 mg) as a yellow solid. LC-MS(ESI): [M+1]⁺ = 438.1.

### Example III-10: Preparation of Compound III-10

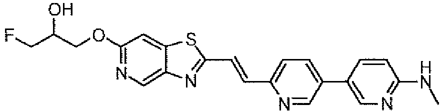

### Synthetic route for Compound III-10:

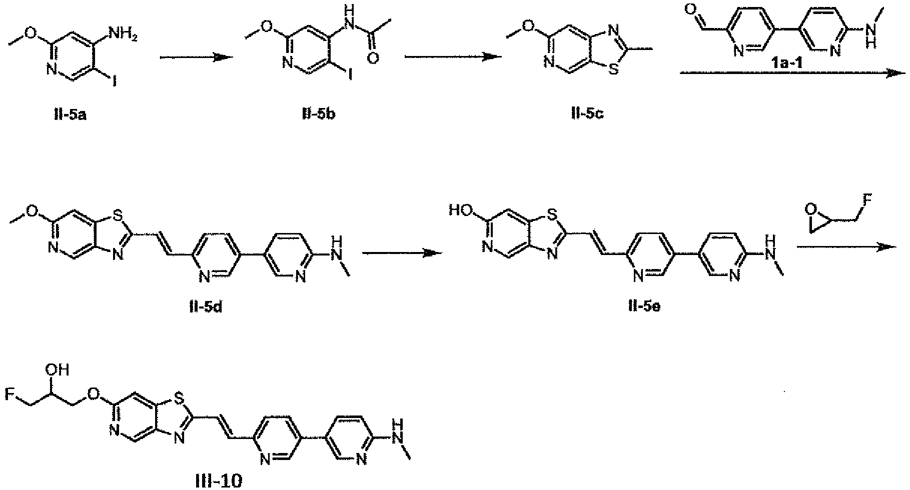

Referring to the synthetic method for III-7 in Example **III-7,** II-2a was replaced with II-5a to give product **III-10** (38 mg) as a yellow solid. LC-MS(ESI): [M+1]⁺ = 438.1.

### Example 111-11: Preparation of Compound III-11

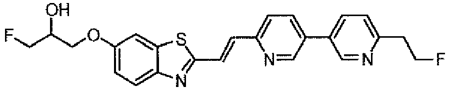

### Synthetic route for Compound III-11:

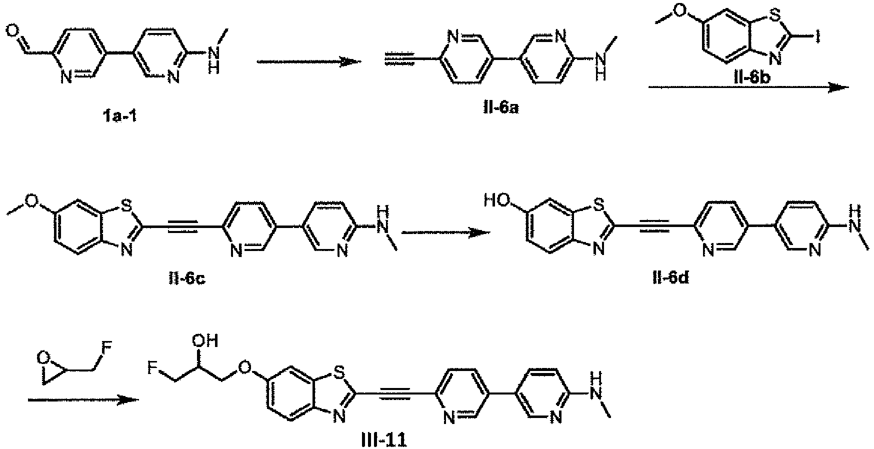

### Step 1: Preparation of Intermediate II-6a

1a-1 (2.13 g, 10 mmol) was dissolved in methanol (43 mL). Potassium carbonate (2.76 g, 20 mmol) was added to the mixture, followed by the addition of a solution of dimethyl (1-diazo-2-oxopropyl)phosphonate (2.14 g, 11 mmol) in methanol (14 mL) at room temperature. The resulting mixture was stirred for 2 h. The mixture was then poured into 1M sodium carbonate solution, extracted with ethyl acetate, and the organic layers were combined, washed with saturated brine, dried over sodium sulfate, filtered, and concentrated. After purification by silica gel column chromatography, Intermediate II-6a (1.29 g, 62%) was obtained as a yellow liquid. LC-MS(ESI): [M+1]⁺ = 210.1.

### Step 2: Preparation of Intermediate II-6c

In a 5 mL microwave tube, II-6a (142.3 mg, 0.68 mmol), II-6b (197.9 mg, 0.68 mmol), tetrakis(triphenylphosphine)palladium(0) (40 mg, 0.034 mmol), copper(I) iodide (0.02 g, 0.05 mmol), and triethylamine (0.28 mL, 2.04 mmol) were added, with acetonitrile (2.0 mL) as the solvent. The suspension was irradiated in a microwave reactor at 100°C for 5 minutes. After cooling to room temperature, the solvent was evaporated under vacuum. The residue was purified by silica gel column chromatography, using n-hexane:dichloromethane (1:0 to 0:1) as the eluent, to give II-6c (53 mg, 21%) as a yellow solid. LC-MS(ESI): [M+1]⁺ = 373.1.

Steps 3 and 4: Referring to the synthetic method in Example **III-6,** product **III-11** (15 mg) was obtained as a yellow solid. LC-MS(ESI): [M+1]⁺ = 435.1.

### Example III-12: Preparation of Compound III-12

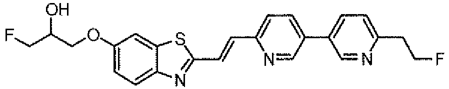

### Synthetic route for Compound III-12:

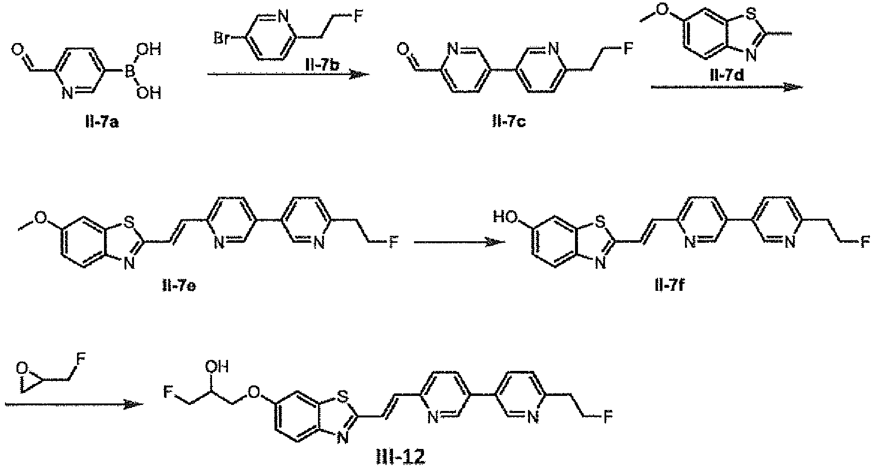

Referring to the synthetic method for II-1 in Example **II-1,** 5-bromo-2-(methylamino)pyridine was replaced with II-7b to give product **III-12** (63 mg) as a yellow solid. LC-MS(ESI): [M+1]⁺ = 454.2.

### Example III-13: Preparation of Compound III-13

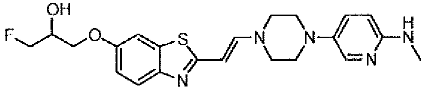

### Synthetic route for Compound III-13:

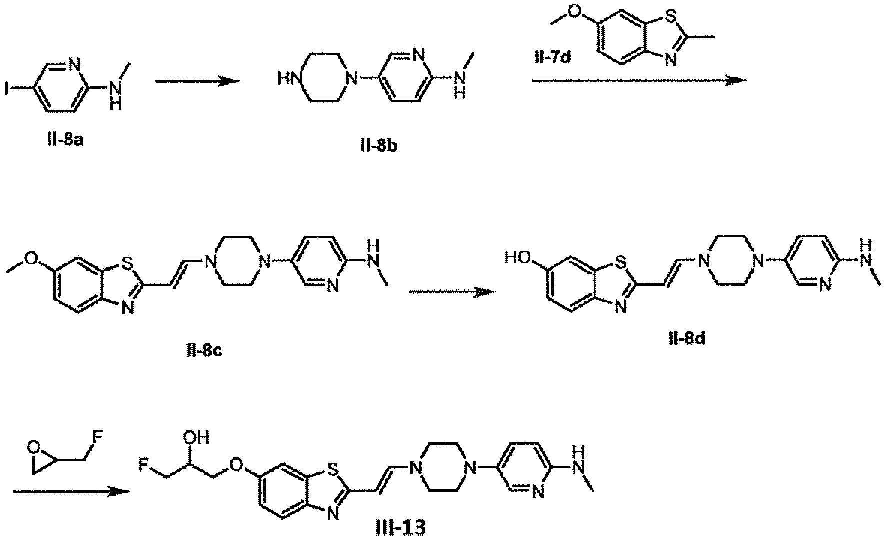

### Step 1: Preparation of Intermediate II-8b

II-8a (4.68 g, 20 mmol), piperazine (7.83 mL, 100 mmol), copper(I) iodide (76.18 mg, 0.4 mmol), 2-[(2,6-dimethylphenyl)amino]-2-oxoacetic acid (386 mg, 2 mmol), and sodium carbonate (5.3 g, 50 mmol) were mixed. N,N-Dimethylformamide (35 mL) was added in a three-neck flask, and the air was replaced with nitrogen. The temperature was raised to 100 °C for the reaction. After the reaction was complete, the reaction solution was concentrated and then separated by column chromatography to give Intermediate II-8b (1.5 g, 39%). LC-MS(ESI): [M+1]⁺ = 193.1.

### Step 2: Preparation of Intermediate II-8c

Under a nitrogen atmosphere, II-7d (358 mg, 2 mmol), 1I-8b (375 mg, 1.95 mmol), and triethyl orthoformate (2.49 mL, 15 mmol) were dissolved in methanol (12 mL). The reaction mixture was heated to 70°C and reacted for 18 hours. After cooling to room temperature, the precipitate was filtered out and washed with diethyl ether. The obtained solid was dried under high vacuum at room temperature to give Intermediate II-8c (119 mg, yield 16%). LC-MS(ESI): [M+1]⁺ = 382.2.

Steps 3 and 4: Referring to the synthetic method in Example **III-6,** product **III-13** (23 mg) was obtained as a gray solid. LC-MS(ESI): [M+1]⁺ = 444.2.

### Example III-14: Preparation of Compound III-14

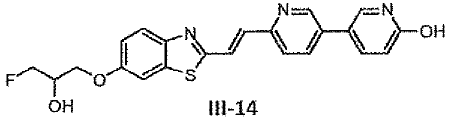

The synthetic method for Compound III-14 followed the method of Example II-1, 5-bromo-2-(methylamino)pyridine was replaced with 2-hydroxy-5-bromopyridine. Product **III-14** (56 mg) was obtained as a brownish-gray solid. LC-MS(ESI): [M+1]⁺ = 424.1.

### Example III-15: Preparation of Compound III-15

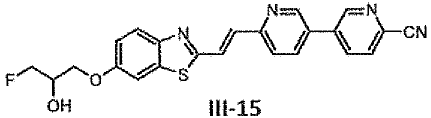

The synthetic method for Compound **III-15** followed the method of Example II-1, 5-bromo-2-(methylamino)pyridine was replaced with 5-bromo-2-cyanopyridine. Product **III-15** (35 mg) was obtained as a yellow solid. LC-MS(ESI): [M+1]⁺ = 433.1.

### Example III-16: Preparation of Compound III-16

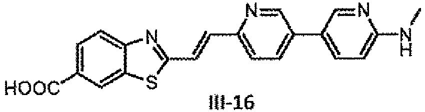

The synthetic method for Compound **III-16** followed the method of Example II-1, 6-methoxy-2-methylbenzothiazole was replaced with 2-methylbenzothiazole-6-carboxylic acid. Product **III-16** (78 mg) was obtained as a yellow solid. LC-MS(ESI): [M+1]⁺ = 389.1.

### Example III-17: Preparation of Compound III-17

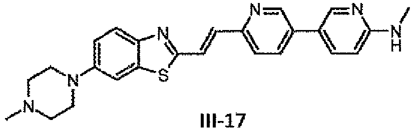

### Synthetic route for Compound III-17:

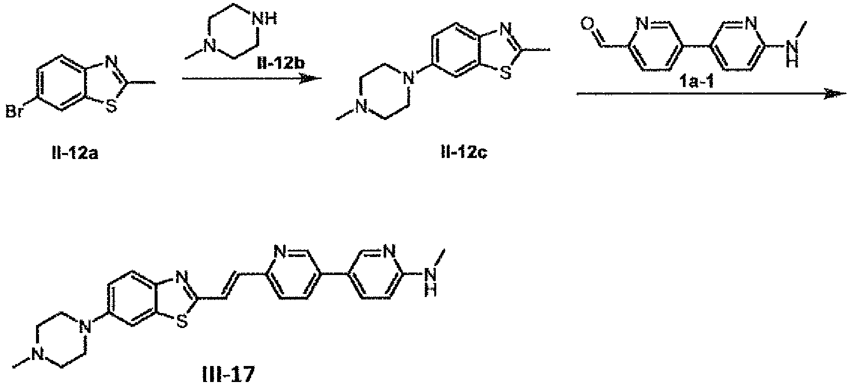

### Step 1: Preparation of Intermediate II-12c

To a solution of dry THF (5 mL) at room temperature were added **II-12a** (114.1 mg, 0.5 mmol), N-methylpiperazine (75 mg, 0.75 mmol), and K₃PO₄ (425 mg, 2.0 mmol). The reaction mixture was then purged with nitrogen for 15 minutes. Ruphos Pd G1 (81.7 mg, 0.1 mmol) was then added. The reaction mixture was purged with argon for 5 minutes, and then the mixture was stirred in a sealed tube at 80 °C for 24 hours. After the reaction, the mixture was filtered through a celite pad, which was washed with ethyl acetate (50 mL). The filtrate was concentrated under reduced pressure and separated by column chromatography to give Intermediate II-12c (28.5 mg, yield 23%). LC-MS(ESI): [M+1]⁺ = 248.1.

### Step 2: Preparation of Compound III-17

Intermediate 1a-1 (24.6 mg, 0.115 mmol) and II-12c (28.5 mg, 0.115 mmol) were dissolved in dimethyl sulfoxide (2 mL). 50% aqueous potassium hydroxide solution (1 mL) was slowly added dropwise, and the mixture was stirred at room temperature overnight. After the reaction was complete, a yellow solid precipitated. The solid was filtered, and the filter cake was washed with water. The solid was collected and dried to give the product **III-17** (35 mg, yellow solid, yield 68%). LC-MS(ESI): [M+1]⁺ = 443.2.

### Example III-18: Preparation of Compound III-18

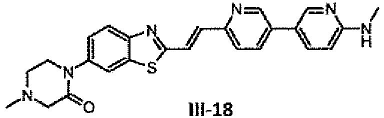

The synthetic method for Compound III-18 followed the method of Example III-17, N-methylpiperazine was replaced with 4-methylpiperazin-2-one. Product **III-18** (12 mg, a yellow solid) was obtained. LC-MS(ESI): [M+1]⁺ = 457.2.

### Example III-19: Preparation of Compound III-19

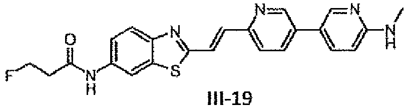

### Synthetic route for Compound III-19:

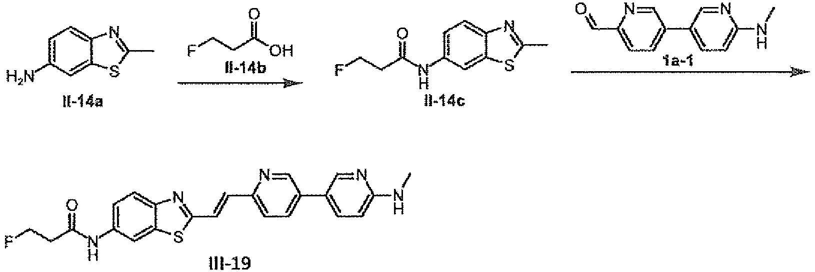

### Step 1: Preparation of Intermediate II-14c

At room temperature, 3-fluoropropanoic acid II-14b (35.5 mg, 0.386 mmol) was dissolved in dichloromethane (4.0 mL). N,N,N',N'-Tetramethyl-O-(7-azabenzotriazol-1-yl)uronium hexafluorophosphate (293 mg, 0.77 mmol), N,N-Diisopropylethylamine (127 µL, 0.77 mmol), and II-14a (63.4 mg, 0.386 mmol) were added. The reaction was carried out at room temperature for 40 minutes. After completion, the mixture was diluted with dichloromethane (100 mL). The organic phase was washed successively with water (50 mL) and saturated brine (50 mL), dried over anhydrous sodium sulfate, filtered, and concentrated under reduced pressure. The obtained residue was purified by silica gel column chromatography (eluent: methanol/dichloromethane = 5/95) to give II-14c (74 mg, yield: 80%) as a light yellow oil. LC-MS(ESI): [M+1]⁺ = 239.1.

Step 2: Referring to the synthetic method in Example III-17, II-12c was replaced with II-14c to give product **III-19** (24 mg, a yellow solid). LC-MS(ESI): [M+1]⁺ = 434.2.

### Example III-20: Preparation of Compound III-20

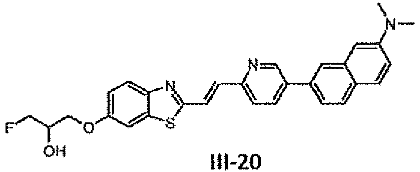

The synthesis of Compound III-20 followed the synthetic method of Example **II-1**, 5-bromo-2-(methylamino)pyridine was replaced with 7-bromo-N,N-dimethylnaphthalen-2-amine. Product III-20 (5.6 mg, a gray solid) was obtained. LC-MS(ESI): [M+1]⁺ = 500.2.

### Example III-21: Preparation of Compound III-21

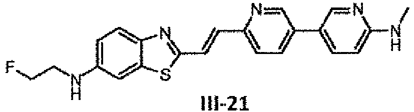

The synthesis of Compound III-21 followed the synthetic method of Example 111-17, N-methylpiperazine was replaced with 2-fluoroethanamine hydrochloride. Product **III-21** (34 mg, a yellow solid) was obtained. LC-MS(ESI): [M+1]⁺ = 406.2.

### Example III-22: Preparation of Compound III-22

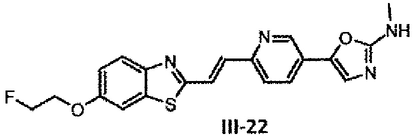

### Synthetic route for Compound III-22:

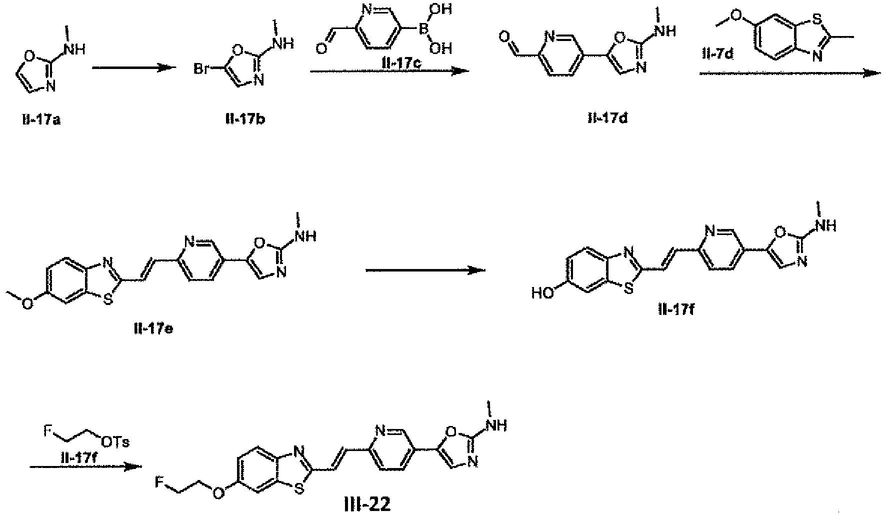

### Step 1: Preparation of Intermediate II-17b

N-Bromosuccinimide (NBS, 585 mg, 3.29 mmol) was added to a solution of II-17a (323 mg, 3.29 mmol) in dimethylformamide (DMF, 17 mL). The reaction mixture was stirred at 30°C for 1 hour. The reaction solution was concentrated and then purified by flash silica gel chromatography (methanol in ethyl acetate increased from 0% to 20%) to give II-17b (286 mg, yield 49%) as a yellow solid. LC-MS(ESI): [M+1]⁺ = 176.9.

### Step 2: Preparation of Intermediate II-17d

II-17b (286 mg, 1.62 mmol), II-17c (244.5 mg, 1.62 mmol), 1,1'-bis(diphenylphosphino)ferrocene]dichloropalladium(II) (118.5 mg, 0.162 mmol), and potassium carbonate (670 mg, 4.85 mmol) were dissolved in 1,4-dioxane (10 mL) and water (3 mL). The mixture was reacted at 80°C overnight under nitrogen protection. After the reaction was complete, the product was purified by flash silica gel column chromatography (PE/EA) to give product **II-17d** (178 mg, yield 54%). LC-MS(ESI): [M+1]⁺ = 204.1.

Steps 3, 4, 5: Referring to the synthetic method in Example **II-1,** 1a-1 was replaced with II-17d and epifluorohydrin was replaced with fluoroethyl p-toluenesulfonate (II-17f), to give product **III-22** (59 mg, a yellow solid). LC-MS(ESI): [M+1]⁺ = 397.1.

### Example III-23: Preparation of Compound III-23

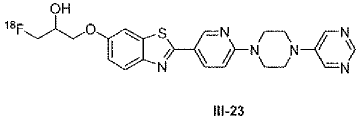

### Synthetic route for Compound III-23:

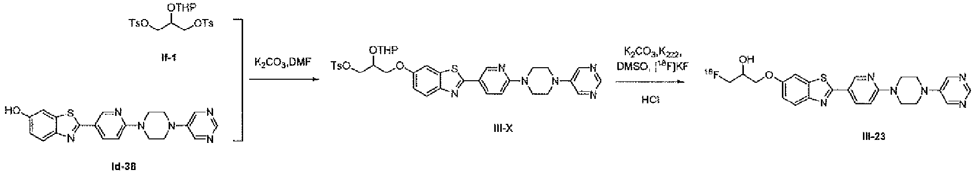

### Step 1: Synthesis of Precursor III-X

Id-38 (0.06 mmol), If-1 (0.29 mmol), and potassium carbonate (16 mg, 0.12 mmol) were added to DMF (0.5 mL) and stirred at 80 °C for 2 h. After the reaction was complete, the mixture was purified by flash silica gel column chromatography (PE/EA) to give product **III-X** (3.8 mg, 0.006 mmol) as a yellow solid in 57% yield. MS-ESI: m/z 703.2 [M + H]⁺.

### Step 2: Synthesis of Precursor III-23

150 mCi of ¹⁸F was trapped on a QMA column. The ¹⁸F was eluted into the reaction vial with 0.6 mL of eluent (150 mg Kryptofix 222, 15 mg K₂CO₃ dissolved in 9 mL acetonitrile and 1 mL water). The solvent was dried under high-purity nitrogen by heating at 110 °C for 20 min. 0.5 mg of I-X was added to the reaction vial and stirred at 140 °C for 10 min. 0.2 mL of 2N hydrochloric acid was then added, and the mixture was stirred at 105 °C for 8 min to remove the THP protecting group. The reaction solution was separated and purified by HPLC (HPLC conditions: CAPCELL PAK C18 UG80 10mm×250mm, acetonitrile: water = 25:75, flow rate: 3 mL/min). The product was collected and subjected to solid-phase extraction using a C18 column, and eluted with ethanol to obtain the final product **III-23,** with a labeling efficiency of 14%.

### Reference Example 1: Preparation of Reference Compound 1

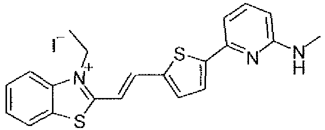

### Synthetic route for Reference Compound 1:

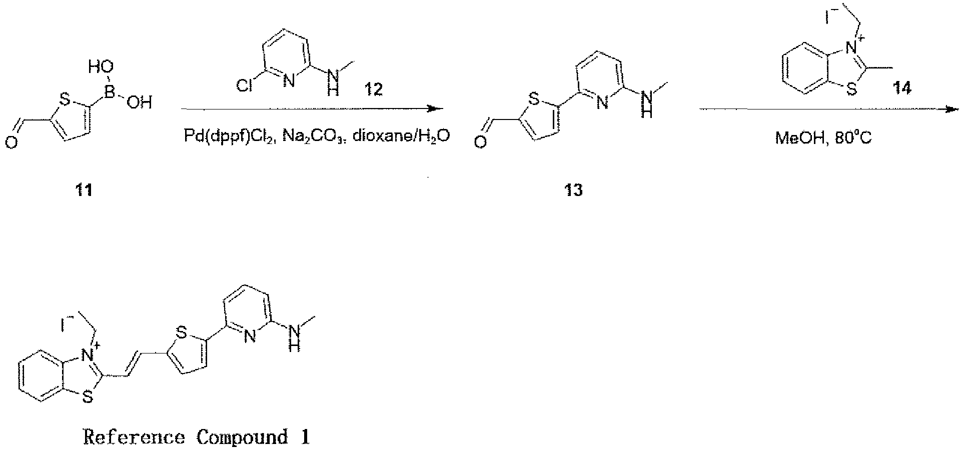

### Step 1: Preparation of Intermediate 13

To a solution of Compound 11 (4 g, 25.64 mmol) and Compound 12 (3.6 g, 25.64 mmol) in 1,4-dioxane (100 mL) and water (20 mL) were added Pd(dppf)Cl₂ (220 mg, 0.3 mmol) and K₂CO₃ (10.46 g, 76.92 mmol). The reaction mixture was heated to 80°C under nitrogen protection and reacted for 15 hours. After the reaction, the reaction mixture was filtered and concentrated. The residue was purified by column chromatography (petroleum ether:ethyl acetate = 3:1) to give Compound 13 (800 mg, 14.3% yield) as a yellow solid. **¹H NMR** (400 MHz, CDCl₃) δ 9.90 (s, 1H), 7.73 (d, J = 4.0 Hz, 1H), 7.69-7.59 (m, 1H), 7.49 (t, J = 8.0 Hz, 1H), 7.04 (d, J = 6.8 Hz, 1H), 6.40 (d, J = 8.4 Hz, 1H), 2.99 (d, J = 4.0 Hz, 3H).

### Step 2: Preparation of Reference Compound 1

Compound 13 (600 mg, 2.75 mmol) and Compound 14 (836 mg, 2.75 mmol) were dissolved in methanol (50 mL) and heated to 80°C for 15 hours. After the reaction was complete, the mixture was cooled to room temperature and filtered. The solid was recrystallized from methanol, washed, and dried to give **Reference Compound 1** (60 mg, 4.3%) as a yellow solid. LCMS: m/z(ESI) = 378.2 [M + H]⁺. ¹H NMR (400 MHz, DMSO) δ 8.45 - 8.41 (m, 2H), 8.28 (d, *J =* 8.6 Hz, 1H), 7.99 (d, *J* = 3.8 Hz, 1H), 7.87 (d, *J* = 15.2 Hz, 2H), 7.82 - 7.70 (m, 2H), 7.49 (t, *J* = 7.8 Hz, 1H), 7.20 (d, *J =* 7.4 Hz, 1H), 6.74 (d, *J =* 4.6 Hz, 1H), 6.49 (d, *J* = 8.4 Hz, 1H), 4.95 (d, *J =* 7.2 Hz, 2H), 2.88 (d, *J* = 4.8 Hz, 3H), 1.48 (t, *J =* 7.2 Hz, 3H).

### Reference Example 2: Preparation of Reference Compound 2

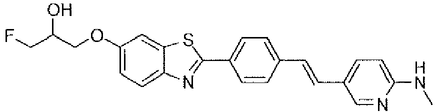

The synthesis of Reference Compound 2 was carried out according to published patent KR20190090448. Reference Compound 2 was obtained as a yellow solid (61.7 mg). LCMS: m/z(ESI) = 435.6 [M+H]⁺.

### Biological Example 1: Protein Level Activity Assay

Fluorescence method and Surface Plasmon Resonance (SPR) are commonly used assays to detect the interaction between small molecules and proteins. The applicant used these two methods to test the binding affinity of the example compounds to α-synuclein.

### 1.1 Experimental Methods:

### (1) Preparation of α-syn:

The α-syn plasmid and the yeast N-acetyltransferase complex B plasmid were co-transformed into BL21 (DE3) E. coli competent cells. The transformation mixture was plated on solid culture plates containing dual antibiotics (ampicillin and chloramphenicol, Gibco, Cat#15140148) and cultured overnight at 37°C for plasmid transformation. A single colony was picked and placed in 20 ml of 2×YT medium (Sigma-Aldrich, 2× Yeast Extract Tryptone Medium, containing corresponding penicillin and chloramphenicol) and cultured overnight at 37°C, 220 rpm. Then, the bacterial solution was inoculated into 1 L of 2×YT medium at a volume ratio of 1:100. After 1 hour (when the OD600 of the culture was 0.8-1.0), 1 mM IPTG (Isopropyl β-D-1-thiogalactopyranoside) was added, and induction was carried out at 37°C for 4 h. Finally, E. coli was collected by centrifugation (4°C, 4500 rpm, 20 min). The collected E. coli was resuspended in 60-80 ml of bacterial lysis buffer (100 mM Tris-HCl, pH 8.0, 1 mM EDTA, 1 mM PMSF). The bacteria were then lysed by high-pressure homogenization (4°C, 10 min). The cell lysate was centrifuged (4°C, 15000 rpm, 25 min), and the supernatant was boiled in a water bath for 15 min. After centrifugation again (4°C, 15000 rpm, 25 min), streptomycin sulfate (20 mg/ml, wt/vol) was added to the supernatant, and the solution was placed in a 4°C environment with continuous stirring for 30 min. The lysate was further centrifuged (4°C, 15000 rpm, 25 min), and the pH of the supernatant was adjusted to 3.5. Finally, after centrifugation (4°C, 15000 rpm, 25 min), the supernatant was dialyzed overnight (4°C) (dialysis buffer: 25 mM Tris-HCl, pH 8.0).

First, purification was performed using an anion exchange column (Q column). The Q column was washed with Buffer B (25 mM Tris-HCl, pH 8.0, 1 M NaCl) and equilibrated with Buffer A (25 mM Tris-HCl, pH 8.0) (for 5-10 column volumes). The protein dialysate was filtered through a 0.22 µm filter membrane and loaded onto the Q column using the protein purification system ÄKTA. Unbound protein was then washed away with Buffer A. Gradient elution was performed with 0-60% Buffer B over 40 min. The eluate was collected using the ÄKTA automatic fraction collector. Then, purification was carried out using a gel size exclusion column (Superdex 75). The target protein solution eluted from the Q column was collected, concentrated, filtered, and loaded using a sample loop. The molecular sieve was eluted with DPBS buffer (Thermo Fisher, Cat#14040117), and the eluate was collected using the ÄKTA automatic fraction collector. Pure α-synuclein monomer was prepared.
(2) Preparation of α-synuclein aggregates: α-Syn protein monomer (200 µM, in 50 mM Tris, pH 7.5, 150 mM KCl buffer) was placed on a ThermoMixer shaker and incubated at 37°C, 900 rpm for 5-7 days to form amyloid fibrils. Finally, transmission electron microscopy (TEM) was used to examine the morphology of mature α-syn fibers.
(3) SPR assay: The CMD 500 chip (GE Compamy) was washed clean with double-distilled water, air-dried, and then the "d°Ck" program was run to enter the biacore T200 instrument. The "desorb" program was used to remove impurities, and the "Prime" program was run to remove bubbles. The incubated α-syn aggregates (5 mg/mL) were diluted with NaAc buffers of pH 4.0, 4.5, 5.0, and 5.5 to 20 µg/mL samples. Samples were injected sequentially for 60 s, eluted with NaOH after injection, and the optimal coupling buffer was selected based on the coupling amount. 200 µL of chip activation solution was injected for 900 s to activate the carboxyl groups on the chip. α-Syn aggregates were diluted to 50 µg/mL protein sample with NaAc buffer (pH=4.0). The protein sample was then injected for 110 s, repeated 10 times, to allow sufficient coupling of the protein to the chip. 900 s of blocking solution (original protein blocking solution from GE Compamy) was injected to block unreacted carboxyl groups on the chip. The small molecules to be tested were diluted to different concentrations, and the "Kinetic" program in Biacore was run for sequential injection. The binding force and kinetic plots of the small molecules were derived using the system software.
(4) Fluorescence-based protein binding assay: The small molecule was prepared into a 10 mM stock solution using DMSO, then diluted to 20 µM with PBS, followed by 7 gradient dilutions (threefold each). 30 µL of the test compound was added to a 384-well plate. The experimental group received 30 µL of α-syn aggregates (Example I and III series: 1 µM; Example II series: 3 µM), while the control group received an equal volume of PBS. The 384-well plate was incubated at room temperature with shaking (50 rpm) for 1 hour. The maximum absorption and emission wavelengths of the small molecule were measured using a microplate reader, and the fluorescence value was measured at these wavelengths. The fluorescence change values for different concentrations were calculated by subtracting the control group from the experimental group, and the binding affinity (Kd) between the small molecule and the protein was calculated using the Saturation binding module of GraphPad Prism.

### 1.2 Experimental Results:

**Table 1: Test Results of α-Synuclein Aggregate Binding Ability of Test Compounds**

| Compound | Fluorescence method | SPR | Compound | Fluorescence method | SPR |
|---|---|---|---|---|---|
| Example I-1 | A | A | Example I-23 | C | B |
| Example I-2 | A | A | Example I-24 | C | D |
| Example I-3 | A | A | Example I-25 | C | C |
| Example I-4 | C | C | Example I-26 | A | A |
| Example I-5 | B | C | Example I-27 | C | B |
| Example I-6 | D | C | Example I-28 | A | A |
| Example I-7 | A | A | Example I-29 | B | B |
| Example I-8 | A | B | Example I-30 | A | A |
| Example I-9 | B | A | Example I-31 | A | A |
| Example I-10 | C | B | Example I-32 | A | A |
| Example I-11 | A | A | Example I-33 | A | B |
| Example I-12 | C | C | Example I-34 | B | B |
| Example I-13 | D | C | Example I-35 | C | C |
| Example I-14 | D | C | Example I-36 | A | B |
| Example I-15 | D | C | Example I-37 | D | B |
| Example I-16 | A | A | Example I-38 | A | A |
| Example I-17 | A | B | Example I-39 | A | c |
| Example I-18 | D | D | Example I-40 | B | B |
| Example I-19 | A | A | Example I-41 | C | C |
| Example I-20 | A | A | Example I-42 | B | A |
| Example I-21 | c | c | Example I-43 | c | A |
| Example I-22 | B | A | Example I-44 | B | B |
| | | | Example I-45 | A | A |
| Example II-1 | A/0.07µM | A | Example II-12 | D | C |
| Example II-1(R) | | | | | |
| | A/0.08µM | | | | |
| Example II-1(S) | A/0.08µM | | | | |
| Example II-2 | A | A | Example II-13 | A | B |
| Example II-3 | C | C | Example II-16 | A | A |
| Example II-4 | B | A | Example II-18 | A | A |
| Example II-5 | B | A | Example 11-19 | A | A |
| Example II-7 | B | C | Example II-20 | B | B |
| Example II-9 | A | A | Example II-23 | A | B |
| Example II-10 | C | B | Example II-24 | A | B |
| Example II-11 | C | C | Example II-25 | A | A |
| Example II-26 | B | B | Example II-27 | A | A |
| Example III-1 | A | ND | Example III-12 | B | C |
| Example III-2 | A | ND | Example III-13 | D | A |
| Example III-3 | C | ND | Example III-14 | C | B |
| Example III-4 | B | ND | Example III-15 | D | C |
| Example III-5 | A | ND | Example III-16 | B | B |
| Example III-6 | A | B | Example III-17 | A | A |
| Example III-7 | A | A | Example III-18 | B | A |
| Example III-8 | A | A | Example III-19 | B | C |
| Example III-9 | A | A | Example III-20 | B | B |
| Example III-10 | A | B | Example III-21 | A | A |
| Example III-11 | B | B | Example III-22 | B | A |
| Reference Compound 1 | not fit | ND | Reference Compound 2 | 3.15 | ND |

| | | | | | |
|---|---|---|---|---|---|
| Note: A represents a K_{d} value less than 0.01-0.5 µM, B represents a K_{d} value between 0.5~2 µM, C represents a K_{d} value between 2~10 µM, D represents a K_{d} value greater than 10 µM, and ND represents not determined. | | | | | |

### Biological Example 2: Immunofluorescence Staining of α-syn Aggregates in Primary Neurons

### 2.1 Experimental Methods:

(1) Primary neuron culture: Coverslips were placed in a 24-well plate, and poly-L-lysine solution was added. SD rats at 15-18 days of gestation were dissected, and the left and right cerebral cortices of the fetal rats were removed and placed in HBSS buffer containing HEPES. The fetal rat cortical tissue was rinsed with HBSS buffer, then digested using activated papain and DNase. The tissue was placed in a 37°C cell culture incubator, gently inverted every 5 min. After digestion, the tissue was filtered through a 40 µm cell strainer, and the filter was rinsed with 15 ml of plating medium (DMEM containing 10% FBS and 1% PS (without pyruvate)). The cell solution was centrifuged at 900 rpm for 5 min, resuspended in plating medium, and then counted. Cells were plated at a density of 10-15×10⁴ cells/well in 0.5 ml of plating medium/well. After culturing for 1-2 h in the cell culture incubator, the medium was replaced with Neurobasal medium (Neurobasal medium containing 1% PS, B27 supplement, and 0.5 mM GlutaMAX), 1 ml/well. Finally, neurons were cultured in the cell culture incubator until mature (7-10 days).
(2) Cell treatment: After primary neurons were cultured for 7-10 days *in vitro,* α-syn PFF was added to the medium to a final concentration of 100 nM. The treated neurons were further cultured for 30 days, and the neurons were collected for sample preparation.
(3) Immunofluorescence staining: The cell culture medium was aspirated, and cells were washed three times with PBS. 0.3% Triton X-100 was added and incubated for 10 min. After washing with PBS, 10% goat serum was added for blocking for 1 h. After washing with PBS, p-129-α-syn antibody (1:600, ab51253, Abcam) was added and incubated overnight at 4°C. After washing with PBS, secondary antibodies (1:1000, goat-anti-rabbit Alexa Fluor 594 and goat-anti-mouse Alexa Fluor 488, Invitrogen) were added and incubated at room temperature for 2 h. Finally, the test compound was added and incubated at room temperature for 1 h. After washing with PBS, the slides were mounted and imaged using a fluorescence microscope or laser confocal (Olympus laser scanning confocal microscope FV3000).

### 2.2 Experimental Results:

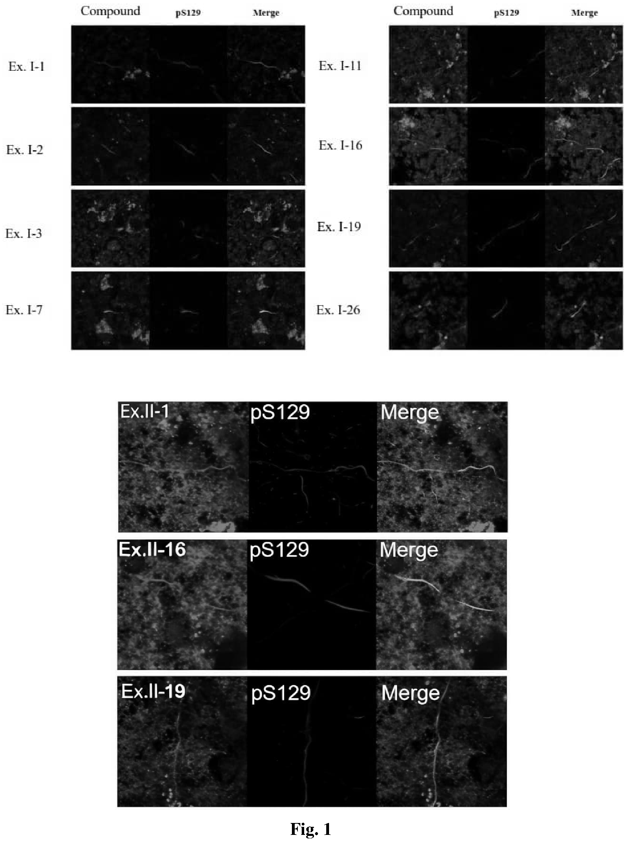

The immunofluorescence staining results are shown in **Figure 1****.** The compounds of the present application possess green autofluorescence. The experimental results show that the fluorescence of the test compounds co-localized with the fluorescence signal of the pS129 antibody (pathological α-syn), indicating that the compounds of the present application can bind to α-syn aggregates in primary neurons. That is, the compounds of the present application can detect α-syn aggregates in primary neurons.

### Biological Example 3: Immunofluorescence Staining of PFF Mouse Brain Sections

### 3.1 Experimental Methods:

(1) Construction of pre-formed fibril (PFF) mouse model (i.e., PD mouse model): Mice were weighed, and the injection volume of α-syn PFF was calculated (0.2 µg/g). 6-week-old mice were anesthetized and fixed on a stereotaxic apparatus, the head position of the mice was adjusted to make it symmetrical between left and right. The hair on the mouse head was shaved, and the scalp was cut to expose the skull. The mouse brain was adjusted under a microscope in conjunction with the microreader on the stereotaxic apparatus to make it flat and symmetrical from front to back and left to right. A cranial drill was used to make a hole in the dorsal striatum (dSTR). α-syn PFF and PBS were then injected into the bilateral brain regions of the mice using a microinjection pump. The PD mice were raised for 6-9 months post-surgery, then euthanized, and brain tissue was dissected.
(2) Brain section preparation: Brain tissue was fixed overnight in 4% paraformaldehyde solution, dehydrated in sucrose solution, embedded using a tissue freezing embedding machine, and then cut into 30 µm thick sections using a cryostat (Leica CM1860). The sections were collected in cryoprotectant solution (30% sucrose (w/v), 30% ethylene glycol (v/v) dissolved in PBS) and stored at -20°C for subsequent fluorescent staining and imaging.
(3) Immunofluorescence staining: Prepared brain sections were washed three times with PBS, then incubated with 0.3% Triton X-100 for 10 min. After washing three times with PBS, 10% goat serum was added for blocking for 1 h. After washing three times with PBS, p-129-α-syn antibody (1:600, ab51253, Abcam) was added and incubated overnight at 4°C. After washing three times with PBS, secondary antibodies (1:1000, goat-anti-rabbit Alex Fluor 594 and goat-anti-mouse Alex Fluor 488, Invitrogen) were added and incubated at room temperature for 2 h. After washing three times with PBS, the test compound was added and incubated at room temperature for 1 h. After washing three times with PBS, the sections were mounted and observed using a fluorescence microscope or laser confocal.

### 3.2 Experimental Results:

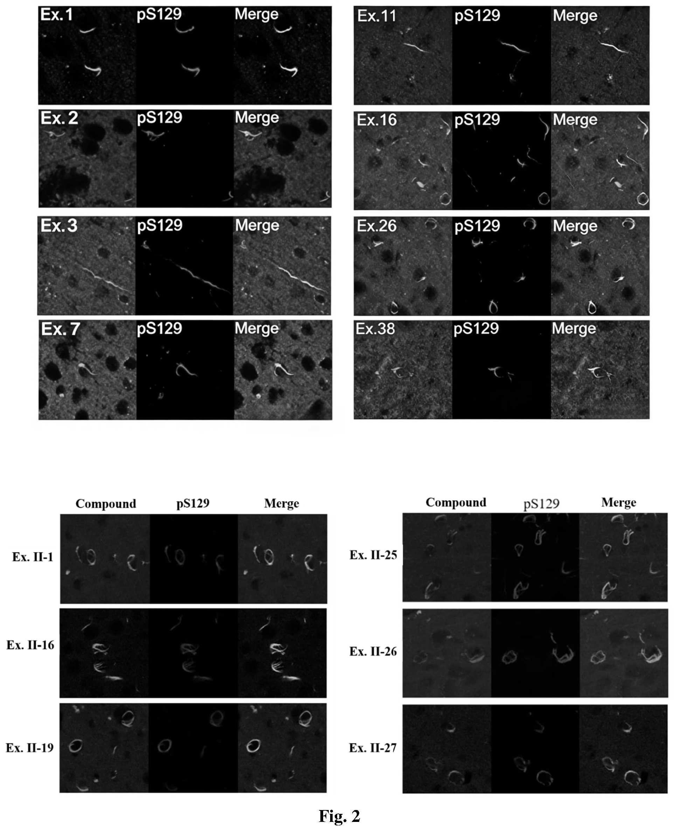

The experimental results are shown in **Figure 2****.** The autofluorescence signal of the preferred compounds of the present application (also referred to as tracers) co-localized with the signal of the pS129 antibody (pathological α-syn), with no other non-specific binding signals. This indicates that the compounds of the present application can specifically recognize pathological α-syn aggregates in brain tissue, validating the ability of the compounds to bind α-syn aggregates in brain tissue.

### Biological Example 4: Immunofluorescence Staining of PD Patient Brain Sections

### 4.1 Experimental Methods:

Brain tissue was obtained from an 86-year-old Asian female PD patient with a 14-year disease duration, dissected within 12 hours post-mortem. The immunofluorescence staining method was consistent with that used for mouse brain sections in Biological Test Example 3.

### 4.2 Experimental Results:

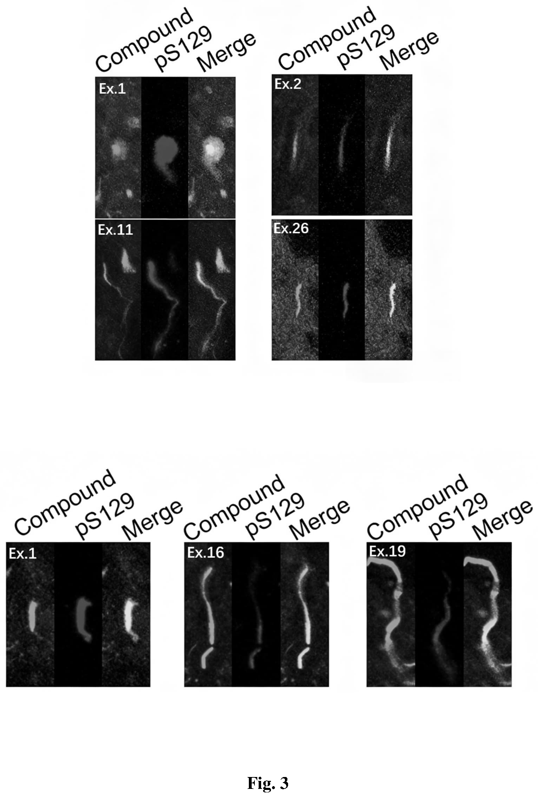

The experimental results **(****Figure 3****)** show that the autofluorescence signal of the compounds of the present application (tracers) co-localized with the α-syn antibody signal, indicating that the compounds of the present application can label pathological α-syn in patient brain sections. Combined with the immunofluorescence staining results from cells and mouse brain sections, this demonstrates that the compounds of the present application possess good target binding ability *in vitro.*

### Biological Example 5: Ex-vivo Staining of PFF Mouse Brain Sections

### 5.1 Experimental Methods:

The PFF mouse model was constructed using the method described in Biological Test Example 3. 1 mg/kg of the test compound was injected into mice via the tail vein. Two hours later, the mice were euthanized, and brain tissue was removed, sectioned, and subjected to immunofluorescence staining.

### 5.2 Experimental Results:

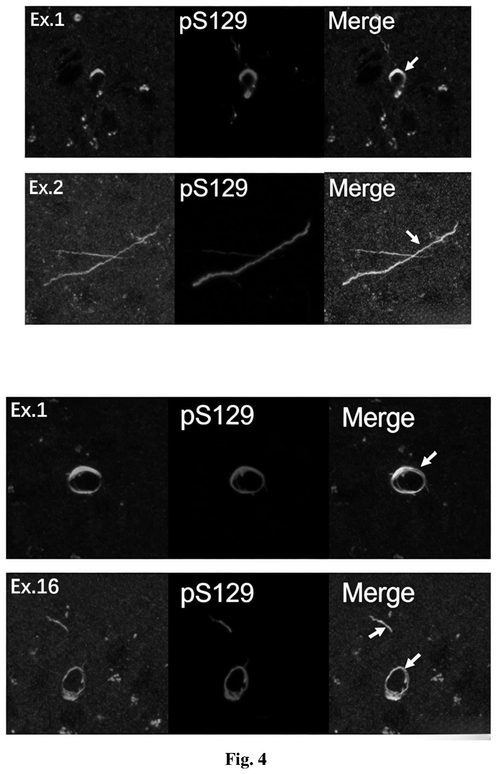

The experimental results are shown in **Figure 4****.** The compounds of the present application (also known as the tracer) were able to cross the blood-brain barrier in mice and specifically recognize pathological α-syn in the mouse brain, indicating good *in vivo* druggability.

### Biological Example 6: Comparison of Binding Activity of Compounds to α-Syn, Aβ, and Tau

### 6.1 Test Compounds

Example II-1 compound, Reference Compound 1, Reference Compound 2, and prior art compound C0505: 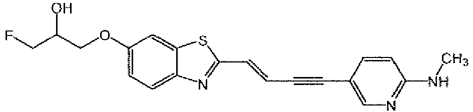 (prepared according to WO2014/097474)

### 6.2 Experimental Methods

1) The preparation method for α-synuclein aggregates is described in 1(1) of Biological Test Example 1.
2) Expression and purification of human Tau protein

Genes encoding human Tau protein (comprising amino acid sequences 266-391 (3R) and 297-391) were inserted into the pRK172 vector. The plasmids were transformed into E. coli BL21 (DE3) competent cells. The cells were cultured at 37°C until OD600 reached 0.8-1.2, 1 mM IPTG (isopropyl β-D-1-thiogalactopyranoside) was added, and expression was induced for 16 hours at 24°C. The cells were lysed in wash buffer (50 mM MES, pH 6.0, 10 mM EDTA, 10 mM DTT, 0.1 mM PMSF), followed by centrifugation and filtration. The protein was purified by SP column, then precipitated with ammonium sulfate. The precipitate was resuspended in phosphate buffer (pH 7.2-7.4) containing DTT (dithiothreitol) and further purified by Superdex 75 gel filtration column. Fractions were analyzed by SDS-PAGE, and protein concentration was determined using a nano-spectrophotometer (Winner801). Purified protein was stored in phosphate buffer (pH 7.4) containing DTT, snap-frozen, and stored at -80°C.

### 3) Preparation of Tau and Aβ fibrils

Preparation of Tau fibrils: 190 µM Tau monomer (commercial, abcam, Cat#ab246003) was dissolved in a buffer containing 10 mM phosphate buffer (PB, pH 6.0), 10 mM DTT, 200 mM MgCl₂, 0.02% NaN₃, incubated in a Corning 96-well black polystyrene microplate (Thermo) at 37°C with orbital shaking at 200 rpm using a FLUOstar Omega microplate reader (BMG LABTECH) for 48 hours.

Preparation of Aβ fibrils: 200 µM Aβ (1-40, E22Δ) peptide was dissolved in a buffer containing 10 mM phosphate buffer (PB, pH 7.4), 100 mM NaCl, 0.05% NaN₃ and incubated in a ThermoMixer at 37°C with shaking at 700 rpm for 7 days.

### 4) Fluorescence binding assay

1 µM test compound was mixed with 1 µM fibrils in a buffer containing 50 mM Tris (pH 7.5) and 150 mM KCl, incubated for 1 hour at 25°C. The fluorescence signal of the mixture was then measured using a microplate reader. A control reaction without fibrils was also set up to assess non-specific fluorescence. The experimental results are shown in the table below.

### 6.3. Experimental Results

### Binding affinity (Kd, µM) of representative compounds to α-Syn, Aβ, and Tau

| **Compound** | **α-Syn** | **Aβ** | **Tau** |
|---|---|---|---|
| Example II-1 | 0.87 | 6.38 | 8.74 |
| C0505 | 1.05 | 5.42 | 1.19 |
| Reference Compound 2 | 3.15 | 2.43 | 1.00 |
| Reference Compound 1 | not fit | not fit | not fit |

Compared to the other reference compounds, Example 11-1 of the present application exhibited significantly better binding activity for α-syn than for Aβ and Tau, indicating that the compounds of the present invention have highly selective binding activity for α-syn.

### Activity Example 7: Autoradiography (ARG) of Mouse and PD Patient Brain Sections

### 7.1 Mouse Model Preparation

Male C57BL/6J mice (2 months old, 18-20g, purchased from Shanghai Lingchang Biotechnology Co.) were housed 5 per cage in a 12-hour light/dark cycle with free access to food and water. Mice were anesthetized with a mixture of 0.5% isoflurane and 1% oxygen, and body temperature was maintained with a heating pad. α-Syn PFF (5 µg) was stereotaxically injected into the dorsal striatum (dSTR) of both hemispheres at coordinates: AP ± 2.0 mm, ML + 0.2 mm, DV - 2.60 mm. The first 0.5 µL was infused at a rate of 0.5 µL/min using a 10 µL microsyringe, followed by the remaining 2 µL at a rate of 0.2 µL/min. Control animals received sterile PBS in the same manner. Mice were monitored regularly after recovery.

### 7.2 Autoradiography (ARG)

Brain sections from the mouse model in 7.1 were prepared as described in the method of Biological Test Example 3, and PD patient brain sections were prepared as described in the method of Biological Test Example 4. Brain sections were pre-incubated in PBS buffer for 30 minutes at 25°C, then incubated with 10 nM ¹⁸F-FD4 (Example II-8 compound) in PBS buffer containing 20% ethanol for 1 hour at 25°C. After rinsing with pre-chilled PBS buffer containing 20% ethanol, the brain sections were dried and placed on imaging plates, covered with a storage phosphor screen overnight. The plates were then scanned using a high-sensitivity imaging plate scanner (CR-35 Bio plus), and the images were analyzed using AutoRAD morphological quantitative analysis software. Non-specific and off-target binding of ¹⁸F-FD4 were excluded by adding excess unlabeled FD4 (Example II-1 compound) (10 µM for mouse brain, 2 µM for human brain) and MAO-A/B inhibitors (Clorgyline/Selegiline, 10 µM), respectively.

### 7.3 Experimental Results

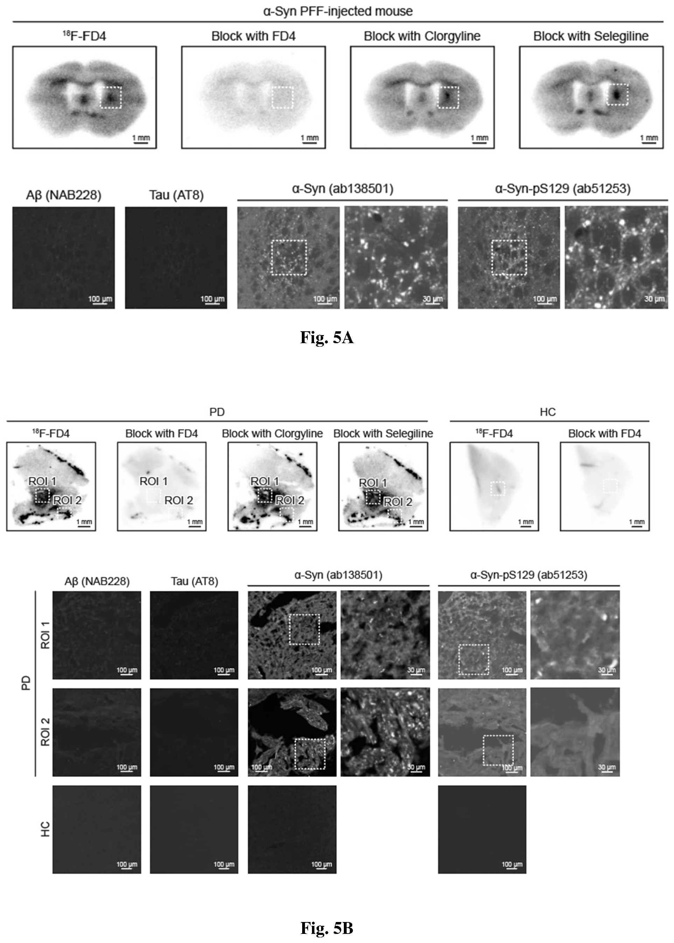

This experiment further evaluated the sensitivity of ¹⁸F-FD4 for α-syn aggregates in mouse and human brain sections. The results, shown in **Figures 5A and 5B****,** demonstrate that at a concentration of 10 nM, ¹⁸F-FD4 produced a significant radioactive signal in brain sections from α-syn PFF-injected mice and Parkinson's disease (PD) patients; no signal was detected in brain sections from healthy individuals. Referring to the experimental method in Biological Test Example 3.1, it was confirmed by antibody immunostaining (β-amyloid antibody: NAB228, Cell Signaling Technology; Tau antibody: AT8, BioLegend; α-syn antibody: ab138501, ab51253, Abeam) that the positive radioactive signal co-localized with α-syn aggregates but not with Aβ and Tau aggregates. After adding excess unlabeled FD4, the radioactive signal completely disappeared, ruling out non-specific binding of ¹⁸F-FD4. Furthermore, the radioactive signal remained unchanged after adding MAO-A and MAO-B inhibitors, excluding possible non-specific binding to monoamine oxidase (MAO). These results support the potential of FD4 as a PET imaging tracer with high sensitivity and selectivity for pathological α-syn aggregates.

### Activity Example 8: PET Imaging of Compounds of the Present Application in Rodents, Marmosets, and Human Subjects

### 8.1 Rat Model Preparation

Female Sprague Dawley rats (10-12 weeks old, 250-350 g, purchased from Shanghai Lingchang Biotechnology Co.) were housed 3 per cage in a 12-hour light/dark cycle with free access to food and water. Rats were anesthetized with a mixture of Zoletil 50 (15 mg/kg) and xylazine hydrochloride (5 mg/kg). Body temperature was maintained using a heating pad. α-Syn PFF (18 µg) was stereotaxically injected into the dorsal striatum (dSTR) of the left hemisphere at coordinates: AP +0.1 mm, ML -2.5 mm, DV -4.5 mm. The first 0.5 µL was infused at a rate of 0.5 µL/min using a glass microelectrode, followed by the remaining PFF at a rate of 0.2 µL/min. Control animals received sterile PBS in the same manner. Rats were monitored regularly after surgery.

### 8.2 Marmoset Model Preparation

Marmosets (2 years old, 400-600 g) were housed at the Jiuting Experimental Center, Institute of Neuroscience. Marmosets were housed 2 per cage in a 12-hour light/dark cycle with free access to food and water. Marmosets were anesthetized with a mixture of 0.5% isoflurane and 1 % oxygen, and body temperature was maintained with a heating pad. α-Syn PFF (200 µg) was stereotaxically injected into the right hemisphere caudate nucleus and putamen. Coordinates for caudate nucleus: interaural 9.5 mm, lateral 3 mm, depth 7.6 mm; for putamen: interaural 9.5 mm, lateral 6 mm, depth 9.3 mm. Infusion was performed at a rate of 10 nL/sec using a 10 µL microsyringe. Marmosets were monitored regularly after surgery.

### 8.3 Human Subjects

This study enrolled 14 subjects. All subjects or legal guardians provided written informed consent according to the Declaration of Helsinki before entering the study. The study was approved by the Ethics Review Committee of Huashan Hospital, Fudan University (HIRB) (Approval No.: (2024) Review No. (592)). The study was registered with the Chinese Clinical Trial Registry (Registration No.: ChiCTR2400093128).

Diagnosis was confirmed by three movement disorder specialists, with PD diagnosis according to the 2015 Movement Disorder Society (MDS) criteria 63, and MSA diagnosis according to the 2022 MDS criteria 64, meeting the definition of clinically probable or clinically established MSA. Healthy control subjects had no history of neurological or psychiatric disorders; furthermore, they showed no deficits on neurological examination or pre-imaging assessments. Pre-imaging assessments included motor and cognitive evaluation, complete blood count (CBC), urinalysis (UA), liver function tests (alanine aminotransferase [ALT] and aspartate aminotransferase [AST]), kidney function tests (serum creatinine and blood urea nitrogen [BUN]), serum electrolyte analysis, and electrocardiogram (ECG). Vital signs were monitored at baseline and at 10 and 30 minutes post-injection. Two-week follow-up included repeat laboratory assessments and documentation of adverse events.

### 8.4. PET Imaging in Rodents and Marmosets

The above-mentioned model rats and marmosets were anesthetized with 2.0-4.0% isoflurane during PET scanning. ¹⁸F-FD4 was injected via the tail vein at a dose of 1-1.5 mCi/rat and 1.5-2.0 mci/marmoset. Dynamic PET scans were performed for 90 minutes using a Siemens INVEON PET/CT scanner. CT was used for attenuation correction during PET acquisition, and CT scans lasted approximately 5 minutes. PET images were reconstructed using a filtered back-projection algorithm with CT attenuation correction. The reconstruction pixel size was 0.2 mm × 0.2 mm × 0.8 mm. Image analysis was performed using PMOD software. The ROI method on the Inveon platform was used to analyze uptake in key brain regions. Each experiment for each group was performed twice.

### 8.5 PET Imaging in Human Subjects

¹⁸F-FD4 imaging was performed in 3D mode on a PET/CT scanner (u780/UExplorer, United Imaging Healthcare, Shanghai, China) at Huashan Hospital, Fudan University, Shanghai. Each participant received an injection of 6-8 mCi of ¹⁸F-FD4. A low-dose CT transmission scan was acquired for attenuation correction before the PET scan. Healthy controls underwent dynamic imaging from 0-100 minutes post-injection, while PD and MSA patients underwent a 20-minute PET scan (60-80 minutes post-injection). Image reconstruction was performed using the Ordered Subset Expectation Maximization 3D (OSEM 3D) method. Additionally, all subjects who underwent ¹⁸F-FD4 PET/CT imaging also underwent a separate 3D T1 MRI scan using a Siemens MAGNETOM Prisma scanner.

### 8.6 Experimental Results

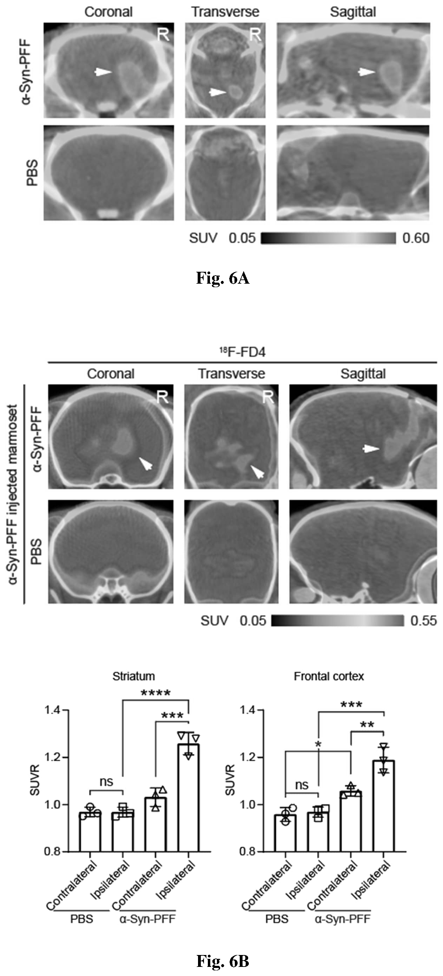

**Figure 6A** shows PET imaging of PFF-injected rats (right striatum, 0.5 months) after 50-60 min of imaging, demonstrating that ¹⁸F-FD4 can effectively cross the blood-brain barrier and accumulate in the right striatum region of α-syn model rats, indicating that this molecule can image α-syn PFF surrounding the striatum.

**Figure 6B** shows PET imaging of PFF-injected marmosets (right striatum, 6 months) after 50-60 min of imaging and a comparison diagram of standard uptake value ratios (SUVR) for the right striatum and frontal cortex. The graphic information shows that ¹⁸F-FD4 can effectively cross the blood-brain barrier, and uptake is elevated in the right striatum region and frontal cortex region of α-syn model marmosets, suggesting that this molecule can image α-syn aggregates surrounding these brain regions.

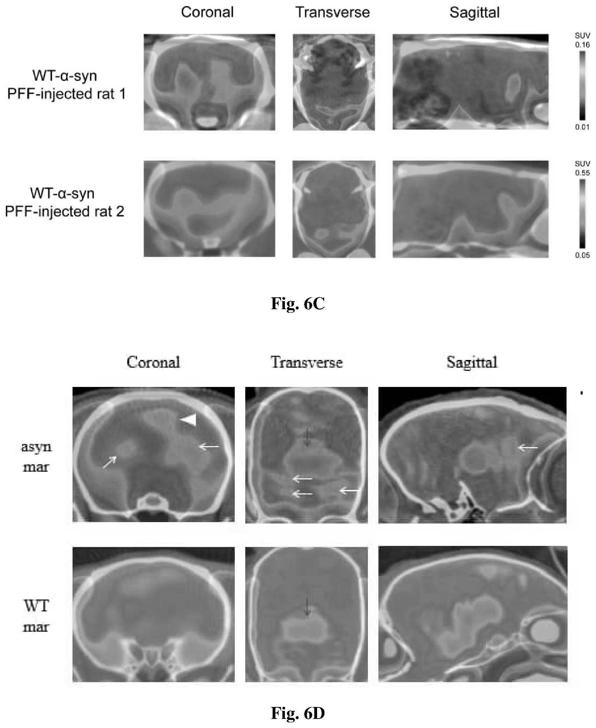

**Figure 6C** shows PET imaging of PFF-injected rats (left striatum, 4 months) after 55-60 min of imaging, demonstrating that FD17 (Example II-29 compound) can effectively cross the blood-brain barrier, and uptake is elevated in bilateral striatum of α-syn model rats, with higher signal in the left striatum than the right. This signal is consistent with the distribution of α-syn in the model rat brain, indicating that FD17 can effectively label α-syn.

**Figure 6D** shows PET imaging of PFF-injected marmosets (right striatum, 5 months) after 50~60 min of imaging, demonstrating that FD17 can effectively cross the blood-brain barrier and accumulate in the striatum region of α-syn model marmosets, with higher uptake in the right striatum than the left. Simultaneously, uptake in the striatum of WT marmosets was not elevated, indicating that this molecule can image α-syn pathology surrounding the striatum.

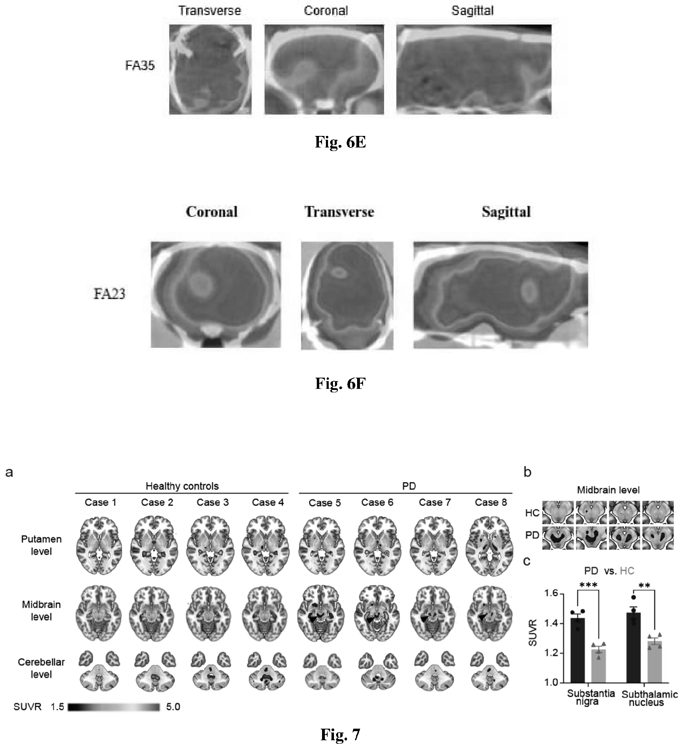

**Figure 6E** shows PET imaging of PFF-injected rats (left striatum, 0.5 months) after 30~40 min of imaging, demonstrating that FA35 (Example III-23 compound) can effectively cross the blood-brain barrier and accumulate in the striatum region of α-syn model rats, indicating that this molecule can image α-syn PFF surrounding the striatum.

**Figure 6F** shows PET imaging of PFF-injected rats (left striatum, 0.5 months) after 60-80 min of imaging, demonstrating that FA23 (Example I-48 compound) can effectively cross the blood-brain barrier and accumulate in the striatum region of α-syn model rats, indicating that this molecule can image striatally injected α-syn PFF.

**Figure 7** shows PET imaging of human subjects treated with the ¹⁸F-FD4 compound. ¹⁸F-FD4 shows a significant increase in radioligand uptake, which could be observed at the midbrain level (as indicated by the arrows). Non-specific signal in the SN and SNc is very low.

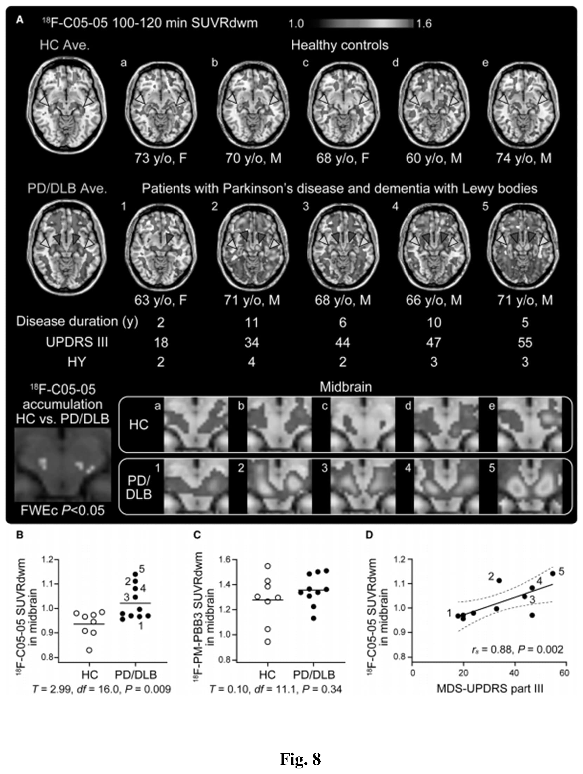

**Figure 8** shows PET imaging of human subjects received with the prior art compound C0505 (Endo et al., Imaging α-synuclein pathologies in animal models and patients with Parkinson's and related diseases, Neuron 112, 1-18, August 7, 2024). Although total tracer uptake in the midbrain was higher in PD/DLB patients, non-specific signals were also observed in healthy volunteers (A). Furthermore, the SUVR values of many PD/DLB cases were close to or even lower than those of healthy volunteers (B), indicating that C0505 is ineffective for PD/DLB imaging.

All documents mentioned in the present invention are cited as references in this application, as if each document were individually cited as a reference. Furthermore, it should be understood that after reading the above teachings of the present invention, those skilled in the art can make various changes or modifications to the present invention, and these equivalent forms also fall within the scope defined by the appended claims.

## Claims

1. A compound specifically recognizing α-synuclein aggregates, **characterized in that** the compound is a compound of Formula A, or a stereoisomer, pharmaceutically acceptable salt, solvate, or stable isotopic variant thereof, 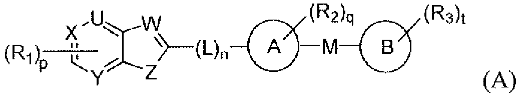 wherein,
X, Y, and U are each independently selected from CH and N, and when any one of them is CH, the hydrogen atom on said CH may be replaced by R₁;
W is selected from CH and N;
Z is selected from CH₂, NH, O, and S;
L is selected from -CH=CH- and -C≡C-, and when n is greater than 1, -(L)ₙ- is a chain formed by connecting Ls that are the same or different;
Ring A is selected from C₆₋₈ cycloalkane, C₆₋₁₂ aromatic ring, 6-10 membered heteroaromatic ring, and 6-8 membered heterocyclic ring;
Ring B is selected from C₄₋₈ cycloalkane, C₆₋₁₂ aromatic ring, 5-10 membered heteroaromatic ring, and 4-8 membered heterocyclic ring;
M is a direct bond or a 4-8 membered heterocyclic ring;
R₁ are each independently selected from: deuterium, tritium, hydroxy, amino, halogen, nitro, cyano, -COOH, -C₁₋₆ alkyl, -C₂₋₆ alkenyl, -C₂₋₆ alkynyl, -O-C₁₋₆ alkyl, -NHC₁₋₆ alkyl, -N(C₁₋₆ alkyl)₂, -SO₂-C₁₋₆ alkyl, -CO-C₁₋₆ alkyl, -C(O)OC₁₋₆ alkyl, -C(O)NH₂, -C(O)NHC₁₋₆ alkyl, -C(O)N(C₁₋₆ alkyl)₂, -NHCO-C₁₋₆ alkyl, -N(C₁₋₆ alkyl)-CO-C₁₋₆ alkyl, -SO₂NH₂, -SO₂NHC₁₋₆ alkyl, -SO₂N(C₁₋₆ alkyl)₂, -NHSO₂-C₁₋₆ alkyl, -N(C₁₋₆ alkyl)-SO₂-C₁₋₆ alkyl, -NH-C₃₋₈ cycloalkyl, -N(C₁₋₆ alkyl)(C₃₋₈ cycloalkyl), -C₃₋₈ cycloalkyl, 3-8 membered heterocyclyl, 5-10 membered heteroaryl,
wherein the -C₁₋₆ alkyl or the C₁₋₆ alkyl as part of a group, -C₃₋₈ cycloalkyl or the -C₃₋₈ cycloalkyl as part of a group, 3-8 membered heterocyclyl, and 5-10 membered heteroaryl in R₁ are each independently optionally substituted by one or more substituents, said substituents are each independently selected from deuterium, tritium, -O-C₁₋₆ alkyl, -C₁₋₆ alkyl, halogen, hydroxy, oxo, - NHC₁₋₆ alkyl, -N(C₁₋₆ alkyl)₂, -O-(3-6 membered heterocyclyl), -O-(p-toluenesulfonyl), and two substituents attached to the same carbon atom optionally together with the carbon atom to which they are attached form a 3-6 membered heterocyclic ring;
R₂ and R₃ are each independently selected from: deuterium, tritium, hydroxy, amino, halogen, nitro, cyano, -COOH, -C₁₋₆ alkyl, -C₂₋₆ alkenyl, -C₂₋₆ alkynyl, -O-C₁₋₆ alkyl, -NHC₁₋₆ alkyl, -N(C₁₋₆ alkyl)₂, -SO₂-C₁₋₆ alkyl, -CO-C₁₋₆ alkyl, -C(O)OC₁₋₆ alkyl, -C(O)NH₂, -C(O)NHC₁₋₆ alkyl, - C(O)N(C₁₋₆ alkyl)₂, -NHCO-C₁₋₆ alkyl, -N(C₁₋₆ alkyl)-CO-C₁₋₆ alkyl, -SO₂NH₂, -SO₂NHC₁₋₆ alkyl, - SO₂N(C₁₋₆ alkyl)₂, -NHSO₂-C₁₋₆ alkyl, -N(C₁₋₆ alkyl)-SO₂-C₁₋₆ alkyl, -NH-C₃₋₈ cycloalkyl, -N(C₁₋₆ alkyl)(C₃₋₈ cycloalkyl), -C₃₋₈ cycloalkyl, 3-8 membered heterocyclyl, 5-10 membered heteroaryl, and C₆₋₁₂ aryl, wherein the -C₃₋₈ cycloalkyl, 3-8 membered heterocyclyl, 5-10 membered heteroaryl, and C₆₋₁₂ aryl each independently are optionally substituted by halogen, hydroxy, -OC₁₋₆ alkyl, -NHC₁₋₆ alkyl, or -N(C₁₋₆ alkyl)₂, wherein the -C₁₋₆ alkyl appearing or the C₁₋₆ alkyl as part of another group or substituent in R₃ is optionally substituted by one or more substituents each independently selected from: deuterium, tritium, halogen, hydroxy, -O-(3-6 membered heterocyclyl), -O-(p-toluenesulfonyl);
n is an integer from 0 to 3;
p is an integer from 1 to 4;
q and t are each independently integers from 0 to 5;
wherein the heteroaryl and heterocyclyl each independently contain 1, 2, 3, or 4 heteroatoms selected from N, S, or O; and
wherein each occurrence of halogen is optionally in its isotopic form, and the carbon atoms and/or hydrogen atoms in the substituents are optionally in their isotopic forms.

2. The compound according to claim 1, or a stereoisomer, pharmaceutically acceptable salt, solvate, or stable isotopic variant thereof, wherein W is selected from CH and N; and Z is selected from O and S.

3. The compound according to claim 1 or 2, or a stereoisomer, pharmaceutically acceptable salt, solvate, or stable isotopic variant thereof, wherein X, Y, and U are all CH, or one or two of X, Y, and U are N and the rest are CH.

4. The compound according to any one of claims 1 to 3, or a stereoisomer, pharmaceutically acceptable salt, solvate, or stable isotopic variant thereof, wherein the fused ring containing X, Y, U, W, and Z is selected from:

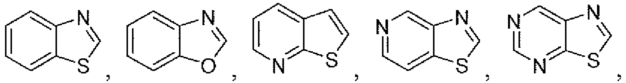

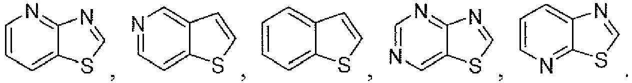

5. The compound according to any one of claims 1 to 4, or a stereoisomer, pharmaceutically acceptable salt, solvate, or stable isotopic variant thereof, wherein p is an integer from 1 to 2, and R₁ are each independently selected from: halogen, nitro, hydroxy, -O-C₁₋₆ alkyl, -NHC₁₋₆ alkyl, -N(C₁₋₆ alkyl)₂, -COOH, -NHCO-C₁₋₆ alkyl, -N(C₁₋₆ alkyl)-CO-C₁₋₆ alkyl, and 5-8 membered heterocyclyl, wherein the -C₁₋₆ alkyl or the C₁₋₆ alkyl as part of a group and the 5-8 membered heterocyclyl in R₁ are each independently optionally substituted by one or more, for example 1-2 substituents, said substituents are each independently selected from deuterium, tritium, -O-C₁₋₆ alkyl, -C₁₋₆ alkyl, halogen, hydroxy, oxo, -NHC₁₋₆ alkyl, -N(C₁₋₆ alkyl)₂, -O-(3-6 membered heterocyclyl), -O-(p-toluenesulfonyl), wherein the halogen appearing in R₁ is optionally in isotopic form, for example 18F.

6. The compound according to any one of claims 1 to 5, or a stereoisomer, pharmaceutically acceptable salt, solvate, or stable isotopic variant thereof, wherein p is an integer from 1 to 2, and R₁ are each independently selected from halogen, nitro, hydroxy, -O-C₁₋₆ alkyl, -O-C₁₋₆ haloalkyl, -O-C₁₋₆ halohydroxyalkyl, -O-C₁₋₆ alkyl substituted with O-(3-6 membered heterocyclyl) and/or -O-(p-toluenesulfonyl), -NHC₁₋₆ alkyl, -NHC₁₋₆ haloalkyl, -NHC₁₋₆ halohydroxyalkyl, -NH-C₁₋₆ alkyl substituted with O-(3-6 membered heterocyclyl) and/or -O-(p-toluenesulfonyl), -N(C₁₋₆ alkyl)₂, - COOH, -NHCO-C₁₋₆ alkyl, -N(C₁₋₆ alkyl)-CO-C₁₋₆ alkyl, and 5-6 membered heterocyclyl, wherein the 5-6 membered heterocyclyl is optionally substituted by one or more, for example 1-2 substituents, said substituents are each independently selected from deuterium, tritium, -O-C₁₋₆ alkyl, -C₁₋₆ alkyl, halogen, hydroxy, oxo, -NHC₁₋₆ alkyl, -N(C₁₋₆ alkyl)₂, wherein the halogen appearing in R₁ is optionally in isotopic form, for example F18.

7. The compound according to any one of claims 1 to 6, or a stereoisomer, pharmaceutically acceptable salt, solvate, or stable isotopic variant thereof, wherein p is an integer from 1 to 2, and R₁ are each independently selected from: F, Br,

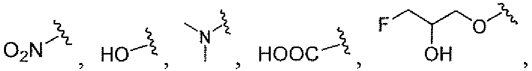

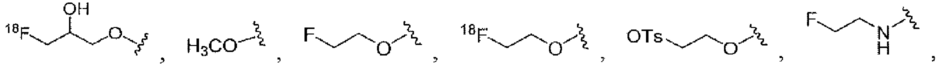

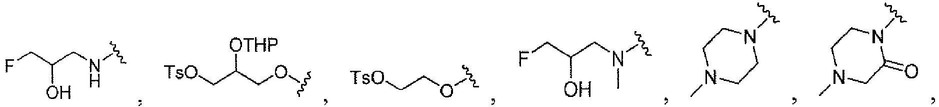

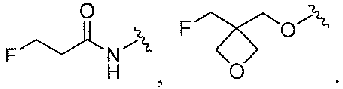

8. The compound according to any one of claims 1 to 7, or a stereoisomer, pharmaceutically acceptable salt, solvate, or stable isotopic variant thereof, wherein p is 1, and R₁ is located on the ring carbon atom adjacent to Y; or p is 2, and R₁ are attached respectively to Y and the ring carbon atom adjacent to Y, or R₁ are attached respectively to X and the ring carbon atom between X and Y.

9. The compound according to any one of claims 1 to 8, or a stereoisomer, pharmaceutically acceptable salt, solvate, or stable isotopic variant thereof, wherein p is 1.

10. The compound according to any one of claims 1 to 9, or a stereoisomer, pharmaceutically acceptable salt, solvate, or stable isotopic variant thereof, wherein R₁ are each independently selected from

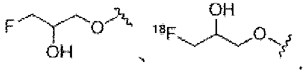

11. The compound according to any one of claims 1 to 10, or a stereoisomer, pharmaceutically acceptable salt, solvate, or stable isotopic variant thereof, wherein n is not 0 and M is absent, and Formula (A) has the following sub-formula:

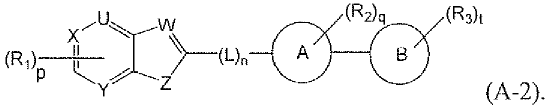

12. The compound according to any one of claims 1 to 10, or a stereoisomer, pharmaceutically acceptable salt, solvate, or stable isotopic variant thereof, wherein n is 0 and M is a 4-8 membered heterocyclic ring, and Formula (A) has the following sub-formula:

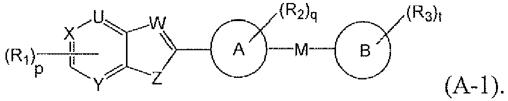

13. The compound according to any one of claims 1 to 11, or a stereoisomer, pharmaceutically acceptable salt, solvate, or stable isotopic variant thereof, wherein
n is 1, and -L- is -CH=CH-, or L is -C=C-; or
n is 2, and -(L)₂- is -CH=CH-CH=CH-, -CH=CH-C=C-, -C=C-CH=CH-, or -C=C-C=C-, preferably -CH=CH-CH=CH- or -CH=CH-C≡C-; or
n is 3, and -(L)₃- is -CH=CH-CH=CH-CH=CH-, -CH=CH-CH=CH-C=C-, -CH=CH-C=C-CH=CH-, -C≡C-CH=CH-CH=CH-, -CH=CH-C≡C-C≡C-, -C=C-CH=CH-C=C- or -C≡C-C≡C-CH=CH- , preferably -CH=CH-CH=CH-CH=CH-, -CH=CH-CH=CH-C=C-, -CH=CH-C≡C-CH=CH- or -CH=CH-C=C-C=C-.

14. The compound according to any one of claims 1 to 13, or a stereoisomer, pharmaceutically acceptable salt, solvate, or stable isotopic variant thereof, wherein Ring A is selected from a C₆₋₁₀ aromatic ring, a 6-8 membered heteroaromatic ring, and a 6-8 membered heterocyclic ring, for example Ring A is selected from a benzene ring and a 6-membered heteroaromatic ring or a 6-membered heterocyclic ring containing 1 or 2 nitrogen heteroatoms, for example A is selected from

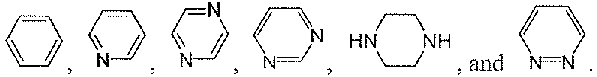

15. The compound according to any one of claims 1 to 14, or a stereoisomer, pharmaceutically acceptable salt, solvate, or stable isotopic variant thereof, wherein Ring A is selected from

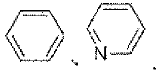

16. The compound according to any one of claims 1 to 15, or a stereoisomer, pharmaceutically acceptable salt, solvate, or stable isotopic variant thereof, wherein Ring A is attached to the rest of the molecule via ring carbon atoms, or when Ring A is a heterocycle, also via ring heteroatom(s).

17. The compound according to any one of claims 1 to 16, or a stereoisomer, pharmaceutically acceptable salt, solvate, or stable isotopic variant thereof, wherein Ring A is unsubstituted, or Ring A is substituted with one R₂, R₂ is selected from halogen, nitro, cyano, and halogen-substituted -C₁₋₆ alkyl, for example selected from halogen, nitro, and cyano, for example R₂ is selected from halogen or an isotope thereof, for example R₂ is selected from F and 18F.

18. The compound according to any one of claims 1 to 17, or a stereoisomer, pharmaceutically acceptable salt, solvate, or stable isotopic variant thereof, wherein Ring B is selected from a C₆₋₁₀ aromatic ring and a 5-8 membered heteroaromatic ring, for example Ring B is selected from a benzene ring, a naphthalene ring, and a 5-6 membered heteroaromatic ring containing 1-3, for example 1 or 2 heteroatoms selected from nitrogen, oxygen, and sulfur, for example B is selected from

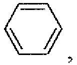

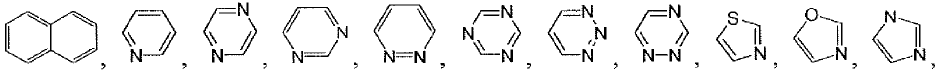

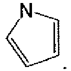

19. The compound according to any one of claims 1 to 18, or a stereoisomer, pharmaceutically acceptable salt, solvate, or stable isotopic variant thereof, wherein Ring B is selected from

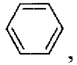

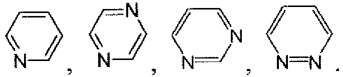

20. The compound according to any one of claims 1 to 19, or a stereoisomer, pharmaceutically acceptable salt, solvate, or stable isotopic variant thereof, wherein Ring B is optionally substituted by 1 or 2 R₃, R₃ is selected from hydroxy, amino, halogen, nitro, cyano, -C₁₋₆ alkyl, -O-C₁₋₆ alkyl, -NHC₁₋₆ alkyl, -N(C₁₋₆ alkyl)₂, -C₃₋₈ cycloalkyl, 5-8 membered heterocyclyl, and C₆₋₁₀ aryl, said -C₃₋₈ cycloalkyl, 5-8 membered heterocyclyl, and C₆₋₁₀ aryl are preferably -C₃₋₆ cycloalkyl, 5-7 membered heterocyclyl, and C₆ aryl, respectively, each independently optionally substituted by halogen, hydroxy, -OC₁₋₆ alkyl, -NHCl₁₋₆ alkyl, or -N(C₁₋₆ alkyl)₂, wherein the -C₁₋₆ alkyl appearing in R₃ is optionally substituted by 1 or 2 substituents each independently selected from: deuterium, tritium, halogen, hydroxy, -O-(3-6 membered heterocyclyl), and -O-(p-toluenesulfonyl), preferably deuterium, tritium, halogen, and hydroxy.

21. The compound according to any one of claims 1 to 20, or a stereoisomer, pharmaceutically acceptable salt, solvate, or stable isotopic variant thereof, wherein Ring B is optionally substituted by 1 or 2 R₃, R₃ is selected from: hydroxy, amino, F, Br, nitro, cyano, -O-CH₃, -NHCH₃, -N(CH₃)₂,

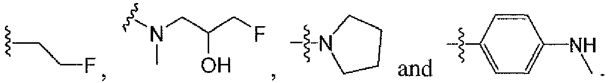

22. The compound according to any one of claims 1 to 21, or a stereoisomer, pharmaceutically acceptable salt, solvate, or stable isotopic variant thereof, wherein Ring B is substituted with -NHC₁₋₆ alkyl.

23. The compound according to any one of claims 1 to 22, or a stereoisomer, pharmaceutically acceptable salt, solvate, or stable isotopic variant thereof, wherein t is 1.

24. The compound according to any one of claims 1 to 8 and 10 to 17, or a stereoisomer, pharmaceutically acceptable salt, solvate, or stable isotopic variant thereof, wherein M is a 6-8 membered nitrogen-containing heterocyclic ring, for example a 6-membered nitrogen-containing heterocyclic ring, for example

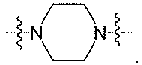

25. The compound according to any one of claims 1 to 24, or a stereoisomer, pharmaceutically acceptable salt, solvate, or stable isotopic variant thereof, wherein the stable isotopic variant comprises an isotope selected from 2H, 3H, 13C, 11C, 14C, 15N, 180, 170, 31P, 32P, 35S, 18F, and 36Cl, preferably comprises 18F.

26. The compound according to claim 1, or a stereoisomer, pharmaceutically acceptable salt, solvate, or stable isotopic variant thereof, selected from:
| | | | |
|---|---|---|---|
| I-1 | 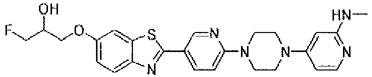 | I-26 | 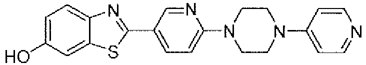 |
| I-2 | 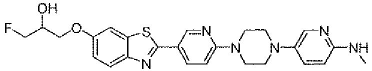 | I-27 | 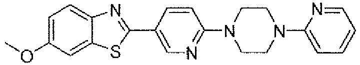 |
| I-3 | 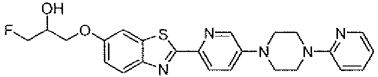 | I-28 | 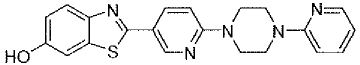 |
| I-4 | 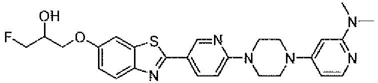 | I-29 | 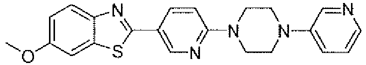 |
| I-5 | 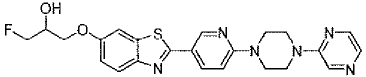 | I-30 | 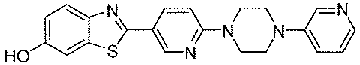 |
| I-6 | 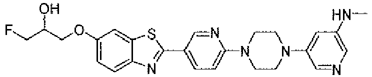 | I-31 | 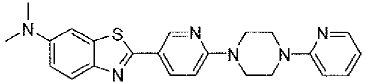 |
| I-7 | 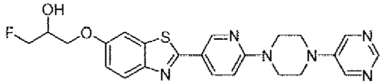 | I-32 | 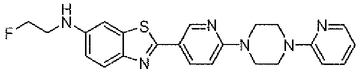 |
| I-8 | 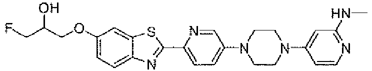 | I-33 | 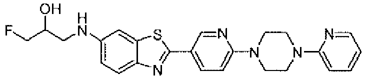 |
| I-9 | 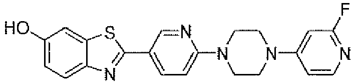 | I-34 | 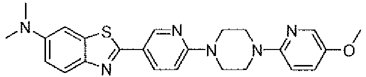 |
| I-10 | 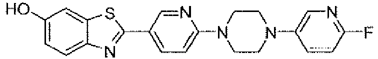 | I-35 | 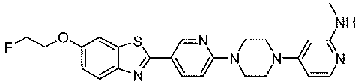 |
| I-11 | 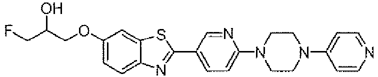 | I-36 | 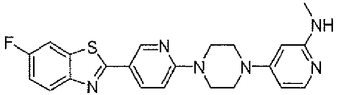 |
| I-12 | 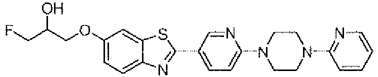 | I-37 | 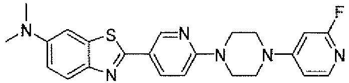 |
| I-13 | 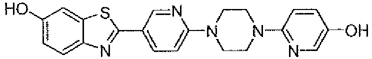 | I-38 | 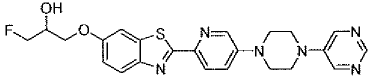 |
| I-14 | 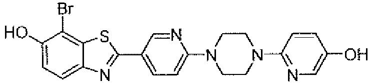 | I-39 | 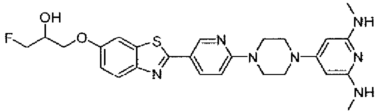 |
| I-15 | 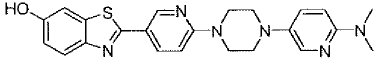 | I-40 | 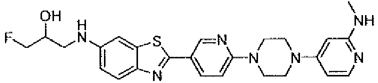 |
| I-16 | 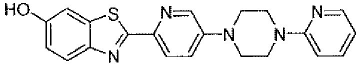 | I-41 | 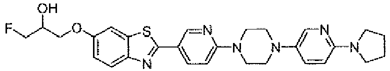 |
| I-17 | 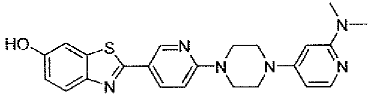 | I-42 | 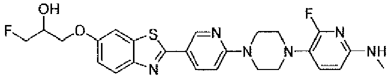 |
| I-18 | 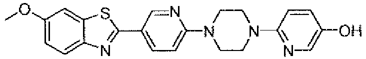 | I-43 | 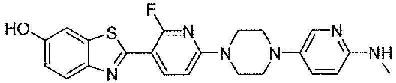 |
| I-19 | 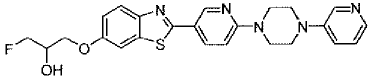 | I-44 | 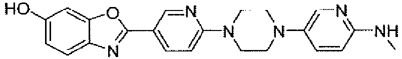 |
| I-20 | 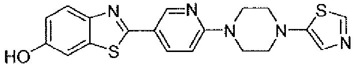 | I-45 | 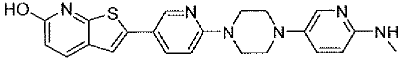 |
| I-21 | 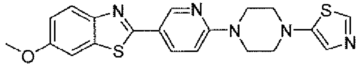 | I-46 | 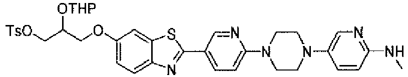 |
| I-22 | 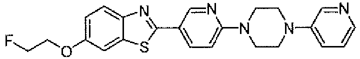 | I-47 | 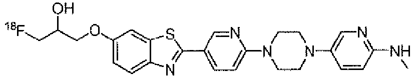 |
| I-23 | 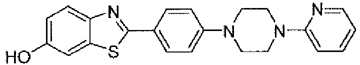 | I-48 | 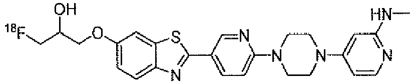 |
| I-24 | 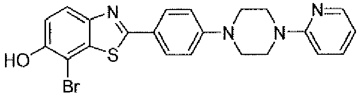 | I-49 | 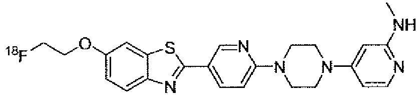 |
| I-25 | 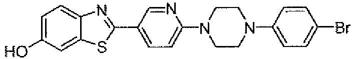 | I-50 | 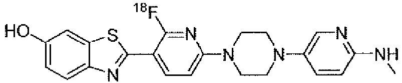 |
| | | I-51 | 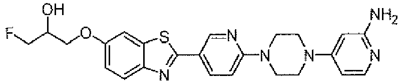 |
| II-1 | 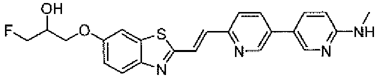 | II-16 | 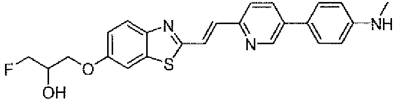 |
| II-2 | 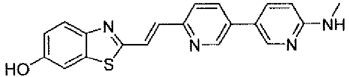 | II-17 | 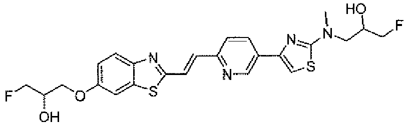 |
| II-3 | 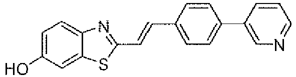 | II-18 | 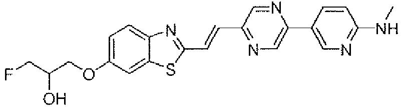 |
| II-4 | 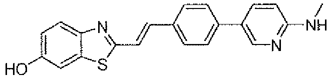 | II-19 | 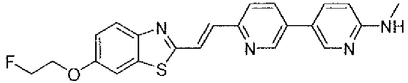 |
| II-5 | 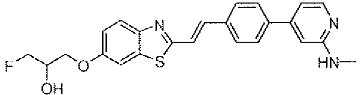 | II-20 | 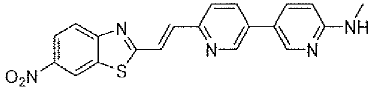 |
| II-6 | 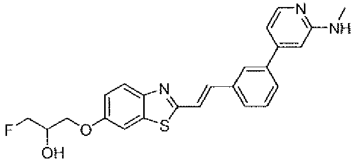 | II-21 | 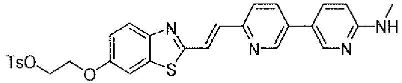 |
| II-7 | 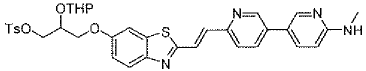 | II-22 | 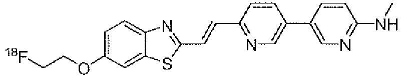 |
| II-8 | 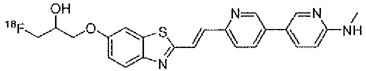 | II-23 | 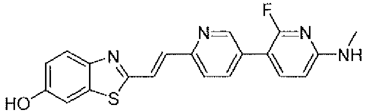 |
| II-9 | 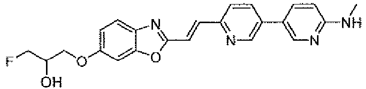 | II-24 | 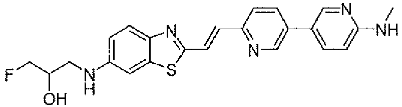 |
| II-10 | 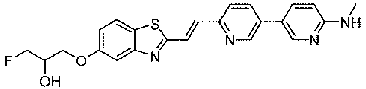 | II-25 | 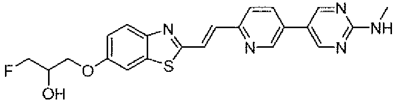 |
| II-11 | 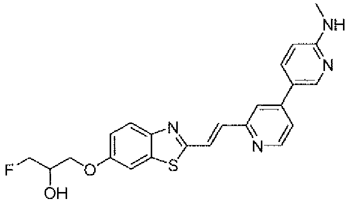 | II-26 | 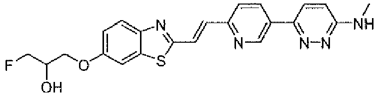 |
| II-12 | 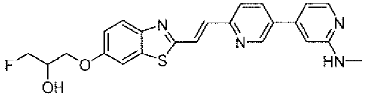 | II-27 | 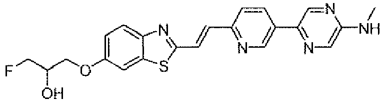 |
| II-13 | 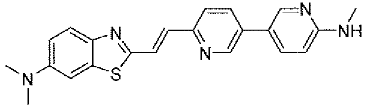 | II-28 | 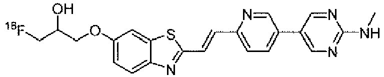 |
| II-14 | 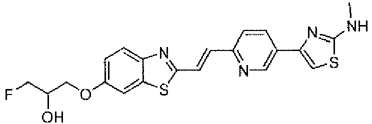 | II-29 | 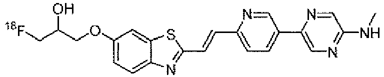 |
| II-15 | 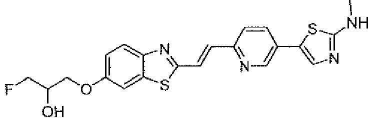 | | |
| III-1 | 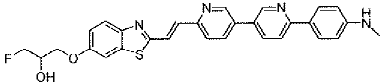 | III-13 | 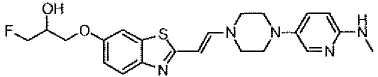 |
| III-2 | 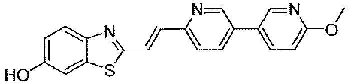 | III-14 | 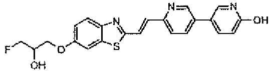 |
| III-3 | 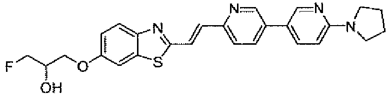 | III-15 | 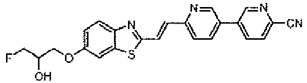 |
| III-4 | 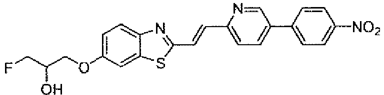 | III-16 | 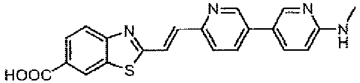 |
| III-5 | 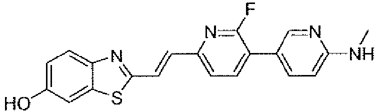 | III-17 | 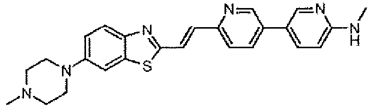 |
| III-6 | 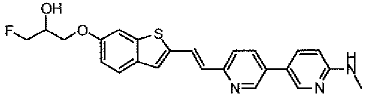 | III-18 | 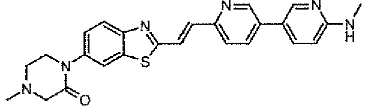 |
| III-7 | 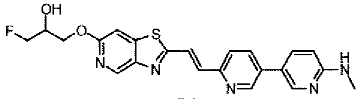 | III-19 | 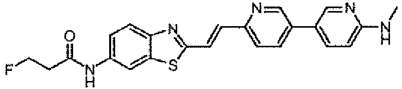 |
| III-8 | 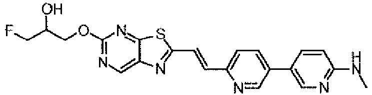 | III-20 | 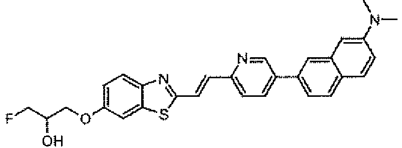 |
| III-9 | 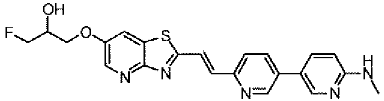 | III-21 | 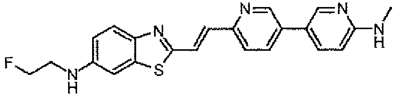 |
| III-10 | 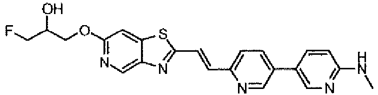 | III-22 | 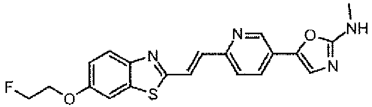 |
| III-11 | 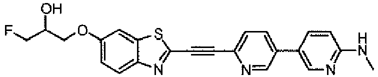 | III-12 | 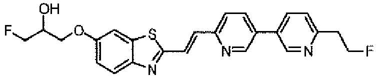 |
| III-23 | 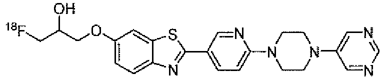 | | |

27. The compound according to claim 26, selected from the following compounds and their stereoisomers, or pharmaceutically acceptable salts or solvates thereof:

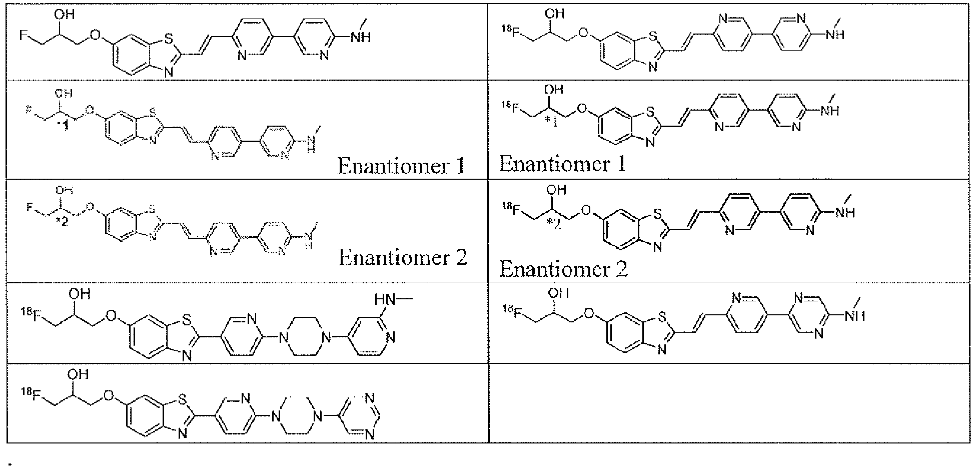

28. The compound according to any one of claims 1 to 27, or a stereoisomer, pharmaceutically acceptable salt, solvate, or stable isotopic variant thereof, for use in treating or diagnosing a neurodegenerative disease associated with α-synuclein aggregates and aggregates of other misfolded proteins.

29. A composition specifically binding to α-synuclein aggregates, comprising the compound according to any one of claims 1 to 28, or a stereoisomer, pharmaceutically acceptable salt, solvate, or stable isotopic variant thereof, and one or more pharmaceutically acceptable carriers.

30. Use of the compound according to any one of claims 1 to 28, or a stereoisomer, pharmaceutically acceptable salt, solvate, or stable isotopic variant thereof, or the composition according to claim 29, in the manufacture of a medicament for treating or diagnosing a neurodegenerative disease associated with α-synuclein aggregates and aggregates of other misfolded proteins.

31. The use according to claim 30, wherein the neurodegenerative disease is selected from Parkinson's disease, dementia with Lewy bodies, multiple system atrophy, Alzheimer's disease, amyotrophic lateral sclerosis, progressive muscular atrophy, progressive supranuclear palsy, preferably Parkinson's disease and progressive muscular atrophy.

32. A method for detecting α-synuclein aggregates in a subject, comprising the steps of:
(A) administering to the subject a safe and effective amount of the compound according to any one of claims 1 to 28 or the composition according to claim 29; and
(B) detecting the binding of said compound or composition to α-synuclein aggregates in the subject.

33. The method according to claim 32, **characterized in that** the detection in step (B) is performed by an imaging technique, preferably by positron emission computed tomography, single-photon emission computed tomography, near-infrared brain function imaging, optical imaging, or a combination thereof.
